# FASCICULE DE BREVET EUROPEEN

(11) **EP 0 946 731 B1**
(45) Date de publication et mention de la délivrance du brevet: **11.02.2009**
(21) Numéro de dépôt: 97950245.7
(22) Date de dépôt: 08.12.1997
(51) Int. Cl.: C12N 15/49, C12N 7/00, C12Q 1/68, A61K 39/21, C07K 16/10, C07K 14/16, G01N 33/50

(54) **SOUCHES DE VIH-1 NON-M NON-O, FRAGMENTS ET APPLICATIONS**
NICHT-M NICHT-O HIV-1-STAMM, FRAGMENTE UND VERWENDUNGEN
NON-M NON-O HIV STRAINS, FRAGMENTS AND APPLICATIONS

(30) Priorité: 09.12.1996 FR 9615087
(43) Date de publication de la demande: 06.10.1999
(73) Titulaire: INSTITUT NATIONAL DE LA SANTE ET DE LA RECHERCHE MEDICALE (INSERM), 75654 Paris Cédex 13 (FR); ASSISTANCE PUBLIQUE, HOPITAUX DE PARIS, 75100 Paris (FR); INSTITUT PASTEUR, 75724 Paris Cédex 15 (FR)
(72) Inventeur: MAUCLERE, Philippe, F-33000 Bordeaux (FR); LOUSSERT-AJAKA, Ibtissam, F-78500 Sartrouville (FR); SIMON, François, F-75018 Paris (FR); SARAGOSTI, Sentob, F-92100 Boulogne Billancourt (FR); BARRE-SINOUSSI, Françoise, F-92130 Issy-les-Moulineaux (FR)
(74) Mandataire: Goulard, Sophie
(86) Numéro de dépôt international: PCT/FR1997/002227
(87) Numéro de publication internationale: WO 1998/026075

(56) Documents cités:
- WO-A-86/02383
- HUET, Z. ET AL.: "A highly defective HIV-1 strain isolated from a healthy Gabonese individual presenting an atypical Western blot" AIDS, vol. 3, no. 11, novembre 1989, pages 707-715, XP002041193
- HUET T ET AL: "GENETIC ORGANIZATION OF A CHIMPANZEE LENTIVIRUS RELATED TO HIV-1" NATURE, vol. 345, no. 6273, 24 mai 1990, pages 356-359, XP000172750
- TOJO, N. ET AL.: "Cloning and nucleotide sequence of the Myxococcus xanthus lon gene: indispensability of lon for vegetative growth" JOURNAL OF BACTERIOLOGY, vol. 175, no. 8, avril 1993, pages 2271-2277, XP002041194
- INAGAKI, N. ET AL.: "Cloning and functional characterization of a third pituitary adenylate cyclase-activating polypeptide receptor subtype expressed in insulin-secreting cells" PROCEEDINGS OF THE NATIONAL ACADEMY OF SCIENCES OF USA., vol. 91, mars 1994, WASHINGTON US, pages 2679-2683, XP002041195
- AYOUBA ET AL.: "HIV-1 group N among HIV-1-seropositive individuals in Cameroon" AIDS, vol. 14, no. 16, 10 novembre 2000 (2000-11-10), pages 2623-2625,
- ROQUES ET AL.: "Phylogenetic characteristics of three new HIV-1 N strains and implications for the origin of group N" AIDS, vol. 18, 2004, pages 1-11,
- LEITNER T. ET AL.: "Updated proposal of References Sequences of HIV-1 Genetic Subtypes" 1997, pages III-19-III-24,
- PEETERS M; FRANSEN K; DELAPORTE E; VAN DEN HAESEVELDE M; GERSHY-DAMET G-M; KESTENS L; VAN DER GROEN G; PIOT P: "ISOLATION AND CHARACTERIZATION OF A NEW CHIMPANZEE LENTIVIRUS SIMIAN IMMUNODEFICIENCY VIRUS ISOLATE CPZ-ANT FROM A WILD-CAPTURED CHIMPANZEE" AIDS, vol. 6, no. 5, pages 447-451,
- DATABASE EMBL [Online] EMBL; 22 juin 1990 (1990-06-22), HUET ET AL.: "Chimpanzee immunodeficiency virus (CIV) (SIV(cpz)) genome" extrait de EMBL Database accession no. X552154

### LISTE DE SEQUENCES

(1) INFORMATIONS GENERALES:
   (i) DEPOSANT:
      (A) NOM: INSTITUT NATIONAL DE LA SANTE ET DE LA RECHERCHE MEDICALE - INSERM
      (B) RUE: 101 rue de Tolbiac
      (C) VILLE: PARIS
      (E) PAYS: FRANCE
      (F) CODE POSTAL: 75654 CEDEX 13

      (A) NOM: ASSISTANCE PUBLIQUE-HOPITAUX DE PARIS
      (B) RUE: 3 avenue Victoria
      (C) VILLE: PARIS
      (E) PAYS: FRANCE
      (F) CODE POSTAL: 75100 RP

      (A) NOM: INSTITUT PASTEUR
      (B) RUE: 28 rue du Docteur Roux
      (C) VILLE: PARIS
      (E) PAYS: FRANCE
      (F) CODE POSTAL: 75724 Cedex 15

      (A) NOM: MAUCLERE Philippe
      (B) RUE: 2 rue Buhan
      (C) VILLE: BORDEAUX
      (E) PAYS: FRANCE
      (F) CODE POSTAL: 33000

      (A) NOM: LOUSSERT-AJAKA Ibtissam
      (B) RUE: 26 avenue de la République
      (C) VILLE: SARTROUVILLE
      (E) PAYS: FRANCE
      (F) CODE POSTAL: 78500

      (A) NOM: SIMON François
      (B) RUE: 8 rue Germain Pilon
      (C) VILLE: PARIS
      (E) PAYS: FRANCE
      (F) CODE POSTAL: 75018

      (A) NOM: SARAGOSTI Sentob
      (B) RUE: 69 bis rue de Billancourt
      (C) VILLE: BOULOGNE BILLANCOURT
      (E) PAYS: FRANCE
      (F) CODE POSTAL: 92100

      (A) NOM: BARRE-SINOUSSI Françoise
      (B) RUE: 104 Le Capricorne, 50 rue d'Erevan
      (C) VILLE: ISSY LES MOULINEAUX
      (E) PAYS: FRANCE
      (F) CODE POSTAL: 92130
   (ii) TITRE DE L'INVENTION: SOUCHES DE VIH-1 NON-M NON-O, FRAGMENTS ET APPLICATIONS.
   (iii) NOMBRE DE SEQUENCES: 61
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 9183 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 1:
   CTTCTCGCTT GTACTGGGTC TCTCTTGCTG GACCAGATTA GAGCCTGGGA GCTCTCTGGC 60
   TAGCAGGGAA CCCACTGCTT AAGCCTCAAT AAAGCTTGCC TTGAGTGCTA AAGTGGTGTG 120
   TGCCCATCCA TTCGGTAACT CTGGTACCTA GAGATCCCTC AGACCATCTA GACTGAGTGA 180
   AAAATCTCTA GCAGTGGCGC CCGAACAGGG ACTTGAAAAC GAAAGTAGAA CCGGAGGCTG 240
   AATCTCTCGA CGCAGGACTC GGCTCGTTGG TGCACACAGC GAGAGGCGAG GCGGCGGAAG 300
   TGTGAGTACG CAATTTTGAC TGGCGGTGGC CAGAAAGTAG GAGAGAGGAT GGGTGCGAGA 360
   GCGTCAGTGT TAACAGGGGG AAAATTAGAT CAATGGGAAT CAATTTATTT GAGACCAGGG 420
   GGAAAGAAAA AATACAGAAT GAAACATTTA GTATGGGCAA GCAGGGAGCT GGAAAGATTC 480
   GCTTGTAACC CAGGTCTCAT GGACACAGCG GACGGCTGTG CCAAGTTACT AAATCAATTA 540
   GAACCAGCTC TCAAGACAGG GTCAGAAGAA CTGCGCTCTT TATATAACGC TCTAGCAGTT 600
   CTTTATTGTG TCCATAGTAG GATACAGATA CACAACACAC AGGAAGCTTT GGACAAGATA 660
   AAAGAGAAAC AGGAACAGCA CAAGCCCGAG CCAAAAAACC CAGAAGCAGG GGCAGCGGCA 720
   GCAACTGATA GCAATATCAG TAGGAATTAT CCTCTAGTCC AGACTGCTCA AGGACAAATG 780
   GTACATCAGC CGCTGACACC CAGAACCTTA AATGCTTGGG TGAAAGTGAT AGAGGAGAAG 840
   GCCTTTAGTC CAGAAGTAAT ACCAATGTTT ATGGCCTTGT CAGAAGGGGC AACGCCCTCA 900
   GATCTAAATA CTATGTTAAA TACAGTAGGG GGACATCAGG CAGCAATGCA GATGCTGAAG 960
   GAAGTCATCA ATGAGGAAGC AGCAGACTGG GATAGGACAC ATCCAGTCCC TGTGGGACCA 1020
   CTACCCCCAG GGCAACTGAG AGACCCTAGA GGAAGTGATA TAGCAGGAAC AACTAGCACC 1080
   CTGGCAGAAC AGGTGGCTTG GATGACTGCT AATCCTCCTG TTCCAGTAGG AGATATTTAT 1140
   AGAAGATGGA TAGTCCTGGG GTTAAACAGA ATTGTGAGAA TGTATAGTCC TGTCAGCATT 1200
   CTAGAGATCA AACAAGGACC AAAAGAACCC TTCAGAGACT ATGTAGACAG GTTCTACAAA 1260
   ACTCTAAGAG CAGAGCAGGC AACACAGGAA GTAAAGAATT GGATGACAGA AACACTCTTA 1320
   GTACAAAATG CAAACCCAGA TTGTAAACAG CTCCTAAAAG CATTAGGGCC AGGAGCTACC 1380
   TTAGAAGAGA TGATGACGGC CTGCCAGGGA GTGGGGGGAC CAGCACATAA GGCAAGAGTG 1440
   CTAGCAGAGG CTATGTCACA GGTGCAGCAG CCAACAACTA GTGTCTTTGC ACAAAGGGGA 1500
   AACTTTAAAG GCATAAGGAA ACCCATTAAA TGTTTCAATT GTGGCAAAGA GGGCCATTTG 1560
   GCAAGAAACT GTAAGGCCCC TAGAAGAGGA GGCTGTTGGA AGTGTGGGCA AGAAGGACAT 1620
   CAAATGAAAG ATTGTAAAAA TGAAGGAAGA CAGGCTAATT TTTTAGGGAA GAGCTGGTCT 1680
   CCCTTCAAAG GGAGACCAGG AAACTTCCCC CAGACAACAA CAAGGAAAGA GCCCACAGCC 1740
   CCGCCACTAG AGAGTTATGG GTTTCAGGAG GAGAAGAGCA CACAGGGGAA GGAGATGCAG 1800
   GAGAACCAGG AGAGGACAGA GAACTCTCTG TACCCACCTT TAACTTCCCT CAGATCACTC 1860
   TTTGGCAACG ACCCGTCATC ACAGTAAAAA TAGGGAAAGA AGTAAGAGAA GCTCTTTTAG 1920
   ATACAGGAGC TGATGATACA GTAATAGAAG AGCTACAATT AGAGGGAAAA TGGAAACCAA 1980
   AAATGATAGG AGGAATTGGA GGATTTATCA AAGTGAGACA ATATGATAAT ATAACAGTAG 2040
   ACATACAGGG AAGAAAAGCA GTTGGTACAG TATTAGTAGG ACCAACACCT GTTAATATTA 2100
   TAGGAAGAAA TCTTTTAACC CAGATTGGCT GTACTTTAAA TTTTCCAATA AGTCCTATTG 2160
   AAACTGTACC AGTAAAATTA AAACCAGGAA TGGATGGCCC AAAGGTAAAA CAATGGCCTT 2220
   TGACAACAGA AAAAATAGAG GCATTAAGAG AAATTTGTAC AGAAATGGAA AAGGAAGGAA 2280
   AAATTTCTAG AATAGGGCCT GAGAATCCAT ATAACACTCC AATTTTTGCT ATAAAAAAGA 2340
   AAGATAGCAC TAAATGGAGA AAATTAGTAG ATTTCAGGGA ATTAAATAAA AGGACCCAAG 2400
   ATTTTTGGGA AGTGCAGCTA GGAATTCCAC ATCCAGCAGG ATTAAAGCAG AAAAAATCAG 2460
   TGACAGTTTT GGATGTAGGA GATGCTTATT TTTCATGTCC CTTGGACAAA GATTTTAGAA 2520
   AGTATACAGC TTTTACCATA CCTAGTATAA ACAATGAGAC ACCTGGTATT AGATACCAGT 2580
   ATAATGTGCT GCCACAAGGC TGGAAAGGGT CACCAGCAAT TTTTCAGAGT ACAATGACAA 2640
   AAATTCTAGA ACCATTCAGA GAGAAACATC CAGAGATAAT CATTTACCAG TACATGGATG 2700
   ACCTCTATGT GGGATCTGAC TTAGAACTAG CACAACATAG AGAGGCAGTA GAAGACCTTA 2760
   GAGATCATCT TTTGAAGTGG GGCTTTACGA CCCCTGACAA AAAACATCAG AAGGAACCCC 2820
   CGTTCCTCTG GATGGGATAT GAACTCCATC CAGACAAATG GACAGTCCAG CCAATAAAGT 2880
   TACCAGAAAA GGATGTATGG ACTGTCAATG ATATACAGAA ATTAGTAGGA AAGTTAAATT 2940
   GGGCAAGTCA GATCTATCCA GGAATCAGAG TAAAACAGCT CTGTAAATTA ATCAGAGGAA 3000
   CCAAAGCTTT GACAGAAGTA GTCAACTTTA CAGAAGAAGC AGAATTAGAA CTAGCAGAAA 3060
   ACAGGGAGAT ATTAAAAGAA CCCCTGCATG GAGTCTATTA TGACCCAGGA AAAGAATTAG 3120
   TAGCAGAAAT TCAAAAGCAA GGACAAGGTC AGTGGACATA TCAGATTTAT CAGGAGTTAC 3180
   ATAAAAATTT AAAAACAGGA AAGTATGCAA AAATGAGATC TGCCCATACT AATGATATAA 3240
   AACAGTTAGT TGAAGTGGTA AGGAAAGTGG CAACAGAAAG TATAGTAATT TGGGGAAAGA 3300
   CTCCTAAATT TAGATTACCA GTACAAAAGG AAGTGTGGGA GGCATGGTGG ACCGATCATT 3360
   GGCAAGCAAC TTGGATTCCT GAGTGGGAAT TTGTCAACAC TCCTCCCCTT GTAAAATTAT 3420
   GGTATCAGTT AGAAACAGAG CCAATCAGTG GGGCAGAAAC TTTCTATGTA GATGGAGCAG 3480
   CTAATAGGGA AACAAAATTG GGAAAAGCAG GTTTTGTGAC AGATAGGGGA AGACAGAAAG 3540
   TGGTCTCTAT TGCAGACACC ACCAATCAAA AGGCTGAGTT ACAAGCTATC CTTATGGCCT 3600
   TACAAGAGTC AGGACGGGAT GTAAACATAG TCACTGACTC TCAGTATGCT ATGGGAATAA 3660
   TTCATTCACA GCCAGATAAA AGTGAATCAG AATTGGTGAG CCAAATAATA GAAGAGCTCA 3720
   TAAAAAAGGA AAGAGTTTAT CTCTCTTGGG TACCTGCACA TAAAGGTATT GGAGGAAATG 3780
   AGCAGGTAGA CAAATTAGTT AGCTCAGGAA TTAGAAAAAT ATTATTCCTA GATGGTATAG 3840
   AAAAAGCCCA AGAAGATCAT GACAGATATC ACAGCAATTG GAAAGCAATG GCCAGTGATT 3900
   TTAACTTACC CCCCATAGTG GCAAAAGAAA TAGTAGCCAG CTGTGACAAA TGCCAGCTAA 3960
   AAGGGGAAGC CATGCATGGA CAGGTCAATT GTAGTCCAGG AGTGTGGCAA TTAGATTGTA 4020
   CACACTTAGA GGGAAAAATC ATCCTTGTGG CGGTCCATGT GGCCAGTGGC TACTTAGAAG 4080
   CAGAAGTTAT TCCTGCAGAG ACAGGACAGG AAACAGCATA TTTTATTTTA AAGTTAGCTG 4140
   GAAGATGGCC AGTAAAAGTT ATACACACTG ATAATGGATC CAATTTCACT AGTGCCACTG 4200
   TAAAAGCAGC CTGTTGGTGG GCAAATATCA AACAGGAATT TGGGATACCC TACAATCCTC 4260
   AAAGTCAGGG AGCAGTAGAG TCCATGAATA AAGAATTAAA GAAAATTATA GGACAAATCA 4320
   GAGATCAAGC AGAACATCTA AAGACAGCAG TGCAAATGGC GGTTTTCATT CACAATTTTA 4380
   AAAGAAAAGG GGGGATTGGG GGGTACACTG CAGGGGAAAG AATAATAGAC ATAATAGCAA 4440
   CAGACATACA GACAACAAAT TTACAAACAC AAATTTTAAA AGTTCAAAAT TTTCGGGTTT 4500
   ATTACAGAGA CAGCAGAGAT CCCATTTGGA AAGGACCAGC CAAACTTCTG TGGAAAGGAG 4560
   AAGGGGCAGT GGTAATTCAA GATAACGGGG ATATAAAAGT AGTCCCACGT AGGAAAGCAA 4620
   AAATAATTAG GGATTATGGA AAACAGATGG CAGGTGATGG TTGTGTGGCA AGTGGACAGG 4680
   ATGAAAATCA GGAAATGGAA TAGCTTAGTA AAACATCATA TGTATGTGTC AAAAAAGGCA 4740
   AAAGGATGGT ATTATAGACA TCATTATGAA ACACATCACC CAAAAATAAG TTCAGAAGTA 4800
   CATATCCCAG TAGGTCAGGC AAGATTAGTG ACAGTCACTT ATTGGGGGCT AACAACAGGA 4860
   GAACAGTCTT GGCATCTAGG ACATGGAGTA TCCATAGAAT GGAGACTAAG AAAATACAAG 4920
   ACACAAGTTG ATCCTGAAAT GGCAGACAAG CTAATACATC TTCATTATTT TGATTGTTTT 4980
   ACAGCCTCTG CCATAAGGCA AGCGGTCTTA GGGAGACCAG TATTACCTAG GTGTGAATAT 5040
   CCAGCAGGGC ACAAACAGGT AGGCACCCTA CAATATCTAG CACTAACAGC CTGGGTGGGA 5100
   GCAAAGAAGA GAAAGCCACC CTTACCTAGT GTGACTAAGC TAACAGAAGA TAGATGGAAC 5160
   GAGCACCAGA AGATGCAGGG CCACAGAGGG AACCCTATAA TGAATGGGCA CTAGAATTAT 5220
   TAGAAGAATT AAAAAATGAA GCTGTGCGCC ATTTTCCAAG GATTTGGCTA CATGGGTTAG 5280
   GACAACACAT CTATAACACA TATGGAGACA CCTGGGAGGG GGTAGAGGCA ATTATCAGGA 5340
   TACTACAACA ATTACTGTTT ATCCATTATA GGATTGGCTG CCAGCACAGC AGAATAGGGA 5400
   TCACTCCTCA AAGGAGAAGG AATGGAACCA GTAGATCCTA GATTAGAGCC CTGGAATCAT 5460
   CCAGGAAGCC AACCTAAAAC AGCTTGCAAT AATTGCTATT GTAAAAGATG TTGCTATCAC 5520
   TGCTTATATT GCTTCACAAA GAAAGGCTTA GGCATCTCAT ATGGCAGGAA GAAGCGGAGT 5580
   CAACGACGAA GAACTCCTCA GAGCAGTAAG AGTCATCAAG ATCTTATACC AGAGCAGTAA 5640
   GTAAAACCTG TATATATGCT GTCATTGGGA TTCATAGCGT TAGGAGCAGC AGTTAGCATA 5700
   GCAGTAATAG TCTGGGCATT ACTATATAGA GAATATAAGA AAATAAAATT GCAGGAAAAA 5760
   ATAAAACACA TAAGACAGAG AATAAGAGAA AGAGAAGAAG ATAGTGGCAA TGAAAGTGAT 5820
   GGGGATGCAG AGTGGTTGGA TGGGGATGAA GAGTGGTTGG TTACTCTTCT ATCTTCTAGT 5880
   AAGCTTGATC AAGGTAATTG GGTCTGAACA ACATTGGGTA ACAGTGTACT ATGGGGTACC 5940
   AGTATGGAGA GAAGCAGAGA CAACTCTTTT CTGTGCTTCA GATGCTAAAG CCCATAGTAC 6000
   AGAGGCTCAC AACATCTGGG CCACACAAGC ATGTGTTCCT ACTGATCCCA ATCCACAAGA 6060
   AGTGCTATTA CCCAATGTAA CTGAAAAATT TAATATGTGG GAAAATAAAA TGGCAGACCA 6120
   AATGCAAGAG GATATTATCA GTCTGTGGGA ACAGAGCTTA AAGCCCTGTG TTAAATTAAC 6180
   CCCATTATGT GTAACTATGC TTTGTAACGA TAGCTATGGG GAGGAAAGGA ACAATACAAA 6240
   TATGACAACA AGAGAACCAG ACATAGGATA CAAACAAATG AAAAATTGCT CATTCAATGC 6300
   AACCACTGAG CTAACAGATA AAAAGAAGCA AGTTTACTCT CTGTTTTATG TAGAAGATGT 6360
   AGTACCAATC AATGCCTATA ATAAAACATA TAGGCTAATA AATTGTAATA CCACAGCTGT 6420
   GACACAAGCT TGTCCTAAGA CTTCCTTTGA GCCAATTCCA ATACATTACT GTGCACCACC 6480
   AGGCTTTGCC ATTATGAAAT GTAATGAAGG AAACTTTAGT GGAAATGGAA GCTGTACAAA 6540
   TGTGAGTACT GTACAATGCA CACATGGAAT AAAGCCAGTG ATATCCACTC AGTTAATCCT 6600
   AAATGGAAGC TTAAATACAG ATGGAATTGT TATTAGAAAT GATAGTCACA GTAATCTGTT 6660
   GGTGCAATGG AATGAGACAG TGCCAATAAA TTGTACAAGG CCAGGAAATA ATACAGGAGG 6720
   ACAGGTGCAG ATAGGACCTG CTATGACATT TTATAACATA GAAAAAATAG TAGGAGACAT 6780
   TAGACAAGCA TACTGTAATG TCTCTAAAGA ACTATGGGAA CCAATGTGGA ATAGAACAAG 6840
   AGAGGAAATA AAGAAAATCC TGGGGAAAAA CAACATAACC TTCAGGGCTC GAGAGAGGAA 6900
   TGAAGGAGAC CTAGAAGTGA CACACTTAAT GTTCAATTGT AGAGGAGAGT TTTTCTATTG 6960
   TAACACTTCC AAATTATTTA ATGAGGAATT ACTTAACGAG ACAGGTGAGC CTATTACTCT 7020
   GCCTTGTAGA ATAAGACAGA TTGTAAATTT GTGGACAAGG GTAGGAAAAG GAATTTATGC 7080
   ACCACCAATT CGGGGAGTTC TTAACTGTAC CTCCAATATT ACTGGACTGG TTCTAGAATA 7140
   TAGTGGTGGG CCTGACACCA AGGAAACAAT AGTATATCCC TCAGGAGGAA ACATGGTTAA 7200
   TCTCTGGAGA CAAGAGTTGT ATAAGTACAA AGTAGTTAGC ATAGAACCCA TAGGAGTAGC 7260
   ACCAGGTAAA GCTAAAAGAC GCACAGTGAG TAGAGAAAAA AGAGCAGCCT TTGGACTAGG 7320
   TGCGCTGTTT CTTGGGTTTC TTGGAGCAGC AGGGAGCACT ATGGGCGCAG CGTCAATAAC 7380
   GCTGACGGTA CAGGCCCGGA CATTATTATC TGGGATAGTG CAACAGCAGA ATATTCTGTT 7440
   GAGAGCAATA GAGGCGCAAC AACATTTGTT GCAACTCTCA ATCTGGGGCA TTAAACAGCT 7500
   CCAGGCAAAA GTCCTTGCTA TAGAAAGATA CCTTAGGGAT CAGCAAATCC TAAGTCTATG 7560
   GGGCTGCTCA GGAAAAACAA TATGCTATAC CACTGTGCCT TGGAATGAGA CTTGGAGCAA 7620
   CAATACCTCT TATGATACAA TCTGGAATAA TTTAACCTGG CAACAATGGG ATGAGAAAGT 7680
   AAGAAACTAT TCAGGTGTCA TTTTTGGACT TATAGAACAG GCACAAGAAC AACAGAACAC 7740
   AAATGAGAAA TCACTCTTGG AATTGGATCA ATGGGACAGT CTGTGGAGCT GGTTTGGTAT 7800
   TACAAAATGG CTGTGGTATA TAAAAATAGC TATAATGATA GTAGCAGGCA TTGTAGGCAT 7860
   AAGAATCATA AGTATAGTAA TAACTATAAT AGCAAGAGTT AGGCAGGGAT ATTCTCCCCT 7920
   TTCGTTGCAG ACCCTTATCC CAACAGCAAG GGGACCAGAC AGGCCAGAAG AAACAGAAGG 7980
   AGGCGTTGGA GAGCAAGACA GAGGCAGATC CGTGCGATTA GTGAGCGGAT TCTCAGCTCT 8040
   TGTCTGGGAG GACCTCCGGA ACCTGTTGAT CTTCCTCTAC CACCGCTTGA CAGACTCACT 8100
   CTTGATACTG AGGAGGACTC TGGAACTCCT GGGACAGAGT CTCAGCAGGG GACTGCAACT 8160
   ACTGAATGAA CTCAGAACAC ACTTGTGGGG AATACTTGCA TATTGGGGAA AAGAGTTAAG 8220
   GGATAGTGCT ATCAGCTTGC TTAATACAAC AGCTATTGTA GTAGCAGAAG GAACAGATAG 8280
   GATTATAGAA TTAGCACAAA GAATAGGAAG GGGAATATTA CACATACCTA GAAGAATCAG 8340
   ACAAGGCCTA GAAAGAGCAC TGATATAAGA TGGGAAAGAT TTGGTCAAAG AGCAGCCTAG 8400
   TAGGATGGCC AGAAATCAGA GAAAGAATGA GAAGACAAAC GCAAGAACCA GCAGTAGAGC 8460
   CAGCAGTAGG AGCAGGAGCA GCTTCTCAAG ATCTAGCTAA TCGAGGGGCC ATCACCATAA 8520
   GAAATACTAG AGACAATAAT GAAAGTATAG CTTGGCTAGA AGCACAAGAA GAAGAAGAGG 8580
   AAGTAGGCTT TCCAGTACGC CCTCAGGTAC CATTAAGGCC AATAACCTAT AAACAGGCTT 8640
   TTGATCTTTC CTTCTTTTTA AAAGATAAGG GGGGACTGGA AGGGCTAGTT TGGTCCAGAA 8700
   AAAGGCAAGA TATTCTAGAC CTCTGGATGT ATCACACACA AGGCATCCTC CCTGACTGGC 8760
   ATAACTACAC ACCAGGGCCA GGAATTAGAT ACCCCGTAAC CTTTGGATGG TGCTTCAAAC 8820
   TAGTACCATT GTCAGCTGAA GAAGTAGAAG AGGCTAATGA AGGAGACAAC AATGCCCTCT 8880
   TACACCCCAT ATGTCAACAT GGAGCAGATG ATGATCATAA AGAAGTGTTG GTGTGGCGAT 8940
   TTGACAGCTC CCTAGCAAGA AGACATGTAG CAAGAGAGCT GCATCCGGAG TTTTACAAGA 9000
   ACTGCTGACA AGGGACTTTA CTGCTGACAA GGGACTTTAT ACTTGGGGAC TTTCCGCCAG 9060
   GGACTTTCCA GGGAGGTGTG GTTGGGGGAG TGGCTTGCCC TCAGAGCTGC ATAAAAGCAG 9120
   CCGCTTCTCG CTTGTACTGG GTCTCTCTTG CTGGACCAGA TTAGAGTCTG GGAGCATATT 9180
   GGG 9183
(2) INFORMATIONS POUR LA SEQ ID NO: 2:
   (i) CARACTERISTIQUES DE LA SEQUENCE:
      (A) LONGUEUR: 813 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 2:
   TTGGAAGGGC TAGTTTGGTC CAGAAAAAGG CAAGATATTC TAGACCTCTG GATGTATCAC   60
   ACACAAGGCA TCCTCCCTGA CTGGCATAAC TACACACCAG GGCCAGGAAT TAGATACCCC  120
   GTAACCTTTG GATGGTGCTT CAAACTAGTA CCATTGTCAG CTGAAGAAGT AGAAGAGGCT  180
   AATGAAGGAG ACAACAATGC CCTCTTACAC CCCATATGTC AACATGGAGC AGATGATGAT  240
   CATAAAGAAG TGTTGGTGTG GCGATTTGAC AGCTCCCTAG CAAGAAGACA TGTAGCAAGA  300
   GAGCTGCATC CGGAGTTTTA CAAGAACTGC TGACAAGGGA CTTTACTGCT GACAAGGGAC  360
   TTTATACTTG GGGACTTTCC GCCAGGGACT TTCCAGGGAG GTGTGGTTGG GGGAGTGGCT  420
   TGCCCTCAGA GCTGCATAAA AGCAGCCGCT TCTCGCTTGT ACTGGGTCTC TCTTGCTGGA  480
   CTATACAGAT TAGAGCCTGG GAGCTCTCTG GCTAGCAGGG AACCCACTGC TTAAGCCTCA  540
   ATAAATACAG CTTGCCTTGA GTGCTAAAGT GGTGTGTGCC CATCCATTCG GTAACTCTGG  600
   TACCTAGAGA ATCCCTCAGA CCATCTAGAC TGAGTGAAAA ATCTCTAGCA GTGGCGCCCG  660
   AACAGGGACT TAGTTGAAAA CGAAAGTAGA ACCGGAGGCT GAATCTCTCG ACGCAGGACT  720
   CGGCTCGTTG GTGCACACAG CGAGAGGCGA GGCGGCGGAA GTGTGAGTAC GCAATTTTGA  780
   CTGGCGGTGG CCAGAAAGTA GGAGAGAGGG AGG                               813
(2) INFORMATIONS POUR LA SEQ ID NO: 3:
   (i) CARACTERISTIQUES DE LA SEQUENCE:
      (A) LONGUEUR: 1539 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: CDS
      (B) EMPLACEMENT:1..1536
   (xi) DESCRIPTION DE LA SEQUENCE: SEQ ID NO: 3:
   ATG GGT GCG AGA GCG TCA GTG TTA ACA GGG GGA AAA TTA GAT CAA TGG       48
   Met Gly Ala Arg Ala Ser Val Leu Thr Gly Gly Lys Leu Asp Gln Trp
    1               5                  10                  15
   GAA TCA ATT TAT TTG AGA CCA GGG GGA AAG AAA AAA TAC AGA ATG AAA       96
   Glu Ser Ile Tyr Leu Arg Pro Gly Gly Lys Lys Lys Tyr Arg Met Lys
               20                  25                  30
   CAT TTA GTA TGG GCA AGC AGG GAG CTG GAA AGA TTC GCT TGT AAC CCA     144
   His Leu Val Trp Ala Ser Arg Glu Leu Glu Arg Phe Ala Cys Asn Pro
            35                  40                  45
   GGT CTC ATG GAC ACA GCG GAC GGC TGT GCC AAG TTA CTA AAT CAA TTA     192
   Gly Leu Met Asp Thr Ala Asp Gly Cys Ala Lys Leu Leu Asn Gln Leu
        50                  55                  60
   GAA CCA GCT CTC AAG ACA GGG TCA GAA GAA CTG CGC TCT TTA TAT AAC     240
   Glu Pro Ala Leu Lys Thr Gly Ser Glu Glu Leu Arg Ser Leu Tyr Asn
    65                  70                  75                  80
   GCT CTA GCA GTT CTT TAT TGT GTC CAT AGT AGG ATA CAG ATA CAC AAC     288
   Ala Leu Ala Val Leu Tyr Cys Val His Ser Arg Ile Gln Ile His Asn
                    85                  90                  95
   ACA CAG GAA GCT TTG GAC AAG ATA AAA GAG AAA CAG GAA CAG CAC AAG     336
   Thr Gln Glu Ala Leu Asp Lys Ile Lys Glu Lys Gln Glu Gln His Lys
               100                 105                 110
   CCC GAG CCA AAA AAC CCA GAA GCA GGG GCA GCG GCA GCA ACT GAT AGC     384
   Pro Glu Pro Lys Asn Pro Glu Ala Gly Ala Ala Ala Ala Thr Asp Ser
           115                 120                 125
   AAT ATC AGT AGG AAT TAT CCT CTA GTC CAG ACT GCT CAA GGA CAA ATG     432
   Asn Ile Ser Arg Asn Tyr Pro Leu Val Gln Thr Ala Gln Gly Gln Met
       130                 135                 140
   GTA CAT CAG CCG CTG ACA CCC AGA ACC TTA AAT GCT TGG GTG AAA GTG     480
   Val His Gln Pro Leu Thr Pro Arg Thr Leu Asn Ala Trp Val Lys Val
   145                 150                 155                 160
   ATA GAG GAG AAG GCC TTT AGT CCA GAA GTA ATA CCA ATG TTT ATG GCC     528
   Ile Glu Glu Lys Ala Phe Ser Pro Glu Val Ile Pro Met Phe Met Ala
                   165                 170                 175
   TTG TCA GAA GGG GCA ACG CCC TCA GAT CTA AAT ACT ATG TTA AAT ACA     576
   Leu Ser Glu Gly Ala Thr Pro Ser Asp Leu Asn Thr Met Leu Asn Thr
               180                 185                 190
   GTA GGG GGA CAT CAG GCA GCA ATG CAG ATG CTG AAG GAA GTC ATC AAT     624
   Val Gly Gly His Gln Ala Ala Met Gln Met Leu Lys Glu Val Ile Asn
           195                 200                 205
   GAG GAA GCA GCA GAC TGG GAT AGG ACA CAT CCA GTC CCT GTG GGA CCA     672
   Glu Glu Ala Ala Asp Trp Asp Arg Thr His Pro Val Pro Val Gly Pro
       210                 215                 220
   CTA CCC CCA GGG CAA CTG AGA GAC CCT AGA GGA AGT GAT ATA GCA GGA     720
   Leu Pro Pro Gly Gln Leu Arg Asp Pro Arg Gly Ser Asp Ile Ala Gly
   225                 230                 235                 240
   ACA ACT AGC ACC CTG GCA GAA CAG GTG GCT TGG ATG ACT GCT AAT CCT     768
   Thr Thr Ser Thr Leu Ala Glu Gln Val Ala Trp Met Thr Ala Asn Pro
                   245                 250                 255
   CCT GTT CCA GTA GGA GAT ATT TAT AGA AGA TGG ATA GTC CTG GGG TTA     816
   Pro Val Pro Val Gly Asp Ile Tyr Arg Arg Trp Ile Val Leu Gly Leu
               260                 265                 270
   AAC AGA ATT GTG AGA ATG TAT AGT CCT GTC AGC ATT CTA GAG ATC AAA     864
   Asn Arg Ile Val Arg Met Tyr Ser Pro Val Ser Ile Leu Glu Ile Lys
           275                 280                 285
   CAA GGA CCA AAA GAA CCC TTC AGA GAC TAT GTA GAC AGG TTC TAC AAA 912
   Gln Gly Pro Lys Glu Pro Phe Arg Asp Tyr Val Asp Arg Phe Tyr Lys
   290 295 300
   ACT CTA AGA GCA GAG CAG GCA ACA CAG GAA GTA AAG AAT TGG ATG ACA 960
   Thr Leu Arg Ala Glu Gln Ala Thr Gln Glu Val Lys Asn Trp Met Thr
   305 310 315 320
   GAA ACA CTC TTA GTA CAA AAT GCA AAC CCA GAT TGT AAA CAG CTC CTA 1008
   Glu Thr Leu Leu Val Gln Asn Ala Asn Pro Asp Cys Lys Gln Leu Leu
   325 330 335
   AAA GCA TTA GGG CCA GGA GCT ACC TTA GAA GAG ATG ATG ACG GCC TGC 1056
   Lys Ala Leu Gly Pro Gly Ala Thr Leu Glu Glu Met Met Thr Ala Cys
   340 345 350
   CAG GGA GTG GGG GGA CCA GCA CAT AAG GCA AGA GTG CTA GCA GAG GCT 1104
   Gln Gly Val Gly Gly Pro Ala His Lys Ala Arg Val Leu Ala Glu Ala
   355 360 365
   ATG TCA CAG GTG CAG CAG CCA ACA ACT AGT GTC TTT GCA CAA AGG GGA 1152
   Met Ser Gln Val Gln Gln Pro Thr Thr Ser Val Phe Ala Gln Arg Gly
   370 375 380
   AAC TTT AAA GGC ATA AGG AAA CCC ATT AAA TGT TTC AAT TGT GGC AAA 1200
   Asn Phe Lys Gly Ile Arg Lys Pro Ile Lys Cys Phe Asn Cys Gly Lys
   385 390 395 400
   GAG GGC CAT TTG GCA AGA AAC TGT AAG GCC CCT AGA AGA GGA GGC TGT 1248
   Glu Gly His Leu Ala Arg Asn Cys Lys Ala Pro Arg Arg Gly Gly Cys
   405 410 415
   TGG AAG TGT GGG CAA GAA GGA CAT CAA ATG AAA GAT TGT AAA AAT GAA 1296
   Trp Lys Cys Gly Gln Glu Gly His Gln Met Lys Asp Cys Lys Asn Glu
   420 425 430
   GGA AGA CAG GCT AAT TTT TTA GGG AAG AGC TGG TCT CCC TTC AAA GGG 1344
   Gly Arg Gln Ala Asn Phe Leu Gly Lys Ser Trp Ser Pro Phe Lys Gly
   435 440 445
   AGA CCA GGA AAC TTC CCC CAG ACA ACA ACA AGG AAA GAG CCC ACA GCC 1392
   Arg Pro Gly Asn Phe Pro Gln Thr Thr Thr Arg Lys Glu Pro Thr Ala
   450 455 460
   CCG CCA CTA GAG AGT TAT GGG TTT CAG GAG GAG AAG AGC ACA CAG GGG 1440
   Pro Pro Leu Glu Ser Tyr Gly Phe Gln Glu Glu Lys Ser Thr Gln Gly
   465 470 475 480
   AAG GAG ATG CAG GAG AAC CAG GAG AGG ACA GAG AAC TCT CTG TAC CCA 1488
   Lys Glu Met Gln Glu Asn Gln Glu Arg Thr Glu Asn Ser Leu Tyr Pro
   485 490 495
   CCT TTA ACT TCC CTC AGA TCA CTC TTT GGC AAC GAC CCG TCA TCA CAG 1536
   Pro Leu Thr Ser Leu Arg Ser Leu Phe Gly Asn Asp Pro Ser Ser Gln
   500 505 510
   TAA 1539
(2) INFORMATIONS POUR LA SEQ ID NO: 4:
   (i) CARACTERISTIQUES DE LA SEQUENCE:
      (A) LONGUEUR: 512 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 4:
   Met Gly Ala Arg Ala Ser Val Leu Thr Gly Gly Lys Leu Asp Gln Trp
   1 5 10 15
   Glu Ser Ile Tyr Leu Arg Pro Gly Gly Lys Lys Lys Tyr Arg Met Lys
   20 25 30
   His Leu Val Trp Ala Ser Arg Glu Leu Glu Arg Phe Ala Cys Asn Pro
   35 40 45
   Gly Leu Met Asp Thr Ala Asp Gly Cys Ala Lys Leu Leu Asn Gln Leu
   50 55 60
   Glu Pro Ala Leu Lys Thr Gly Ser Glu Glu Leu Arg Ser Leu Tyr Asn
   65 70 75 80
   Ala Leu Ala Val Leu Tyr Cys Val His Ser Arg Ile Gln Ile His Asn
   85 90 95
   Thr Gln Glu Ala Leu Asp Lys Ile Lys Glu Lys Gln Glu Gln His Lys
   100 105 110
   Pro Glu Pro Lys Asn Pro Glu Ala Gly Ala Ala Ala Ala Thr Asp Ser
   115 120 125
   Asn Ile Ser Arg Asn Tyr Pro Leu Val Gln Thr Ala Gln Gly Gln Met
   130 135 140
   Val His Gln Pro Leu Thr Pro Arg Thr Leu Asn Ala Trp Val Lys Val
   145 150 155 160
   Ile Glu Glu Lys Ala Phe Ser Pro Glu Val Ile Pro Met Phe Met Ala
   165 170 175
   Leu Ser Glu Gly Ala Thr Pro Ser Asp Leu Asn Thr Met Leu Asn Thr
   180 185 190
   Val Gly Gly His Gln Ala Ala Met Gln Met Leu Lys Glu Val Ile Asn
   195 200 205
   Glu Glu Ala Ala Asp Trp Asp Arg Thr His Pro Val Pro Val Gly Pro
   210 215 220
   Leu Pro Pro Gly Gln Leu Arg Asp Pro Arg Gly Ser Asp Ile Ala Gly
   225 230 235 240
   Thr Thr Ser Thr Leu Ala Glu Gln Val Ala Trp Met Thr Ala Asn Pro
   245 250 255
   Pro Val Pro Val Gly Asp Ile Tyr Arg Arg Trp Ile Val Leu Gly Leu
   260 265 270
   Asn Arg Ile Val Arg Met Tyr Ser Pro Val Ser Ile Leu Glu Ile Lys
   275 280 285
   Gln Gly Pro Lys Glu Pro Phe Arg Asp Tyr Val Asp Arg Phe Tyr Lys
   290 295 300
   Thr Leu Arg Ala Glu Gln Ala Thr Gln Glu Val Lys Asn Trp Met Thr
   305 310 315 320
   Glu Thr Leu Leu Val Gln Asn Ala Asn Pro Asp Cys Lys Gln Leu Leu
   325 330 335
   Lys Ala Leu Gly Pro Gly Ala Thr Leu Glu Glu Met Met Thr Ala Cys
               340                 345                 350
   Gln Gly Val Gly Gly Pro Ala His Lys Ala Arg Val Leu Ala Glu Ala
           355                 360                 365
   Met Ser Gln Val Gln Gln Pro Thr Thr Ser Val Phe Ala Gln Arg Gly
       370                 375                 380
   Asn Phe Lys Gly Ile Arg Lys Pro Ile Lys Cys Phe Asn Cys Gly Lys
   385                 390                 395                 400
   Glu Gly His Leu Ala Arg Asn Cys Lys Ala Pro Arg Arg Gly Gly Cys
                   405                 410                 415
   Trp Lys Cys Gly Gln Glu Gly His Gln Met Lys Asp Cys Lys Asn Glu
               420                 425                 430
   Gly Arg Gln Ala Asn Phe Leu Gly Lys Ser Trp Ser Pro Phe Lys Gly
           435                 440                 445
   Arg Pro Gly Asn Phe Pro Gln Thr Thr Thr Arg Lys Glu Pro Thr Ala
       450                 455                 460
   Pro Pro Leu Glu Ser Tyr Gly Phe Gln Glu Glu Lys Ser Thr Gln Gly
   465                 470                 475                 480
   Lys Glu Met Gln Glu Asn Gln Glu Arg Thr Glu Asn Ser Leu Tyr Pro
                   485                 490                 495
   Pro Leu Thr Ser Leu Arg Ser Leu Phe Gly Asn Asp Pro Ser Ser Gln
               500                 505                 510
(2) INFORMATIONS POUR LA SEQ ID NO: 5:
   (i) CARACTERISTIQUES DE LA SEQUENCE:
      (A) LONGUEUR: 3045 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: CDS
      (B) EMPLACEMENT:1..3042
   (xi) DESCRIPTION DE LA SEQUENCE: SEQ ID NO: 5:
   TTT TTT AGG GAA GAG CTG GTC TCC CTT CAA AGG GAG ACC AGG AAA CTT      48
   Phe Phe Arg Glu Glu Leu Val Ser Leu Gln Arg Glu Thr Arg Lys Leu
           515                 520                 525
   CCC CCA GAC AAC AAC AAG GAA AGA GCC CAC AGC CCC GCC ACT AGA GAG      96
   Pro Pro Asp Asn Asn Lys Glu Arg Ala His Ser Pro Ala Thr Arg Glu
       530                 535                 540
   TTA TGG GTT TCA GGA GGA GAA GAG CAC ACA GGG GAA GGA GAT GCA GGA     144
   Leu Trp Val Ser Gly Gly Glu Glu His Thr Gly Glu Gly Asp Ala Gly
   545                 550                 555                 560
   GAA CCA GGA GAG GAC AGA GAA CTC TCT GTA CCC ACC TTT AAC TTC CCT     192
   Glu Pro Gly Glu Asp Arg Glu Leu Ser Val Pro Thr Phe Asn Phe Pro
                   565                     570                     575
   CAG ATC ACT CTT TGG CAA CGA CCC GTC ATC ACA GTA AAA ATA GGG AAA     240
   Gln Ile Thr Leu Trp Gln Arg Pro Val Ile Thr Val Lys Ile Gly Lys
               580                     585                     590
   GAA GTA AGA GAA GCT CTT TTA GAT ACA GGA GCT GAT GAT ACA GTA ATA     288
   Glu Val Arg Glu Ala Leu Leu Asp Thr Gly Ala Asp Asp Thr Val Ile
           595                     600                     605
   GAA GAG CTA CAA TTA GAG GGA AAA TGG AAA CCA AAA ATG ATA GGA GGA     336
   Glu Glu Leu Gln Leu Glu Gly Lys Trp Lys Pro Lys Met Ile Gly Gly
       610                     615                     620
   ATT GGA GGA TTT ATC AAA GTG AGA CAA TAT GAT AAT ATA ACA GTA GAC     384
   Ile Gly Gly Phe Ile Lys Val Arg Gln Tyr Asp Asn Ile Thr Val Asp
   625                     630                     635                     640
   ATA CAG GGA AGA AAA GCA GTT GGT ACA GTA TTA GTA GGA CCA ACA CCT     432
   Ile Gln Gly Arg Lys Ala Val Gly Thr Val Leu Val Gly Pro Thr Pro
                   645                     650                     655
   GTT AAT ATT ATA GGA AGA AAT CTT TTA ACC CAG ATT GGC TGT ACT TTA     480
   Val Asn Ile Ile Gly Arg Asn Leu Leu Thr Gln Ile Gly Cys Thr Leu
               660                     665                     670
   AAT TTT CCA ATA AGT CCT ATT GAA ACT GTA CCA GTA AAA TTA AAA CCA     528
   Asn Phe Pro Ile Ser Pro Ile Glu Thr Val Pro Val Lys Leu Lys Pro
           675                     680                     685
   GGA ATG GAT GGC CCA AAG GTA AAA CAA TGG CCT TTG ACA ACA GAA AAA     576
   Gly Met Asp Gly Pro Lys Val Lys Gln Trp Pro Leu Thr Thr Glu Lys
       690                     695                     700
   ATA GAG GCA TTA AGA GAA ATT TGT ACA GAA ATG GAA AAG GAA GGA AAA     624
   Ile Glu Ala Leu Arg Glu Ile Cys Thr Glu Met Glu Lys Glu Gly Lys
   705                     710                     715                     720
   ATT TCT AGA ATA GGG CCT GAG AAT CCA TAT AAC ACT CCA ATT TTT GCT     672
   Ile Ser Arg Ile Gly Pro Glu Asn Pro Tyr Asn Thr Pro Ile Phe Ala
                   725                     730                     735
   ATA AAA AAG AAA GAT AGC ACT AAA TGG AGA AAA TTA GTA GAT TTC AGG     720
   Ile Lys Lys Lys Asp Ser Thr Lys Trp Arg Lys Leu Val Asp Phe Arg
               740                     745                     750
   GAA TTA AAT AAA AGG ACC CAA GAT TTT TGG GAA GTG CAG CTA GGA ATT     768
   Glu Leu Asn Lys Arg Thr Gln Asp Phe Trp Glu Val Gln Leu Gly Ile
           755                     760                     765
   CCA CAT CCA GCA GGA TTA AAG CAG AAA AAA TCA GTG ACA GTT TTG GAT     816
   Pro His Pro Ala Gly Leu Lys Gln Lys Lys Ser Val Thr Val Leu Asp
       770                     775                     780
   GTA GGA GAT GCT TAT TTT TCA TGT CCC TTG GAC AAA GAT TTT AGA AAG     864
   Val Gly Asp Ala Tyr Phe Ser Cys Pro Leu Asp Lys Asp Phe Arg Lys
   785                     790                     795                     800
   TAT ACA GCT TTT ACC ATA CCT AGT ATA AAC AAT GAG ACA CCT GGT ATT     912
   Tyr Thr Ala Phe Thr Ile Pro Ser Ile Asn Asn Glu Thr Pro Gly Ile
                   805                     810                     815
   AGA TAC CAG TAT AAT GTG CTG CCA CAA GGC TGG AAA GGG TCA CCA GCA    960
   Arg Tyr Gln Tyr Asn Val Leu Pro Gln Gly Trp Lys Gly Ser Pro Ala
               820                 825                 830
   ATT TTT CAG AGT ACA ATG ACA AAA ATT CTA GAA CCA TTC AGA GAG AAA   1008
   Ile Phe Gln Ser Thr Met Thr Lys Ile Leu Glu Pro Phe Arg Glu Lys
           835                 840                 845
   CAT CCA GAG ATA ATC ATT TAC CAG TAC ATG GAT GAC CTC TAT GTG GGA   1056
   His Pro Glu Ile Ile Ile Tyr Gln Tyr Met Asp Asp Leu Tyr Val Gly
       850                 855                 860
   TCT GAC TTA GAA CTA GCA CAA CAT AGA GAG GCA GTA GAA GAC CTC AGA   1104
   Ser Asp Leu Glu Leu Ala Gln His Arg Glu Ala Val Glu Asp Leu Arg
   865                 870                 875                 880
   GAT CAT CTT TTG AAG TGG GGC TTT ACG ACC CCT GAC AAA AAA CAT CAG   1152
   Asp His Leu Leu Lys Trp Gly Phe Thr Thr Pro Asp Lys Lys His Gln
                   885                 890                 895
   AAG GAG CCC CCG TTC CTC TGG ATG GGA TAT GAA CTC CAT CCA GAC AAA   1200
   Lys Glu Pro Pro Phe Leu Trp Met Gly Tyr Glu Leu His Pro Asp Lys
               900                 905                 910
   TGG ACA GTC CAG CCA ATA AAG TTA CCA GAA AAG GAT GTA TGG ACT GTC   1248
   Trp Thr Val Gln Pro Ile Lys Leu Pro Glu Lys Asp Val Trp Thr Val
           915                 920                 925
   AAT GAT ATA CAG AAA TTA GTA GGA AAG TTA AAT TGG GCA AGT CAG ATC   1296
   Asn Asp Ile Gln Lys Leu Val Gly Lys Leu Asn Trp Ala Ser Gln Ile
       930                 935                 940
   TAT CCA GGA ATC AGA GTA AAA CAG CTC TGT AAA TTA ATC AGA GGA GCC   1344
   Tyr Pro Gly Ile Arg Val Lys Gln Leu Cys Lys Leu Ile Arg Gly Ala
   945                 950                 955                 960
   AGA GCT TTG ACA GAA GTA GTC AAC TTT ACA GAA GAA GCA GAA TTA GAA   1392
   Arg Ala Leu Thr Glu Val Val Asn Phe Thr Glu Glu Ala Glu Leu Glu
                   965                 970                 975
   CTA GCA GAA AAC AGG GAG ATA TTA AAA GAA CCC CTG CAT GGA GTC TAT   1440
   Leu Ala Glu Asn Arg Glu Ile Leu Lys Glu Pro Leu His Gly Val Tyr
               980                 985                 990
   TAT GAC CCA GGA AAA GAA TTA GTA GCA GAA ATT CAA AAG CAA GGA CAA   1488
   Tyr Asp Pro Gly Lys Glu Leu Val Ala Glu Ile Gln Lys Gln Gly Gln
           995                1000                1005
   GGT CAG TGG ACA TAT CAG ATT TAT CAG GAG TTA CAT AAA AAT TTA AAA   1536
   Gly Gln Trp Thr Tyr Gln Ile Tyr Gln Glu Leu His Lys Asn Leu Lys
      1010                1015                1020
   ACA GGA AAG TAT GCA AAA ATG AGA TCT GCC CAT ACT AAT GAT ATA AAA   1584
   Thr Gly Lys Tyr Ala Lys Met Arg Ser Ala His Thr Asn Asp Ile Lys
   1025               1030                1035                1040
   CAG TTA GTT GAA GTG GTA AGG AAA GTG GCA ACA GAA AGT ATA GTA ATT   1632
   Gln Leu Val Glu Val Val Arg Lys Val Ala Thr Glu Ser Ile Val Ile
                  1045                1050                1055
   TGG GGA AAG ACT CCT AAA TTT AGA TTA CCA GTA CAA AAG GAA GTG TGG   1680
   Trp Gly Lys Thr Pro Lys Phe Arg Leu Pro Val Gln Lys Glu Val Trp
              1060                1065                1070
   GAG GCA TGG TGG ACC GAT CAT TGG CAA GCA ACT TGG ATT CCT GAG TGG   1728
   Glu Ala Trp Trp Thr Asp His Trp Gln Ala Thr Trp Ile Pro Glu Trp
           1075                1080                1085
   GAA TTT GTC AAC ACT CCT CCC CTT GTA AAA TTA TGG TAT CAG TTA GAA   1776
   Glu Phe Val Asn Thr Pro Pro Leu Val Lys Leu Trp Tyr Gln Leu Glu
       1090                1095                1100
   ACA GAG CCA ATC AGT GGG GCA GAA ACT TTC TAT GTA GAT GGA GCA GCT   1824
   Thr Glu Pro Ile Ser Gly Ala Glu Thr Phe Tyr Val Asp Gly Ala Ala
   1105                1110                1115                1120
   AAT AGG GAA ACA AAA TTG GGA AAA GCA GGT TTT GTG ACA GAT AGG GGA   1872
   Asn Arg Glu Thr Lys Leu Gly Lys Ala Gly Phe Val Thr Asp Arg Gly
                   1125                1130                1135
   AGA CAG AAA GTG GTC TCT ATT GCA GAC ACC ACC AAT CAA AAG GCT GAG   1920
   Arg Gln Lys Val Val Ser Ile Ala Asp Thr Thr Asn Gln Lys Ala Glu
               1140                1145                1150
   TTA CAA GCT ATC CTT ATG GCC TTA CAA GAG TCA GGA CGG GAT GTA AAC   1968
   Leu Gln Ala Ile Leu Met Ala Leu Gln Glu Ser Gly Arg Asp Val Asn
           1155                1160                1165
   ATA GTC ACT GAC TCT CAG TAT GCT ATG GGA ATA ATT CAT TCA CAG CCA   2016
   Ile Val Thr Asp Ser Gln Tyr Ala Met Gly Ile Ile His Ser Gln Pro
       1170                1175                1180
   GAT AAA AGT GAA TCA GAA TTG GTG AGC CAA ATA ATA GAA GAG CTC ATA   2064
   Asp Lys Ser Glu Ser Glu Leu Val Ser Gln Ile Ile Glu Glu Leu Ile
   1185                1190                1195                1200
   AAA AAG GAA AGA GTT TAT CTC TCT TGG GTA CCT GCA CAT AAA GGT ATT   2112
   Lys Lys Glu Arg Val Tyr Leu Ser Trp Val Pro Ala His Lys Gly Ile
                   1205                1210                1215
   GGA GGA AAT GAG CAG GTA GAC AAA TTA GTT AGC TCA GGA ATT AGA AAA   2160
   Gly Gly Asn Glu Gln Val Asp Lys Leu Val Ser Ser Gly Ile Arg Lys
               1220                1225                1230
   ATA TTA TTC CTA GAT GGT ATA GAA AAA GCC CAA GAA GAT CAT GAC AGA   2208
   Ile Leu Phe Leu Asp Gly Ile Glu Lys Ala Gln Glu Asp His Asp Arg
           1235                1240                1245
   TAT CAC AGC AAT TGG AAA GCA ATG GCC AGT GAT TTT AAC TTA CCC CCC   2256
   Tyr His Ser Asn Trp Lys Ala Met Ala Ser Asp Phe Asn Leu Pro Pro
       1250                1255                1260
   ATA GTG GCA AAA GAA ATA GTA GCC AGC TGT GAC AAA TGC CAG CTA AAA   2304
   Ile Val Ala Lys Glu Ile Val Ala Ser Cys Asp Lys Cys Gln Leu Lys
   1265                1270                1275                1280
   GGG GAA GCC ATG CAT GGA CAG GTC AAT TGT AGT CCA GGA GTG TGG CAA   2352
   Gly Glu Ala Met His Gly Gln Val Asn Cys Ser Pro Gly Val Trp Gln
                   1285                1290                1295
   TTA GAT TGT ACA CAC TTA GAG GGA AAA ATC ATC CTT GTG GCG GTC CAT   2400
   Leu Asp Cys Thr His Leu Glu Gly Lys Ile Ile Leu Val Ala Val His
               1300                1305                1310
   GTG GCC AGT GGC TAC TTA GAA GCA GAA GTT ATT CCT GCA GAG ACA GGA   2448
   Val Ala Ser Gly Tyr Leu Glu Ala Glu Val Ile Pro Ala Glu Thr Gly
           1315                1320                1325
   CAG GAA ACA GCA TAT TTT ATT TTA AAG TTA GCT GGA AGA TGG CCA GTA   2496
   Gln Glu Thr Ala Tyr Phe Ile Leu Lys Leu Ala Gly Arg Trp Pro Val
       1330                1335                1340
   AAA GTT ATA CAC ACT GAT AAT GGA TCC AAT TTC ACT AGT GCC ACT GTA   2544
   Lys Val Ile His Thr Asp Asn Gly Ser Asn Phe Thr Ser Ala Thr Val
   1345                1350                1355                1360
   AAA GCA GCC TGT TGG TGG GCA AAT ATC AAA CAG GAA TTT GGG ATA CCC   2592
   Lys Ala Ala Cys Trp Trp Ala Asn Ile Lys Gln Glu Phe Gly Ile Pro
                   1365                1370                1375
   TAC AAT CCT CAA AGT CAG GGA GCA GTA GAG TCC ATG AAT AAA GAA TTA   2640
   Tyr Asn Pro Gln Ser Gln Gly Ala Val Glu Ser Met Asn Lys Glu Leu
               1380                1385                1390
   AAG AAA ATT ATA GGA CAA ATC AGA GAT CAA GCA GAA CAT CTA AAG ACA   2688
   Lys Lys Ile Ile Gly Gln Ile Arg Asp Gln Ala Glu His Leu Lys Thr
           1395                1400                1405
   GCA GTG CAA ATG GCG GTT TTC ATT CAC AAT TTT AAA AGA AAA GGG GGG   2736
   Ala Val Gln Met Ala Val Phe Ile His Asn Phe Lys Arg Lys Gly Gly
       1410                1415                1420
   ATT GGG GGG TAC ACT GCA GGG GAA AGA ATA ATA GAC ATA ATA GCA ACA   2784
   Ile Gly Gly Tyr Thr Ala Gly Glu Arg Ile Ile Asp Ile Ile Ala Thr
   1425                1430                1435                1440
   GAC ATA CAG ACA ACA AAT TTA CAA ACA CAA ATT TTA AAA GTT CAA AAT   2832
   Asp Ile Gln Thr Thr Asn Leu Gln Thr Gln Ile Leu Lys Val Gln Asn
                   1445                1450                1455
   TTT CGG GTT TAT TAC AGA GAC AGC AGA GAT CCC ATT TGG AAA GGA CCA   2880
   Phe Arg Val Tyr Tyr Arg Asp Ser Arg Asp Pro Ile Trp Lys Gly Pro
               1460                1465                1470
   GCC AAA CTT CTG TGG AAA GGA GAA GGG GCA GTG GTA ATT CAA GAT AAC   2928
   Ala Lys Leu Leu Trp Lys Gly Glu Gly Ala Val Val Ile Gln Asp Asn
           1475                1480                1485
   GGG GAT ATA AAA GTA GTC CCA CGT AGG AAA GCA AAA ATA ATT AGG GAT   2976
   Gly Asp Ile Lys Val Val Pro Arg Arg Lys Ala Lys Ile Ile Arg Asp
       1490                1495                1500
   TAT GGA AAA CAG ATG GCA GGT GAT GGT TGT GTG GCA AGT GGA CAG GAT   3024
   Tyr Gly Lys Gln Met Ala Gly Asp Gly Cys Val Ala Ser Gly Gln Asp
   1505                1510                1515                1520
   GAA AAT CAG GAA ATG GAA TAG                                       3045
   Glu Asn Gln Glu Met Glu
                   1525
(2) INFORMATIONS POUR LA SEQ ID NO: 6:
   (i) CARACTERISTIQUES DE LA SEQUENCE:
      (A) LONGUEUR: 1014 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 6:
   Phe Phe Arg Glu Glu Leu Val Ser Leu Gln Arg Glu Thr Arg Lys Leu
   1 5 10 15
   Pro Pro Asp Asn Asn Lys Glu Arg Ala His Ser Pro Ala Thr Arg Glu
               20                  25                  30
   Leu Trp Val Ser Gly Gly Glu Glu His Thr Gly Glu Gly Asp Ala Gly
           35                  40                  45
   Glu Pro Gly Glu Asp Arg Glu Leu Ser Val Pro Thr Phe Asn Phe Pro
       50                  55                  60
   Gln Ile Thr Leu Trp Gln Arg Pro Val Ile Thr Val Lys Ile Gly Lys
   65                  70                  75                  80
   Glu Val Arg Glu Ala Leu Leu Asp Thr Gly Ala Asp Asp Thr Val Ile
                   85                  90                  95
   Glu Glu Leu Gln Leu Glu Gly Lys Trp Lys Pro Lys Met Ile Gly Gly
               100                 105                 110
   Ile Gly Gly Phe Ile Lys Val Arg Gln Tyr Asp Asn Ile Thr Val Asp
           115                 120                 125
   Ile Gln Gly Arg Lys Ala Val Gly Thr Val Leu Val Gly Pro Thr Pro
       130                 135                 140
   Val Asn Ile Ile Gly Arg Asn Leu Leu Thr Gln Ile Gly Cys Thr Leu
   145                 150                 155                 160
   Asn Phe Pro Ile Ser Pro Ile Glu Thr Val Pro Val Lys Leu Lys Pro
                   165                 170                 175
   Gly Met Asp Gly Pro Lys Val Lys Gln Trp Pro Leu Thr Thr Glu Lys
               180                 185                 190
   Ile Glu Ala Leu Arg Glu Ile Cys Thr Glu Met Glu Lys Glu Gly Lys
           195                 200                 205
   Ile Ser Arg Ile Gly Pro Glu Asn Pro Tyr Asn Thr Pro Ile Phe Ala
       210                 215                 220
   Ile Lys Lys Lys Asp Ser Thr Lys Trp Arg Lys Leu Val Asp Phe Arg
   225                 230                 235                 240
   Glu Leu Asn Lys Arg Thr Gln Asp Phe Trp Glu Val Gln Leu Gly Ile
                   245                 250                 255
   Pro His Pro Ala Gly Leu Lys Gln Lys Lys Ser Val Thr Val Leu Asp
               260                 265                 270
   Val Gly Asp Ala Tyr Phe Ser Cys Pro Leu Asp Lys Asp Phe Arg Lys
           275                 280                 285
   Tyr Thr Ala Phe Thr Ile Pro Ser Ile Asn Asn Glu Thr Pro Gly Ile
       290                 295                 300
   Arg Tyr Gln Tyr Asn Val Leu Pro Gln Gly Trp Lys Gly Ser Pro Ala
   305                 310                 315                 320
   Ile Phe Gln Ser Thr Met Thr Lys Ile Leu Glu Pro Phe Arg Glu Lys
                   325                 330                 335
   His Pro Glu Ile Ile Ile Tyr Gln Tyr Met Asp Asp Leu Tyr Val Gly
               340                 345                 350
   Ser Asp Leu Glu Leu Ala Gln His Arg Glu Ala Val Glu Asp Leu Arg
           355                 360                 365
   Asp His Leu Leu Lys Trp Gly Phe Thr Thr Pro Asp Lys Lys His Gln
       370                 375                 380
   Lys Glu Pro Pro Phe Leu Trp Met Gly Tyr Glu Leu His Pro Asp Lys
   385                 390                 395                 400
   Trp Thr Val Gln Pro Ile Lys Leu Pro Glu Lys Asp Val Trp Thr Val
                   405                 410                 415
   Asn Asp Ile Gln Lys Leu Val Gly Lys Leu Asn Trp Ala Ser Gln Ile
               420                 425                 430
   Tyr Pro Gly Ile Arg Val Lys Gln Leu Cys Lys Leu Ile Arg Gly Ala
           435                 440                 445
   Arg Ala Leu Thr Glu Val Val Asn Phe Thr Glu Glu Ala Glu Leu Glu
       450                 455                 460
   Leu Ala Glu Asn Arg Glu Ile Leu Lys Glu Pro Leu His Gly Val Tyr
   465                 470                 475                 480
   Tyr Asp Pro Gly Lys Glu Leu Val Ala Glu Ile Gln Lys Gln Gly Gln
                   485                 490                 495
   Gly Gln Trp Thr Tyr Gln Ile Tyr Gln Glu Leu His Lys Asn Leu Lys
               500                 505                 510
   Thr Gly Lys Tyr Ala Lys Met Arg Ser Ala His Thr Asn Asp Ile Lys
           515                 520                 525
   Gln Leu Val Glu Val Val Arg Lys Val Ala Thr Glu Ser Ile Val Ile
       530                 535                 540
   Trp Gly Lys Thr Pro Lys Phe Arg Leu Pro Val Gln Lys Glu Val Trp
   545                 550                 555                 560
   Glu Ala Trp Trp Thr Asp His Trp Gln Ala Thr Trp Ile Pro Glu Trp
                   565                 570                 575
   Glu Phe Val Asn Thr Pro Pro Leu Val Lys Leu Trp Tyr Gln Leu Glu
               580                 585                 590
   Thr Glu Pro Ile Ser Gly Ala Glu Thr Phe Tyr Val Asp Gly Ala Ala
           595                 600                 605
   Asn Arg Glu Thr Lys Leu Gly Lys Ala Gly Phe Val Thr Asp Arg Gly
       610                 615                 620
   Arg Gln Lys Val Val Ser Ile Ala Asp Thr Thr Asn Gln Lys Ala Glu
   625                 630                 635                 640
   Leu Gln Ala Ile Leu Met Ala Leu Gln Glu Ser Gly Arg Asp Val Asn
                   645                 650                 655
   Ile Val Thr Asp Ser Gln Tyr Ala Met Gly Ile Ile His Ser Gln Pro
               660                 665                 670
   Asp Lys Ser Glu Ser Glu Leu Val Ser Gln Ile Ile Glu Glu Leu Ile
           675                 680                 685
   Lys Lys Glu Arg Val Tyr Leu Ser Trp Val Pro Ala His Lys Gly Ile
       690                 695                 700
   Gly Gly Asn Glu Gln Val Asp Lys Leu Val Ser Ser Gly Ile Arg Lys
   705                 710                 715                 720
   Ile Leu Phe Leu Asp Gly Ile Glu Lys Ala Gln Glu Asp His Asp Arg
                   725                 730                 735
   Tyr His Ser Asn Trp Lys Ala Met Ala Ser Asp Phe Asn Leu Pro Pro
               740                 745                 750
   Ile Val Ala Lys Glu Ile Val Ala Ser Cys Asp Lys Cys Gln Leu Lys
           755                 760                 765
   Gly Glu Ala Met His Gly Gln Val Asn Cys Ser Pro Gly Val Trp Gln
       770                 775                 780
   Leu Asp Cys Thr His Leu Glu Gly Lys Ile Ile Leu Val Ala Val His
   785                 790                 795                 800
   Val Ala Ser Gly Tyr Leu Glu Ala Glu Val Ile Pro Ala Glu Thr Gly
                   805                 810                 815
   Gln Glu Thr Ala Tyr Phe Ile Leu Lys Leu Ala Gly Arg Trp Pro Val
               820                 825                 830
   Lys Val Ile His Thr Asp Asn Gly Ser Asn Phe Thr Ser Ala Thr Val
           835                 840                 845
   Lys Ala Ala Cys Trp Trp Ala Asn Ile Lys Gln Glu Phe Gly Ile Pro
       850                 855                 860
   Tyr Asn Pro Gln Ser Gln Gly Ala Val Glu Ser Met Asn Lys Glu Leu
   865                 870                 875                 880
   Lys Lys Ile Ile Gly Gln Ile Arg Asp Gln Ala Glu His Leu Lys Thr
                   885                 890                 895
   Ala Val Gln Met Ala Val Phe Ile His Asn Phe Lys Arg Lys Gly Gly
               900                 905                 910
   Ile Gly Gly Tyr Thr Ala Gly Glu Arg Ile Ile Asp Ile Ile Ala Thr
           915                 920                 925
   Asp Ile Gln Thr Thr Asn Leu Gln Thr Gln Ile Leu Lys Val Gln Asn
       930                 935                 940
   Phe Arg Val Tyr Tyr Arg Asp Ser Arg Asp Pro Ile Trp Lys Gly Pro
   945                 950                 955                 960
   Ala Lys Leu Leu Trp Lys Gly Glu Gly Ala Val Val Ile Gln Asp Asn
                   965                 970                 975
   Gly Asp Ile Lys Val Val Pro Arg Arg Lys Ala Lys Ile Ile Arg Asp
               980                 985                 990
   Tyr Gly Lys Gln Met Ala Gly Asp Gly Cys Val Ala Ser Gly Gln Asp
           995                 1000                1005
   Glu Asn Gln Glu Met Glu
       1010
(2) INFORMATIONS POUR LA SEQ ID NO: 7:
   (i) CARACTERISTIQUES DE LA SEQUENCE:
      (A) LONGUEUR: 579 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: CDS
      (B) EMPLACEMENT:1..576
   (xi) DESCRIPTION DE LA SEQUENCE: SEQ ID NO: 7:
   ATG GAA AAC AGA TGG CAG GTG ATG GTT GTG TGG CAA GTG GAC AGG ATG      48
   Met Glu Asn Arg Trp Gln Val Met Val Val Trp Gln Val Asp Arg Met
   1015                1020                1025                1030
   AAA ATC AGG AAA TGG AAT AGC TTA GTA AAA CAT CAT ATG TAT GTG TCA      96
   Lys Ile Arg Lys Trp Asn Ser Leu Val Lys His His Met Tyr Val Ser
                   1035                1040                1045
   AAA AAG GCA AAA GGA TGG TAT TAT AGA CAT CAT TAT GAA ACA CAT CAC     144
   Lys Lys Ala Lys Gly Trp Tyr Tyr Arg His His Tyr Glu Thr His His
               1050                1055                1060
   CCA AAA ATA AGT TCA GAA GTA CAT ATC CCA GTA GGT CAG GCA AGA TTA     192
   Pro Lys Ile Ser Ser Glu Val His Ile Pro Val Gly Gln Ala Arg Leu
           1065                1070                1075
   GTG ACA GTC ACT TAT TGG GGG CTA ACA ACA GGA GAA CAG TCT TGG CAT     240
   Val Thr Val Thr Tyr Trp Gly Leu Thr Thr Gly Glu Gln Ser Trp His
       1080                1085                1090
   CTA GGA CAT GGA GTA TCC ATA GAA TGG AGA CTA AGA AAA TAC AAG ACA     288
   Leu Gly His Gly Val Ser Ile Glu Trp Arg Leu Arg Lys Tyr Lys Thr
   1095                1100                1105                1110
   CAA GTT GAT CCT GAA ATG GCA GAC AAG CTA ATA CAT CTT CAT TAT TTT     336
   Gln Val Asp Pro Glu Met Ala Asp Lys Leu Ile His Leu His Tyr Phe
                   1115                1120                1125
   GAT TGT TTT ACA GCC TCT GCC ATA AGG CAA GCG GTC TTA GGG AGA CCA     384
   Asp Cys Phe Thr Ala Ser Ala Ile Arg Gln Ala Val Leu Gly Arg Pro
               1130                1135                1140
   GTA TTA CCT AGG TGT GAA TAT CCA GCA GGG CAC AAA CAG GTA GGC ACC     432
   Val Leu Pro Arg Cys Glu Tyr Pro Ala Gly His Lys Gln Val Gly Thr
           1145                1150                1155
   CTA CAA TAT CTA GCA CTA ACA GCC TGG GTG GGA GCA AAG AAG AGA AAG     480
   Leu Gln Tyr Leu Ala Leu Thr Ala Trp Val Gly Ala Lys Lys Arg Lys
       1160                1165                1170
   CCA CCC TTA CCT AGT GTG ACT AAG CTA ACA GAA GAT AGA TGG AAC GAG     528
   Pro Pro Leu Pro Ser Val Thr Lys Leu Thr Glu Asp Arg Trp Asn Glu
   1175                1180                1185                1190
   CAC CAG AAG ATG CAG GGC CAC AGA GGG AAC CCT ATA ATG AAT GGG CAC     576
   His Gln Lys Met Gln Gly His Arg Gly Asn Pro Ile Met Asn Gly His
                   1195                1200                1205
   TAG                                                                 579
(2) INFORMATIONS POUR LA SEQ ID NO: 8:
   (i) CARACTERISTIQUES DE LA SEQUENCE:
      (A) LONGUEUR: 192 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 8:
   Met Glu Asn Arg Trp Gln Val Met Val Val Trp Gln Val Asp Arg Met
    1               5                  10                  15
   Lys Ile Arg Lys Trp Asn Ser Leu Val Lys His His Met Tyr Val Ser
               20                  25                  30
   Lys Lys Ala Lys Gly Trp Tyr Tyr Arg His His Tyr Glu Thr His His
           35                  40                  45
   Pro Lys Ile Ser Ser Glu Val His Ile Pro Val Gly Gln Ala Arg Leu
       50                  55                  60
   Val Thr Val Thr Tyr Trp Gly Leu Thr Thr Gly Glu Gln Ser Trp His
   65                  70                  75                  80
   Leu Gly His Gly Val Ser Ile Glu Trp Arg Leu Arg Lys Tyr Lys Thr
                   85                  90                  95
   Gln Val Asp Pro Glu Met Ala Asp Lys Leu Ile His Leu His Tyr Phe
               100                 105                 110
   Asp Cys Phe Thr Ala Ser Ala Ile Arg Gln Ala Val Leu Gly Arg Pro
           115                 120                 125
   Val Leu Pro Arg Cys Glu Tyr Pro Ala Gly His Lys Gln Val Gly Thr
       130                 135                 140
   Leu Gln Tyr Leu Ala Leu Thr Ala Trp Val Gly Ala Lys Lys Arg Lys
   145                 150                 155                 160
   Pro Pro Leu Pro Ser Val Thr Lys Leu Thr Glu Asp Arg Trp Asn Glu
                   165                 170                 175
   His Gln Lys Met Gln Gly His Arg Gly Asn Pro Ile Met Asn Gly His
               180                 185                 190
(2) INFORMATIONS POUR LA SEQ ID NO: 9:
   (i) CARACTERISTIQUES DE LA SEQUENCE:
      (A) LONGUEUR: 288 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: CDS
      (B) EMPLACEMENT:1..285
   (xi) DESCRIPTION DE LA SEQUENCE: SEQ ID NO: 9:
   ATG GAA CGA GCA CCA GAA GAT GCA GGG CCA CAG AGG GAA CCC TAT AAT      48
   Met Glu Arg Ala Pro Glu Asp Ala Gly Pro Gln Arg Glu Pro Tyr Asn
           195                 200                 205
   GAA TGG GCA CTA GAA TTA TTA GAA GAA TTA AAA AAT GAA GCT GTG CGC      96
   Glu Trp Ala Leu Glu Leu Leu Glu Glu Leu Lys Asn Glu Ala Val Arg
       210                 215                 220
   CAT TTT CCA AGG ATT TGG CTA CAT GGG TTA GGA CAA CAC ATC TAT AAC     144
   His Phe Pro Arg Ile Trp Leu His Gly Leu Gly Gln His Ile Tyr Asn
   225                 230                 235                 240
   ACA TAT GGA GAC ACC TGG GAG GGG GTA GAG GCA ATT ATC AGG ATA CTA     192
   Thr Tyr Gly Asp Thr Trp Glu Gly Val Glu Ala Ile Ile Arg Ile Leu
                   245                 250                 255
   CAA CAA TTA CTG TTT ATC CAT TAT AGG ATT GGC TGC CAG CAC AGC AGA     240
   Gln Gln Leu Leu Phe Ile His Tyr Arg Ile Gly Cys Gln His Ser Arg
               260                 265                 270
   ATA GGG ATC ACT CCT CAA AGG AGA AGG AAT GGA ACC AGT AGA TCC         285
   Ile Gly Ile Thr Pro Gln Arg Arg Arg Asn Gly Thr Ser Arg Ser
           275                 280                 285
   TAG                                                                 288
(2) INFORMATIONS POUR LA SEQ ID NO: 10:
   (i) CARACTERISTIQUES DE LA SEQUENCE:
      (A) LONGUEUR: 95 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 10:
   Met Glu Arg Ala Pro Glu Asp Ala Gly Pro Gln Arg Glu Pro Tyr Asn
    1               5                  10                  15
   Glu Trp Ala Leu Glu Leu Leu Glu Glu Leu Lys Asn Glu Ala Val Arg
               20                  25                  30
   His Phe Pro Arg Ile Trp Leu His Gly Leu Gly Gln His Ile Tyr Asn
           35                  40                  45
   Thr Tyr Gly Asp Thr Trp Glu Gly Val Glu Ala Ile Ile Arg Ile Leu
       50                  55                  60
   Gln Gln Leu Leu Phe Ile His Tyr Arg Ile Gly Cys Gln His Ser Arg
   65                  70                  75                  80
   Ile Gly Ile Thr Pro Gln Arg Arg Arg Asn Gly Thr Ser Arg Ser
                   85                  90                  95
(2) INFORMATIONS POUR LA SEQ ID NO: 11:
   (i) CARACTERISTIQUES DE LA SEQUENCE:
      (A) LONGUEUR: 252 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: CDS
      (B) EMPLACEMENT:1..249
   (xi) DESCRIPTION DE LA SEQUENCE: SEQ ID NO: 11:
   ATG CTG TCA TTG GGA TTC ATA GCG TTA GGA GCA GCA GTT AGC ATA GCA      48
   Met Leu Ser Leu Gly Phe Ile Ala Leu Gly Ala Ala Val Ser Ile Ala
                   100                 105                 110
   GTA ATA GTC TGG GCA TTA CTA TAT AGA GAA TAT AAG AAA ATA AAA TTG      96
   Val Ile Val Trp Ala Leu Leu Tyr Arg Glu Tyr Lys Lys Ile Lys Leu
               115                 120                 125
   CAG GAA AAA ATA AAA CAC ATA AGA CAG AGA ATA AGA GAA AGA GAA GAA     144
   Gln Glu Lys Ile Lys His Ile Arg Gln Arg Ile Arg Glu Arg Glu Glu
           130                 135                 140
   GAT AGT GGC AAT GAA AGT GAT GGG GAT GCA GAG TGG TTG GAT GGG GAT     192
   Asp Ser Gly Asn Glu Ser Asp Gly Asp Ala Glu Trp Leu Asp Gly Asp
       145                 150                 155
   GAA GAG TGG TTG GTT ACT CTT CTA TCT TCT AGT AAG CTT GAT CAA GGT     240
   Glu Glu Trp Leu Val Thr Leu Leu Ser Ser Ser Lys Leu Asp Gln Gly
   160                 165                 170                 175
   AAT TGG GTC TGA                                                     252
   Asn Trp Val
(2) INFORMATIONS POUR LA SEQ ID NO: 12:
   (i) CARACTERISTIQUES DE LA SEQUENCE:
      (A) LONGUEUR: 83 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 12:
   Met Leu Ser Leu Gly Phe Ile Ala Leu Gly Ala Ala Val Ser Ile Ala
     1               5                  10                  15
   Val Ile Val Trp Ala Leu Leu Tyr Arg Glu Tyr Lys Lys Ile Lys Leu
                20                  25                  30
   Gln Glu Lys Ile Lys His Ile Arg Gln Arg Ile Arg Glu Arg Glu Glu
           35                  40                  45
   Asp Ser Gly Asn Glu Ser Asp Gly Asp Ala Glu Trp Leu Asp Gly Asp
        50                  55                  60
   Glu Glu Trp Leu Val Thr Leu Leu Ser Ser Ser Lys Leu Asp Gln Gly
    65                  70                  75                  80
   Asn Trp Val
(2) INFORMATIONS POUR LA SEQ ID NO: 13:
   (i) CARACTERISTIQUES DE LA SEQUENCE:
      (A) LONGUEUR: 306 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: CDS
      (B) EMPLACEMENT:1..303
   (xi) DESCRIPTION DE LA SEQUENCE: SEQ ID NO: 13:
   ATG GAA CCA GTA GAT CCT AGA TTA GAG CCC TGG AAT CAT CCA GGA AGC      48
   Met Glu Pro Val Asp Pro Arg Leu Glu Pro Trp Asn His Pro Gly Ser
        85                  90                  95
   CAA CCT AAA ACA GCT TGC AAT AAT TGC TAT TGT AAA AGA TGT TGC TAT      96
   Gln Pro Lys Thr Ala Cys Asn Asn Cys Tyr Cys Lys Arg Cys Cys Tyr
   100                 105                 110                 115
   CAC TGC TTA TAT TGC TTC ACA AAG AAA GGC TTA GGC ATC TCA TAT GGC     144
   His Cys Leu Tyr Cys Phe Thr Lys Lys Gly Leu Gly Ile Ser Tyr Gly
                   120                 125                 130
   AGG AAG AAG CGG AGT CAA CGA CGA AGA ACT CCT CAG AGC AGT AAG AGT     192
   Arg Lys Lys Arg Ser Gln Arg Arg Arg Thr Pro Gln Ser Ser Lys Ser
               135                 140                 145
   CAT CAA GAT CTT ATA CCA GAG CAG CCC TTA TCC CAA CAG CAA GGG GAC     240
   His Gln Asp Leu Ile Pro Glu Gln Pro Leu Ser Gln Gln Gln Gly Asp
       150                 155                 160
   CAG ACA GGC CAG AAG AAA CAG AAG GAG GCG TTG GAG AGC AAG ACA GAG     288
   Gln Thr Gly Gln Lys Lys Gln Lys Glu Ala Leu Glu Ser Lys Thr Glu
       165                 170                 175
   GCA GAT CCG TGC GAT TAG                                             306
   Ala Asp Pro Cys Asp
   180
(2) INFORMATIONS POUR LA SEQ ID NO: 14:
   (i) CARACTERISTIQUES DE LA SEQUENCE:
      (A) LONGUEUR: 101 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 14:
   Met Glu Pro Val Asp Pro Arg Leu Glu Pro Trp Asn His Pro Gly Ser
     1               5                  10                  15
   Gln Pro Lys Thr Ala Cys Asn Asn Cys Tyr Cys Lys Arg Cys Cys Tyr
                20                  25                  30
   His Cys Leu Tyr Cys Phe Thr Lys Lys Gly Leu Gly Ile Ser Tyr Gly
            35                  40                  45
   Arg Lys Lys Arg Ser Gln Arg Arg Arg Thr Pro Gln Ser Ser Lys Ser
        50                  55                  60
   His Gln Asp Leu Ile Pro Glu Gln Pro Leu Ser Gln Gln Gln Gly Asp
    65                  70                  75                  80
   Gln Thr Gly Gln Lys Lys Gln Lys Glu Ala Leu Glu Ser Lys Thr Glu
                    85                  90                  95
   Ala Asp Pro Cys Asp
               100
(2) INFORMATIONS POUR LA SEQ ID NO: 15:
   (i) CARACTERISTIQUES DE LA SEQUENCE:
      (A) LONGUEUR: 369 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: CDS
      (B) EMPLACEMENT:1..366
   (xi) DESCRIPTION DE LA SEQUENCE: SEQ ID NO: 15:
   ATG GCA GGA AGA AGC GGA GTC AAC GAC GAA GAA CTC CTC AGA GCA GTA      48
   Met Ala Gly Arg Ser Gly Val Asn Asp Glu Glu Leu Leu Arg Ala Val
               105                 110                 115
   AGA GTC ATC AAG ATC TTA TAC CAG AGC AGT TAT CCC AAC AGC AAG GGG      96
   Arg Val Ile Lys Ile Leu Tyr Gln Ser Ser Tyr Pro Asn Ser Lys Gly
           120                 125                 130
   ACC AGA CAG GCC AGA AGA AAC AGA AGG AGG CGT TGG AGA GCA AGA CAG     144
   Thr Arg Gln Ala Arg Arg Asn Arg Arg Arg Arg Trp Arg Ala Arg Gln
       135                 140                 145
   AGG CAG ATC CGT GCG ATT AGT GAG CGG ATT CTC AGC TCT TGT CTG GGA     192
   Arg Gln Ile Arg Ala Ile Ser Glu Arg Ile Leu Ser Ser Cys Leu Gly
   150                 155                 160                 165
   GGA CCT CCG GAA CCT GTT GAT CTT CCT CTA CCA CCG CTT GAC AGA CTC     240
   Gly Pro Pro Glu Pro Val Asp Leu Pro Leu Pro Pro Leu Asp Arg Leu
                   170                 175                 180
   ACT CTT GAT ACT GAG GAG GAC TCT GGA ACT CCT GGG ACA GAG TCT CAG     288
   Thr Leu Asp Thr Glu Glu Asp Ser Gly Thr Pro Gly Thr Glu Ser Gln
               185                 190                 195
   CAG GGG ACT GCA ACT ACT GAA TGA ACT CAG AAC ACA CTT GTG GGG AAT     336
   Gln Gly Thr Ala Thr Thr Glu  *  Thr Gln Asn Thr Leu Val Gly Asn
           200                 205                 210
   ACT TGC ATA TTG GGG AAA AGA GTT AAG GGA TAG                         369
   Thr Cys Ile Leu Gly Lys Arg Val Lys Gly
       215                 220
(2) INFORMATIONS POUR LA SEQ ID NO: 16:
   (i) CARACTERISTIQUES DE LA SEQUENCE:
      (A) LONGUEUR: 122 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 16:
   Met Ala Gly Arg Ser Gly Val Asn Asp Glu Glu Leu Leu Arg Ala Val
    1               5                  10                  15
   Arg Val Ile Lys Ile Leu Tyr Gln Ser Ser Tyr Pro Asn Ser Lys Gly
               20                  25                  30
   Thr Arg Gln Ala Arg Arg Asn Arg Arg Arg Arg Trp Arg Ala Arg Gln
           35                  40                  45
   Arg Gln Ile Arg Ala Ile Ser Glu Arg Ile Leu Ser Ser Cys Leu Gly
       50                  55                  60
   Gly Pro Pro Glu Pro Val Asp Leu Pro Leu Pro Pro Leu Asp Arg Leu
   65                  70                  75                  80
   Thr Leu Asp Thr Glu Glu Asp Ser Gly Thr Pro Gly Thr Glu Ser Gln
                   85                  90                  95
   Gln Gly Thr Ala Thr Thr Glu  *  Thr Gln Asn Thr Leu Val Gly Asn
               100                 105                 110
   Thr Cys Ile Leu Gly Lys Arg Val Lys Gly
           115                 120
(2) INFORMATIONS POUR LA SEQ ID NO: 17:
   (i) CARACTERISTIQUES DE LA SEQUENCE:
      (A) LONGUEUR: 2559 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: CDS
      (B) EMPLACEMENT:1..2556
   (xi) DESCRIPTION DE LA SEQUENCE: SEQ ID NO: 17:
   ATG AAA GTG ATG GGG ATG CAG AGT GGT TGG ATG GGG ATG AAG AGT GGT      48
   Met Lys Val Met Gly Met Gln Ser Gly Trp Met Gly Met Lys Ser Gly
           125                 130                 135
   TGG TTA CTC TTC TAT CTT CTA GTA AGC TTG ATC AAG GTA ATT GGG TCT      96
   Trp Leu Leu Phe Tyr Leu Leu Val Ser Leu Ile Lys Val Ile Gly Ser
       140                 145                 150
   GAA CAA CAT TGG GTA ACA GTG TAC TAT GGG GTA CCA GTA TGG AGA GAA     144
   Glu Gln His Trp Val Thr Val Tyr Tyr Gly Val Pro Val Trp Arg Glu
   155                 160                 165                 170
   GCA GAG ACA ACT CTT TTC TGT GCT TCA GAT GCT AAA GCC CAT AGT ACA     192
   Ala Glu Thr Thr Leu Phe Cys Ala Ser Asp Ala Lys Ala His Ser Thr
                   175                 180                 185
   GAG GCT CAC AAC ATC TGG GCC ACA CAA GCA TGT GTT CCT ACT GAT CCC     240
   Glu Ala His Asn Ile Trp Ala Thr Gln Ala Cys Val Pro Thr Asp Pro
               190                 195                 200
   AAT CCA CAA GAA GTG CTA TTA CCC AAT GTA ACT GAA AAA TTT AAT ATG     288
   Asn Pro Gln Glu Val Leu Leu Pro Asn Val Thr Glu Lys Phe Asn Met
           205                 210                 215
   TGG GAA AAT AAA ATG GCA GAC CAA ATG CAA GAG GAT ATT ATC AGT CTG     336
   Trp Glu Asn Lys Met Ala Asp Gln Met Gln Glu Asp Ile Ile Ser Leu
       220                 225                 230
   TGG GAA CAG AGC TTA AAG CCC TGT GTT AAA TTA ACC CCA TTA TGT GTA    384
   Trp Glu Gln Ser Leu Lys Pro Cys Val Lys Leu Thr Pro Leu Cys Val
   235                 240                 245                 250
   ACT ATG CTT TGT AAC GAT AGC TAT GGG GAG GAA AGG AAC AAT ACA AAT    432
   Thr Met Leu Cys Asn Asp Ser Tyr Gly Glu Glu Arg Asn Asn Thr Asn
                   255                 260                 265
   ATG ACA ACA AGA GAA CCA GAC ATA GGA TAC AAA CAA ATG AAA AAT TGC    480
   Met Thr Thr Arg Glu Pro Asp Ile Gly Tyr Lys Gln Met Lys Asn Cys
               270                 275                 280
   TCA TTC AAT GCA ACC ACT GAG CTA ACA GAT AAA AAG AAG CAA GTT TAC    528
   Ser Phe Asn Ala Thr Thr Glu Leu Thr Asp Lys Lys Lys Gln Val Tyr
           285                 290                 295
   TCT CTG TTT TAT GTA GAA GAT GTA GTA CCA ATC AAT GCC TAT AAT AAA    576
   Ser Leu Phe Tyr Val Glu Asp Val Val Pro Ile Asn Ala Tyr Asn Lys
       300                 305                 310
   ACA TAT AGG CTA ATA AAT TGT AAT ACC ACA GCT GTG ACA CAA GCT TGT    624
   Thr Tyr Arg Leu Ile Asn Cys Asn Thr Thr Ala Val Thr Gln Ala Cys
   315                 320                 325                 330
   CCT AAG ACT TCC TTT GAG CCA ATT CCA ATA CAT TAC TGT GCA CCA CCA    672
   Pro Lys Thr Ser Phe Glu Pro Ile Pro Ile His Tyr Cys Ala Pro Pro
                   335                 340                 345
   GGC TTT GCC ATT ATG AAA TGT AAT GAA GGA AAC TTT AGT GGA AAT GGA    720
   Gly Phe Ala Ile Met Lys Cys Asn Glu Gly Asn Phe Ser Gly Asn Gly
               350                 355                 360
   AGC TGT ACA AAT GTG AGT ACT GTA CAA TGC ACA CAT GGA ATA AAG CCA    768
   Ser Cys Thr Asn Val Ser Thr Val Gln Cys Thr His Gly Ile Lys Pro
           365                 370                 375
   GTG ATA TCC ACT CAG TTA ATC CTA AAT GGA AGC TTA AAT ACA GAT GGA    816
   Val Ile Ser Thr Gln Leu Ile Leu Asn Gly Ser Leu Asn Thr Asp Gly
       380                 385                 390
   ATT GTT ATT AGA AAT GAT AGT CAC AGT AAT CTG TTG GTG CAA TGG AAT    864
   Ile Val Ile Arg Asn Asp Ser His Ser Asn Leu Leu Val Gln Trp Asn
   395                 400                 405                 410
   GAG ACA GTG CCA ATA AAT TGT ACA AGG CCA GGA AAT AAT ACA GGA GGA    912
   Glu Thr Val Pro Ile Asn Cys Thr Arg Pro Gly Asn Asn Thr Gly Gly
                   415                 420                 425
   CAG GTG CAG ATA GGA CCT GCT ATG ACA TTT TAT AAC ATA GAA AAA ATA    960
   Gln Val Gln Ile Gly Pro Ala Met Thr Phe Tyr Asn Ile Glu Lys Ile
               430                 435                 440
   GTA GGA GAC ATT AGA CAA GCA TAC TGT AAT GTC TCT AAA GAA CTA TGG   1008
   Val Gly Asp Ile Arg Gln Ala Tyr Cys Asn Val Ser Lys Glu Leu Trp
           445                 450                 455
   GAA CCA ATG TGG AAT AGA ACA AGA GAG GAA ATA AAG AAA ATC CTG GGG   1056
   Glu Pro Met Trp Asn Arg Thr Arg Glu Glu Ile Lys Lys Ile Leu Gly
       460                 465                 470
   AAA AAC AAC ATA ACC TTC AGG GCT CGA GAG AGG AAT GAA GGA GAC CTA   1104
   Lys Asn Asn Ile Thr Phe Arg Ala Arg Glu Arg Asn Glu Gly Asp Leu
   475                 480                 485                 490
   GAA GTG ACA CAC TTA ATG TTC AAT TGT AGA GGA GAG TTT TTC TAT TGT   1152
   Glu Val Thr His Leu Met Phe Asn Cys Arg Gly Glu Phe Phe Tyr Cys
                   495                 500                 505
   AAC ACT TCC AAA TTA TTT AAT GAG GAA TTA CTT AAC GAG ACA GGT GAG   1200
   Asn Thr Ser Lys Leu Phe Asn Glu Glu Leu Leu Asn Glu Thr Gly Glu
               510                 515                 520
   CCT ATT ACT CTG CCT TGT AGA ATA AGA CAG ATT GTA AAT TTG TGG ACA   1248
   Pro Ile Thr Leu Pro Cys Arg Ile Arg Gln Ile Val Asn Leu Trp Thr
           525                 530                 535
   AGG GTA GGA AAA GGA ATT TAT GCA CCA CCA ATT CGG GGA GTT CTT AAC   1296
   Arg Val Gly Lys Gly Ile Tyr Ala Pro Pro Ile Arg Gly Val Leu Asn
       540                 545                 550
   TGT ACC TCC AAT ATT ACT GGA CTG GTT CTA GAA TAT AGT GGT GGG CCT   1344
   Cys Thr Ser Asn Ile Thr Gly Leu Val Leu Glu Tyr Ser Gly Gly Pro
   555                 560                 565                 570
   GAC ACC AAG GAA ACA ATA GTA TAT CCC TCA GGA GGA AAC ATG GTT AAT   1392
   Asp Thr Lys Glu Thr Ile Val Tyr Pro Ser Gly Gly Asn Met Val Asn
                   575                 580                 585
   CTC TGG AGA CAA GAG TTG TAT AAG TAC AAA GTA GTT AGC ATA GAA CCC   1440
   Leu Trp Arg Gln Glu Leu Tyr Lys Tyr Lys Val Val Ser Ile Glu Pro
               590                 595                 600
   ATA GGA GTA GCA CCA GGT AAA GCT AAA AGA CGC ACA GTG AGT AGA GAA   1488
   Ile Gly Val Ala Pro Gly Lys Ala Lys Arg Arg Thr Val Ser Arg Glu
           605                 610                 615
   AAA AGA GCA GCC TTT GGA CTA GGT GCG CTG TTT CTT GGG TTT CTT GGA   1536
   Lys Arg Ala Ala Phe Gly Leu Gly Ala Leu Phe Leu Gly Phe Leu Gly
       620                 625                 630
   GCA GCA GGG AGC ACT ATG GGC GCA GCG TCA ATA ACG CTG ACG GTA CAG   1584
   Ala Ala Gly Ser Thr Met Gly Ala Ala Ser Ile Thr Leu Thr Val Gln
   635                 640                 645                 650
   GCC CGG ACA TTA TTA TCT GGG ATA GTG CAA CAG CAG AAT ATT CTG TTG   1632
   Ala Arg Thr Leu Leu Ser Gly Ile Val Gln Gln Gln Asn Ile Leu Leu
                   655                 660                 665
   AGA GCA ATA GAG GCG CAA CAA CAT TTG TTG CAA CTC TCA ATC TGG GGC   1680
   Arg Ala Ile Glu Ala Gln Gln His Leu Leu Gln Leu Ser Ile Trp Gly
               670                 675                 680
   ATT AAA CAG CTC CAG GCA AAA GTC CTT GCT ATA GAA AGA TAC CTT AGG   1728
   Ile Lys Gln Leu Gln Ala Lys Val Leu Ala Ile Glu Arg Tyr Leu Arg
           685                 690                 695
   GAT CAG CAA ATC CTA AGT CTA TGG GGC TGC TCA GGA AAA ACA ATA TGC   1776
   Asp Gln Gln Ile Leu Ser Leu Trp Gly Cys Ser Gly Lys Thr Ile Cys
       700                 705                 710
   TAT ACC ACT GTG CCT TGG AAT GAG ACT TGG AGC AAC AAT ACC TCT TAT   1824
   Tyr Thr Thr Val Pro Trp Asn Glu Thr Trp Ser Asn Asn Thr Ser Tyr
   715                 720                 725                 730
   GAT ACA ATC TGG AAT AAT TTA ACC TGG CAA CAA TGG GAT GAG AAA GTA   1872
   Asp Thr Ile Trp Asn Asn Leu Thr Trp Gln Gln Trp Asp Glu Lys Val
                   735                 740                 745
   AGA AAC TAT TCA GGT GTC ATT TTT GGA CTT ATA GAA CAG GCA CAA GAA    1920
   Arg Asn Tyr Ser Gly Val Ile Phe Gly Leu Ile Glu Gln Ala Gln Glu
               750                 755                 760
   CAA CAG AAC ACA AAT GAG AAA TCA CTC TTG GAA TTG GAT CAA TGG GAC    1968
   Gln Gln Asn Thr Asn Glu Lys Ser Leu Leu Glu Leu Asp Gln Trp Asp
           765                 770                 775
   AGT CTG TGG AGC TGG TTT GGT ATT ACA AAA TGG CTG TGG TAT ATA AAA    2016
   Ser Leu Trp Ser Trp Phe Gly Ile Thr Lys Trp Leu Trp Tyr Ile Lys
       780                 785                 790
   ATA GCT ATA ATG ATA GTA GCA GGC ATT GTA GGC ATA AGA ATC ATA AGT    2064
   Ile Ala Ile Met Ile Val Ala Gly Ile Val Gly Ile Arg Ile Ile Ser
   795                 800                 805                 810
   ATA GTA ATA ACT ATA ATA GCA AGA GTT AGG CAG GGA TAT TCT CCC CTT    2112
   Ile Val Ile Thr Ile Ile Ala Arg Val Arg Gln Gly Tyr Ser Pro Leu
                   815                 820                 825
   TCG TTG CAG ACC CTT ATC CCA ACA GCA AGG GGA CCA GAC AGG CCA GAA    2160
   Ser Leu Gln Thr Leu Ile Pro Thr Ala Arg Gly Pro Asp Arg Pro Glu
               830                 835                 840
   GAA ACA GAA GGA GGC GTT GGA GAG CAA GAC AGA GGC AGA TCC GTG CGA    2208
   Glu Thr Glu Gly Gly Val Gly Glu Gln Asp Arg Gly Arg Ser Val Arg
           845                 850                 855
   TTA GTG AGC GGA TTC TCA GCT CTT GTC TGG GAG GAC CTC CGG AAC CTG    2256
   Leu Val Ser Gly Phe Ser Ala Leu Val Trp Glu Asp Leu Arg Asn Leu
       860                 865                 870
   TTG ATC TTC CTC TAC CAC CGC TTG ACA GAC TCA CTC TTG ATA CTG AGG    2304
   Leu Ile Phe Leu Tyr His Arg Leu Thr Asp Ser Leu Leu Ile Leu Arg
   875                 880                 885                 890
   AGG ACT CTG GAA CTC CTG GGA CAG AGT CTC AGC AGG GGA CTG CAA CTA    2352
   Arg Thr Leu Glu Leu Leu Gly Gln Ser Leu Ser Arg Gly Leu Gln Leu
                   895                 900                 905
   CTG AAT GAA CTC AGA ACA CAC TTG TGG GGA ATA CTT GCA TAT TGG GGA    2400
   Leu Asn Glu Leu Arg Thr His Leu Trp Gly Ile Leu Ala Tyr Trp Gly
               910                 915                 920
   AAA GAG TTA AGG GAT AGT GCT ATC AGC TTG CTT AAT ACA ACA GCT ATT    2448
   Lys Glu Leu Arg Asp Ser Ala Ile Ser Leu Leu Asn Thr Thr Ala Ile
           925                 930                 935
   GTA GTA GCA GAA GGA ACA GAT AGG ATT ATA GAA TTA GCA CAA AGA ATA    2496
   Val Val Ala Glu Gly Thr Asp Arg Ile Ile Glu Leu Ala Gln Arg Ile
       940                 945                 950
   GGA AGG GGA ATA TTA CAC ATA CCT AGA AGA ATC AGA CAA GGC CTA GAA    2544
   Gly Arg Gly Ile Leu His Ile Pro Arg Arg Ile Arg Gln Gly Leu Glu
   955                 960                 965                 970
   AGA GCA CTG ATA TAA                                                2559
   Arg Ala Leu Ile
(2) INFORMATIONS POUR LA SEQ ID NO: 18:
   (i) CARACTERISTIQUES DE LA SEQUENCE:
      (A) LONGUEUR: 852 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 18:
   Met Lys Val Met Gly Met Gln Ser Gly Trp Met Gly Met Lys Ser Gly
    1               5                  10                  15
   Trp Leu Leu Phe Tyr Leu Leu Val Ser Leu Ile Lys Val Ile Gly Ser
               20                  25                  30
   Glu Gln His Trp Val Thr Val Tyr Tyr Gly Val Pro Val Trp Arg Glu
           35                  40                  45
   Ala Glu Thr Thr Leu Phe Cys Ala Ser Asp Ala Lys Ala His Ser Thr
       50                  55                  60
   Glu Ala His Asn Ile Trp Ala Thr Gln Ala Cys Val Pro Thr Asp Pro
   65                  70                  75                  80
   Asn Pro Gln Glu Val Leu Leu Pro Asn Val Thr Glu Lys Phe Asn Met
                   85                  90                  95
   Trp Glu Asn Lys Met Ala Asp Gln Met Gln Glu Asp Ile Ile Ser Leu
               100                 105                 110
   Trp Glu Gln Ser Leu Lys Pro Cys Val Lys Leu Thr Pro Leu Cys Val
           115                 120                 125
   Thr Met Leu Cys Asn Asp Ser Tyr Gly Glu Glu Arg Asn Asn Thr Asn
       130                 135                 140
   Met Thr Thr Arg Glu Pro Asp Ile Gly Tyr Lys Gln Met Lys Asn Cys
   145                 150                 155                 160
   Ser Phe Asn Ala Thr Thr Glu Leu Thr Asp Lys Lys Lys Gln Val Tyr
                   165                 170                 175
   Ser Leu Phe Tyr Val Glu Asp Val Val Pro Ile Asn Ala Tyr Asn Lys
               180                 185                 190
   Thr Tyr Arg Leu Ile Asn Cys Asn Thr Thr Ala Val Thr Gln Ala Cys
           195                 200                 205
   Pro Lys Thr Ser Phe Glu Pro Ile Pro Ile His Tyr Cys Ala Pro Pro
       210                 215                 220
   Gly Phe Ala Ile Met Lys Cys Asn Glu Gly Asn Phe Ser Gly Asn Gly
   225                 230                 235                 240
   Ser Cys Thr Asn Val Ser Thr Val Gln Cys Thr His Gly Ile Lys Pro
                   245                 250                 255
   Val Ile Ser Thr Gln Leu Ile Leu Asn Gly Ser Leu Asn Thr Asp Gly
               260                 265                 270
   Ile Val Ile Arg Asn Asp Ser His Ser Asn Leu Leu Val Gln Trp Asn
           275                 280                 285
   Glu Thr Val Pro Ile Asn Cys Thr Arg Pro Gly Asn Asn Thr Gly Gly
       290                 295                 300
   Gln Val Gln Ile Gly Pro Ala Met Thr Phe Tyr Asn Ile Glu Lys Ile
   305                 310                 315                 320
   Val Gly Asp Ile Arg Gln Ala Tyr Cys Asn Val Ser Lys Glu Leu Trp
                   325                 330                 335
   Glu Pro Met Trp Asn Arg Thr Arg Glu Glu Ile Lys Lys Ile Leu Gly
               340                 345                 350
   Lys Asn Asn Ile Thr Phe Arg Ala Arg Glu Arg Asn Glu Gly Asp Leu
           355                 360                 365
   Glu Val Thr His Leu Met Phe Asn Cys Arg Gly Glu Phe Phe Tyr Cys
       370                 375                 380
   Asn Thr Ser Lys Leu Phe Asn Glu Glu Leu Leu Asn Glu Thr Gly Glu
   385                 390                 395                 400
   Pro Ile Thr Leu Pro Cys Arg Ile Arg Gln Ile Val Asn Leu Trp Thr
                   405                 410                 415
   Arg Val Gly Lys Gly Ile Tyr Ala Pro Pro Ile Arg Gly Val Leu Asn
               420                 425                 430
   Cys Thr Ser Asn Ile Thr Gly Leu Val Leu Glu Tyr Ser Gly Gly Pro
           435                 440                 445
   Asp Thr Lys Glu Thr Ile Val Tyr Pro Ser Gly Gly Asn Met Val Asn
       450                 455                 460
   Leu Trp Arg Gln Glu Leu Tyr Lys Tyr Lys Val Val Ser Ile Glu Pro
   465                 470                 475                 480
   Ile Gly Val Ala Pro Gly Lys Ala Lys Arg Arg Thr Val Ser Arg Glu
                   485                 490                 495
   Lys Arg Ala Ala Phe Gly Leu Gly Ala Leu Phe Leu Gly Phe Leu Gly
               500                 505                 510
   Ala Ala Gly Ser Thr Met Gly Ala Ala Ser Ile Thr Leu Thr Val Gln
           515                 520                 525
   Ala Arg Thr Leu Leu Ser Gly Ile Val Gln Gln Gln Asn Ile Leu Leu
       530                 535                 540
   Arg Ala Ile Glu Ala Gln Gln His Leu Leu Gln Leu Ser Ile Trp Gly
   545                 550                 555                 560
   Ile Lys Gln Leu Gln Ala Lys Val Leu Ala Ile Glu Arg Tyr Leu Arg
                   565                 570                 575
   Asp Gln Gln Ile Leu Ser Leu Trp Gly Cys Ser Gly Lys Thr Ile Cys
               580                 585                 590
   Tyr Thr Thr Val Pro Trp Asn Glu Thr Trp Ser Asn Asn Thr Ser Tyr
           595                 600                 605
   Asp Thr Ile Trp Asn Asn Leu Thr Trp Gln Gln Trp Asp Glu Lys Val
       610                 615                 620
   Arg Asn Tyr Ser Gly Val Ile Phe Gly Leu Ile Glu Gln Ala Gln Glu
   625                 630                 635                 640
   Gln Gln Asn Thr Asn Glu Lys Ser Leu Leu Glu Leu Asp Gln Trp Asp
                   645                 650                 655
   Ser Leu Trp Ser Trp Phe Gly Ile Thr Lys Trp Leu Trp Tyr Ile Lys
   660 665 670
   Ile Ala Ile Met Ile Val Ala Gly Ile Val Gly Ile Arg Ile Ile Ser
   675 680 685
   Ile Val Ile Thr Ile Ile Ala Arg Val Arg Gln Gly Tyr Ser Pro Leu
   690 695 700
   Ser Leu Gln Thr Leu Ile Pro Thr Ala Arg Gly Pro Asp Arg Pro Glu
   705 710 715 720
   Glu Thr Glu Gly Gly Val Gly Glu Gln Asp Arg Gly Arg Ser Val Arg
   725 730 735
   Leu Val Ser Gly Phe Ser Ala Leu Val Trp Glu Asp Leu Arg Asn Leu
   740 745 750
   Leu Ile Phe Leu Tyr His Arg Leu Thr Asp Ser Leu Leu Ile Leu Arg
   755 760 765
   Arg Thr Leu Glu Leu Leu Gly Gln Ser Leu Ser Arg Gly Leu Gln Leu
   770 775 780
   Leu Asn Glu Leu Arg Thr His Leu Trp Gly Ile Leu Ala Tyr Trp Gly
   785 790 795 800
   Lys Glu Leu Arg Asp Ser Ala Ile Ser Leu Leu Asn Thr Thr Ala Ile
   805 810 815
   Val Val Ala Glu Gly Thr Asp Arg Ile Ile Glu Leu Ala Gln Arg Ile
   820 825 830
   Gly Arg Gly Ile Leu His Ile Pro Arg Arg Ile Arg Gln Gly Leu Glu
   835 840 845
   Arg Ala Leu Ile
   850
(2) INFORMATIONS POUR LA SEQ ID NO: 19:
   (i) CARACTERISTIQUES DE LA SEQUENCE:
      (A) LONGUEUR: 639 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: CDS
      (B) EMPLACEMENT:1..636
   (xi) DESCRIPTION DE LA SEQUENCE: SEQ ID NO: 19:
   ATG GGA AAG ATT TGG TCA AAG AGC AGC CTA GTA GGA TGG CCA GAA ATC      48
   Met Gly Lys Ile Trp Ser Lys Ser Ser Leu Val Gly Trp Pro Glu Ile
           855                 860                 865
   AGA GAA AGA ATG AGA AGA CAA ACG CAA GAA CCA GCA GTA GAG CCA GCA      96
   Arg Glu Arg Met Arg Arg Gln Thr Gln Glu Pro Ala Val Glu Pro Ala
       870                 875                 880
   GTA GGA GCA GGA GCA GCT TCT CAA GAT CTA GCT AAT CGA GGG GCC ATC     144
   Val Gly Ala Gly Ala Ala Ser Gln Asp Leu Ala Asn Arg Gly Ala Ile
   885                 890                 895                 900
   ACC ATA AGA AAT ACT AGA GAC AAT AAT GAA AGT ATA GCT TGG CTA GAA     192
   Thr Ile Arg Asn Thr Arg Asp Asn Asn Glu Ser Ile Ala Trp Leu Glu
                   905                 910                 915
   GCA CAA GAA GAA GAA GAG GAA GTA GGC TTT CCA GTA CGC CCT CAG GTA     240
   Ala Gln Glu Glu Glu Glu Glu Val Gly Phe Pro Val Arg Pro Gln Val
               920                 925                 930
   CCA TTA AGG CCA ATA ACC TAT AAA CAG GCT TTT GAT CTT TCC TTC TTT     288
   Pro Leu Arg Pro Ile Thr Tyr Lys Gln Ala Phe Asp Leu Ser Phe Phe
           935                 940                 945
   TTA AAA GAT AAG GGG GGA CTG GAA GGG CTA GTT TGG TCC AGA AAA AGG     336
   Leu Lys Asp Lys Gly Gly Leu Glu Gly Leu Val Trp Ser Arg Lys Arg
       950                 955                 960
   CAA GAT ATT CTA GAC CTC TGG ATG TAT CAC ACA CAA GGC ATC CTC CCT     384
   Gln Asp Ile Leu Asp Leu Trp Met Tyr His Thr Gln Gly Ile Leu Pro
   965                 970                 975                 980
   GAC TGG CAT AAC TAC ACA CCA GGG CCA GGA ATT AGA TAC CCC GTA ACC     432
   Asp Trp His Asn Tyr Thr Pro Gly Pro Gly Ile Arg Tyr Pro Val Thr
                   985                 990                 995
   TTT GGA TGG TGC TTC AAA CTA GTA CCA TTG TCA GCT GAA GAA GTA GAA     480
   Phe Gly Trp Cys Phe Lys Leu Val Pro Leu Ser Ala Glu Glu Val Glu
               1000                1005                1010
   GAG GCT AAT GAA GGA GAC AAC AAT GCC CTC TTA CAC CCC ATA TGT CAA     528
   Glu Ala Asn Glu Gly Asp Asn Asn Ala Leu Leu His Pro Ile Cys Gln
           1015                1020                1025
   CAT GGA GCA GAT GAT GAT CAT AAA GAA GTG TTG GTG TGG CGA TTT GAC     576
   His Gly Ala Asp Asp Asp His Lys Glu Val Leu Val Trp Arg Phe Asp
       1030                1035                1040
   AGC TCC CTA GCA AGA AGA CAT GTA GCA AGA GAG CTG CAT CCG GAG TTT     624
   Ser Ser Leu Ala Arg Arg His Val Ala Arg Glu Leu His Pro Glu Phe
   1045                1050                1055                1060
   TAC AAG AAC TGC TGA                                                 639
   Tyr Lys Asn Cys
(2) INFORMATIONS POUR LA SEQ ID NO: 20:
   (i) CARACTERISTIQUES DE LA SEQUENCE:
      (A) LONGUEUR: 212 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 20:
   Met Gly Lys Ile Trp Ser Lys Ser Ser Leu Val Gly Trp Pro Glu Ile
    1               5                  10                  15
   Arg Glu Arg Met Arg Arg Gln Thr Gln Glu Pro Ala Val Glu Pro Ala
               20                  25                  30
   Val Gly Ala Gly Ala Ala Ser Gln Asp Leu Ala Asn Arg Gly Ala Ile
           35                  40                  45
   Thr Ile Arg Asn Thr Arg Asp Asn Asn Glu Ser Ile Ala Trp Leu Glu
       50                  55                  60
   Ala Gln Glu Glu Glu Glu Glu Val Gly Phe Pro Val Arg Pro Gln Val
   65                  70                  75                  80
   Pro Leu Arg Pro Ile Thr Tyr Lys Gln Ala Phe Asp Leu Ser Phe Phe
                   85                  90                  95
   Leu Lys Asp Lys Gly Gly Leu Glu Gly Leu Val Trp Ser Arg Lys Arg
               100                 105                 110
   Gln Asp Ile Leu Asp Leu Trp Met Tyr His Thr Gln Gly Ile Leu Pro
           115                 120                 125
   Asp Trp His Asn Tyr Thr Pro Gly Pro Gly Ile Arg Tyr Pro Val Thr
       130                 135                 140
   Phe Gly Trp Cys Phe Lys Leu Val Pro Leu Ser Ala Glu Glu Val Glu
   145                 150                 155                 160
   Glu Ala Asn Glu Gly Asp Asn Asn Ala Leu Leu His Pro Ile Cys Gln
                   165                 170                 175
   His Gly Ala Asp Asp Asp His Lys Glu Val Leu Val Trp Arg Phe Asp
               180                 185                 190
   Ser Ser Leu Ala Arg Arg His Val Ala Arg Glu Leu His Pro Glu Phe
           195                 200                 205
   Tyr Lys Asn Cys
       210
(2) INFORMATIONS POUR LA SEQ ID NO: 21:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 21:
      ATTGCGTACT CACACTTCCG 20
(2) INFORMATIONS POUR LA SEQ ID NO: 22:
   (i) CARACTERISTIQUES DE LA SEQUENCE:
      (A) LONGUEUR: 17 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 22:
      GGCAAGCAGG GAGCTGG 17
(2) INFORMATIONS POUR LA SEQ ID NO: 23:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 23:
      TCCTTGAGCA GTCTGGAC 18
(2) INFORMATIONS POUR LA SEQ ID NO: 24:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 24:
      GAACAGGAGG ATTAGCAG 18
(2) INFORMATIONS POUR LA SEQ ID NO: 25:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 25:
      AGCAGAGGCT ATGTCACA 18
(2) INFORMATIONS POUR LA SEQ ID NO: 26:
   (i) CARACTERISTIQUES DE LA SEQUENCE:
      (A) LONGUEUR: 19 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 26:
      TGTAAGGCCC CTAGAAGAG 19
(2) INFORMATIONS POUR LA SEQ ID NO: 27:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"

      (xi) DESCRIPTION DE LA SEQUENCE: SEQ ID NO: 27:
      ACAGAGAACT CTCTGTAC 18
(2) INFORMATIONS POUR LA SEQ ID NO: 28:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 28:
      AAGAAAAGCA GTTGGTAC 18
(2) INFORMATIONS POUR LA SEQ ID NO: 29:
   (i) CARACTERISTIQUES DE LA SEQUENCE:
      (A) LONGUEUR: 17 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 29:
      TTTCTTCCCT GTATGTC 17
(2) INFORMATIONS POUR LA SEQ ID NO: 30:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 30:
      GTTATATGGA T'"CTCAGG 18
(2) INFORMATIONS POUR LA SEQ ID NO: 31:
   (i) CARACTERISTIQUES DE LA SEQUENCE:
      (A) LONGUEUR: 19 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 31:
      TGGCAGCACA TTATACTGG 19
(2) INFORMATIONS POUR LA SEQ ID NO: 32:
   (i) CARACTERISTIQUES DE LA SEQUENCE:
      (A) LONGUEUR: 23 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 32:
      ATCATTTACC AGTACATGGA CGA 23
(2) INFORMATIONS POUR LA SEQ ID NO: 33:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 33:
      TGTCAGGGGT CGTAAAGC 18
(2) INFORMATIONS POUR LA SEQ ID NO: 34:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 34:
      TCCTCTGGAT GGGATATG 18
(2) INFORMATIONS POUR LA SEQ ID NO: 35:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 35:
      TCTATCCAGG AATCAGAG 18
(2) INFORMATIONS POUR LA SEQ ID NO: 36:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 36:
      AATGAGATCT GCCCATAC 18
(2) INFORMATIONS POUR LA SEQ ID NO: 37:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 37:
      TGACAGATAG GGGAAGAC 18
(2) INFORMATIONS POUR LA SEQ ID NO: 38:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = 'AMORCE'
   (xi) DESCRIPTION DE LA SEQUENCE: SEQ ID NO: 38:
      AACCGCCATT TGCACTGC 18
(2) INFORMATIONS POUR LA SEQ ID NO: 39:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 39:
      ACATGGACCG CCACAAGG 18
(2) INFORMATIONS POUR LA SEQ ID NO: 40:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 40:
      AGCAACAGAC ATACAGAC 18
(2) INFORMATIONS POUR LA SEQ ID NO: 41:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 41:
      AAAGTAGTCC CACGTAGG 18
(2) INFORMATIONS POUR LA SEQ ID NO: 42:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 42:
      ATATCCCAGT AGGTCAGG 18
(2) INFORMATIONS POUR LA SEQ ID NO: 43:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE:: Autre acide nucléique
      (A) DESCRIPTION: /desc = "AMORCE"
   (xi) DESCRIPTION DE LA SEQUENCE: SEQ ID NO: 43:
      TCTAGCACTA ACAGCCTG 18
(2) INFORMATIONS POUR LA SEQ ID NO: 44:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 44:
      ACTCTTACTG CTCTGAGG 18
(2) INFORMATIONS POUR LA SEQ ID NO: 45:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 45:
      CCATAGTACA CTGTTACC 18
(2) INFORMATIONS POUR LA SEQ ID NO: 46:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 46:
      CATAGCTATC GTTACAAAGC 20
(2) INFORMATIONS POUR LA SEQ ID NO: 47:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 47:
      TCATAATGGC AAAGCCTG 18
(2) INFORMATIONS POUR LA SEQ ID NO: 48:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 48:
      CTATTCCACA TTGGTTCC 18
(2) INFORMATIONS POUR LA SEQ ID NO: 49:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 49:
      ATTCTAGAAC CAGTCCAG 18
(2) INFORMATIONS POUR LA SEQ ID NO: 50:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 50:
      CCTTAGGGAT CAGCAAATCC 20
(2) INFORMATIONS POUR LA SEQ ID NO: 51:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 51:
      TGGGACAGTC TGTGGAGC 18
(2) INFORMATIONS POUR LA SEQ ID NO: 52:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 52:
      TTCTCAGCTC TTGTCTGG 18
(2) INFORMATIONS POUR LA SEQ ID NO: 53:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 53:
      ATTAAGCAAG CTGATAGC 18
(2) INFORMATIONS POUR LA SEQ ID NO: 54:
   (i) CARACTERISTIQUES DE LA SEQUENCE:
      (A) LONGUEUR: 16 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 54:
      TGTGCTTCTA GCCAAG 16
(2) INFORMATIONS POUR LA SEQ ID NO: 55:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 55:
      GCTCCATGTT GACATATG 18
(2) INFORMATIONS POUR LA SEQ ID NO: 56:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 56:
      AGAGAGACCC AGTACAAG 18
(2) INFORMATIONS POUR LA SEQ ID NO: 57:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 57:
      ATAAAAGCAG CCGCTTCTCG 20
(2) INFORMATIONS POUR LA SEQ ID NO: 58:
   (i) CARACTERISTIQUES DE LA SEQUENCE:
      (A) LONGUEUR: 35 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 58:
   Cys Thr Arg Pro Gly Asn Asn Thr Gly Gly Gln Val Gln Ile Gly Pro
   1               5                   10                  15
   Ala Met Thr Phe Tyr Asn Ile Glu Lys Ile Val Gly Asp Ile Arg Gln
               20                  25                  30
   Ala Tyr Cys
           35
(2) INFORMATIONS POUR LA SEQ ID NO: 59:
   (i) CARACTERISTIQUES DE LA SEQUENCE:
      (A) LONGUEUR: 35 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 59:
   Cys His Arg Pro Gly Asn Asn Thr Arg Gly Glu Val Gln Ile Gly Pro
   1               5                   10                  15
   Gly Met Thr Phe Tyr Asn Ile Glu Asn Val Tyr Gly Asp Thr Arg Ser
               20                  25                  30
   Ala Tyr Cys
           35
(2) INFORMATIONS POUR LA SEQ ID NO: 60:
   (i) CARACTERISTIQUES DE LA SEQUENCE:
      (A) LONGUEUR: 35 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 60:
   Cys Ile Arg Pro Gly Asn Arg Thr Tyr Arg Asn Leu Gln Ile Gly Pro
   1               5                   10                  15
   Gly Met Thr Phe Tyr Asn Val Glu Ile Ala Thr Gly Asp Ile Arg Lys
               20                  25                  30
   Ala Phe Cys
           35
(2) INFORMATIONS POUR LA SEQ ID NO: 61:
   (i) CARACTERISTIQUES DE LA SEQUENCE:
      (A) LONGUEUR: 35 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 61:
   Cys Thr Arg Pro Asn Asn Asn Thr Arg Lys Ser Val Arg Ile Gly Pro
   1               5                   10                  15
   Gly Gln Ala Phe Tyr Ala Thr Gly Asp Ile Ile Gly Asp Ile Arg Gln
               20                  25                  30
   Ala His Cys
           35

## Description

La présente invention est relative à des souches de rétrovirus du groupe VIH-1, non-M non-O, notamment une souche dénommée YBF30, à ses fragments ainsi qu'à ses applications, en tant que réactif de diagnostic et en tant qu'agent immunogène.

Les virus humains de l'immunodéficience acquise, VIH-1 et VIH-2 sont des rétrolentivirus, virus retrouvés chez de nombreux primates africains. Tous ces virus semblent avoir un ancêtre commun ; il est toutefois très difficile de préjuger de la période à laquelle ces différents virus se sont séparés de ce précurseur. D'autres virus plus distants bien que faisant partie du même groupe sont retrouvés chez d'autres mammifères (ongulés et félins).

Tous ces virus sont associés à des infections longues ; l'absence de symptômes est la règle chez les singes infectés naturellement.

Du fait de sa forte homologie avec le virus du Sooty Mangabey (Afrique de l'Ouest), l'origine du VIH-2 semble claire, mais aucun virus proche du VIH-1 n'a été retrouvé chez les singes. Les virus les plus proches sont des virus retrouvés chez deux chimpanzés (SIV CPZGAB, SIV ANT).

Une importante variabilité génétique est retrouvée chez tous les lentivirus, et l'étude phylogénétique de ces variants obtenus à partir de nombreux points géographiques différents a permis de distinguer pour vIH-1, 8 sous-types (clades), tous également équidistants entre eux. Les clades ne sont qu'une représentation mathématique de l'expression de la variabilité l'analyse phénétique, basée non sur les acides nucléiques mais sur les acides aminés donne des résultats différents (Korber et al, 1994).

La mise en évidence de sous-types correspond à une analyse phylogénétique qui n'a pas, à ce jour de corrélation physiopathologique, mais une correspondance géographique. En effet, chaque sous-type est retrouvé principalement dans un certain espace géographique. En Europe et aux États-Unis, le sous-type B est majoritaire, alors qu'en Thailande, deux sous-types E et B sont retrouvés, et qu'il existe une corrélation forte entre le mode de transmission qui, en fait, correspond à une certaine population et le sous-type retrouvé. Tous les clades ont été retrouvés en Afrique et leurs distributions à travers le reste du monde reflète une probabilité de rencontre entre personnes à comportement à haut risque. Le clade majoritaire, car présent en proportion importante en Afrique est le clade A. Dans certains pays d'Afrique, une très grande variabilité a été retrouvée (G. Myers, 1994 ; P.M. Sharp et al., 1994). Plusieurs sous-types ont été caractérisés dans les pays d'Afrique centrale de l'ouest, comme la République Centre Africaine (Murphy et al, 1993) et le Cameroun (Nkengasong et al, 1994).

Dernièrement, des patients porteurs de virus variants du VIH-1, dont les sérums posaient des problèmes de détection pour certains kits commercialisés sur le marché français et dont les western blots de confirmation étaient atypiques, ont été caractérisés (Loussert-Ajaka et al ; 1994; Simon et al, 1994 , Demande Internationale PCT WO 96/27013)

L'analyse de ces variants a permis de confirmer que les virus VIF de type 1, devaient être sous-divisés en deux groupes, le groupe M (majeur) et un groupe O (Outlier) incluant ces isolats, comme l'avaient proposé Chameau et al, 1994. L'analyse du rapport des mutations synonymes/mutations non synonymes sur les séquences des virus du groupe O connus, indique que ce nouveau groupe est aussi ancien, si ce n'est plus, que le groupe M (Loussert-Ajaka et al, 1995). Sa faible prévalence à ce jour, 8% des patients infectés par VIH-1 au Cameroun (Zekeng et al, 1994), et 18 cas caractérisés en France, serait due à des facteurs purement épidémiologiques.

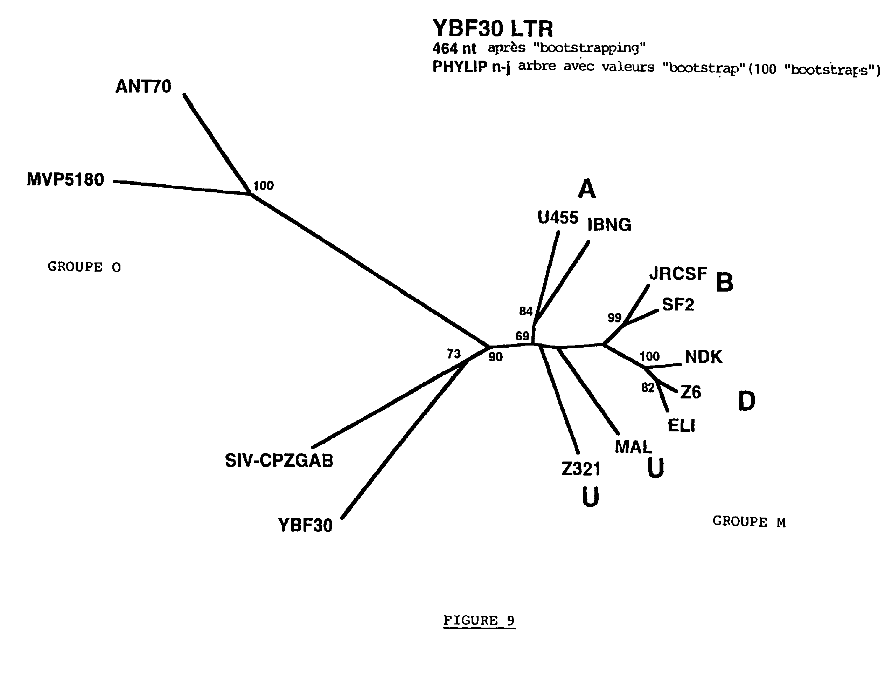

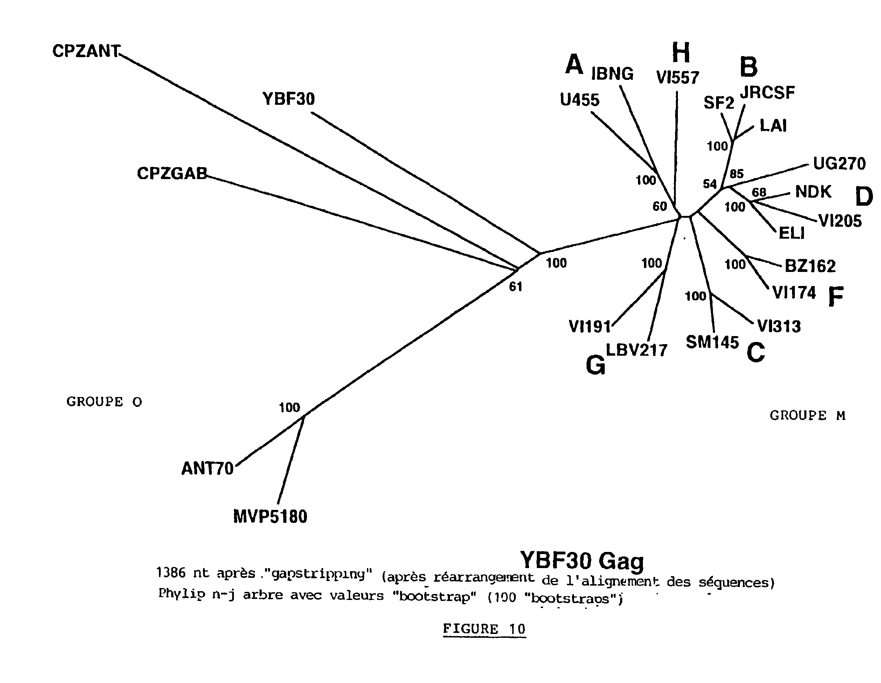

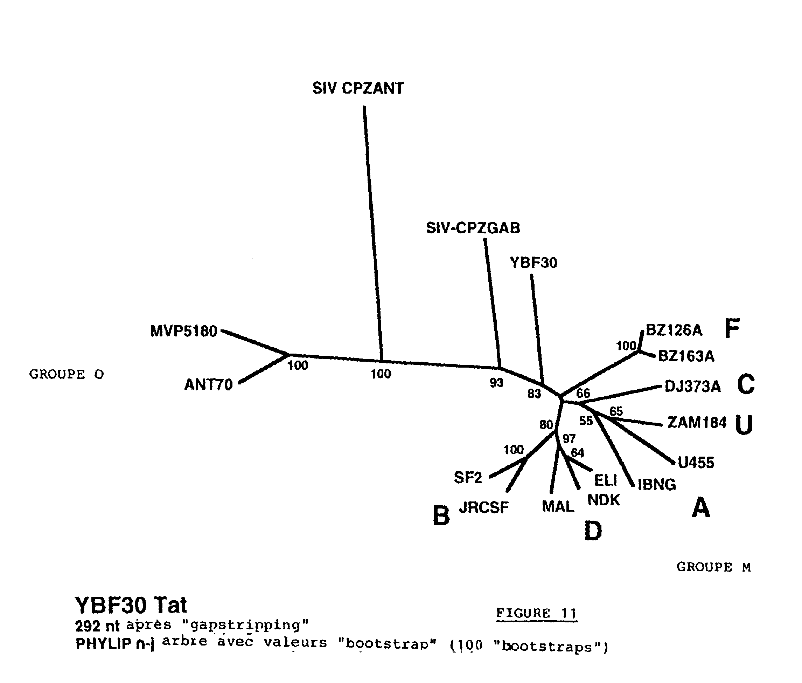

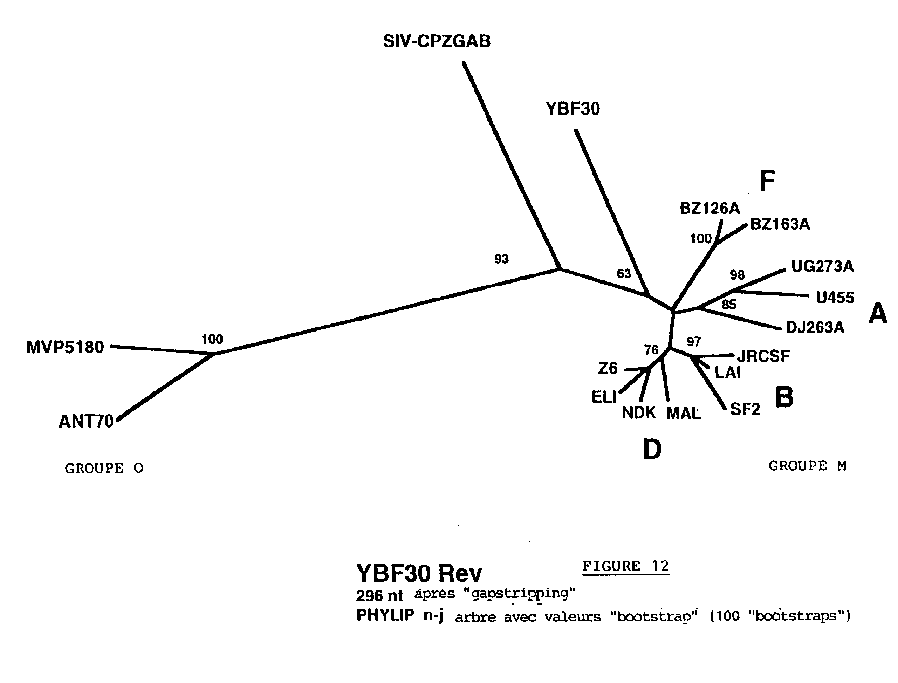

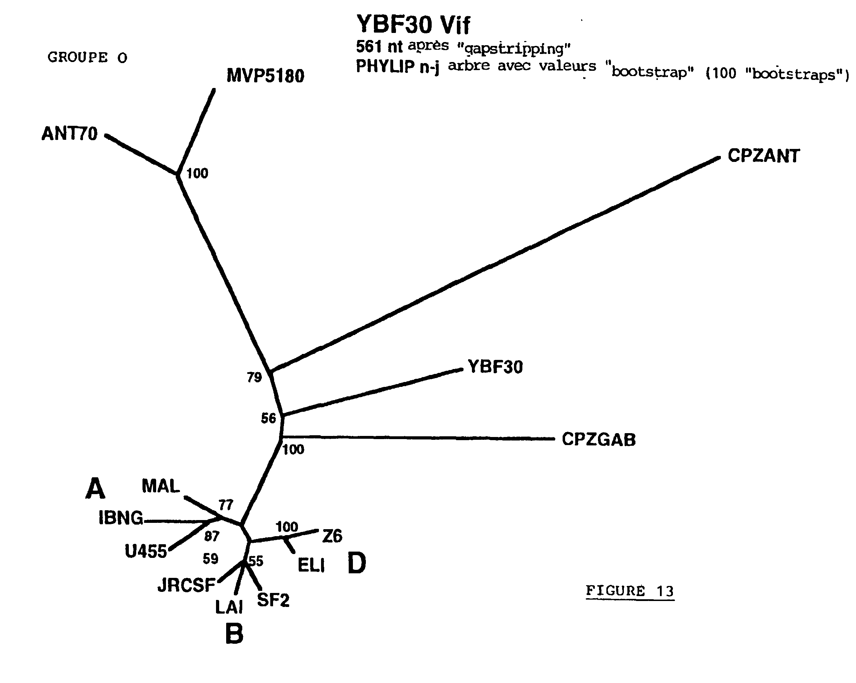

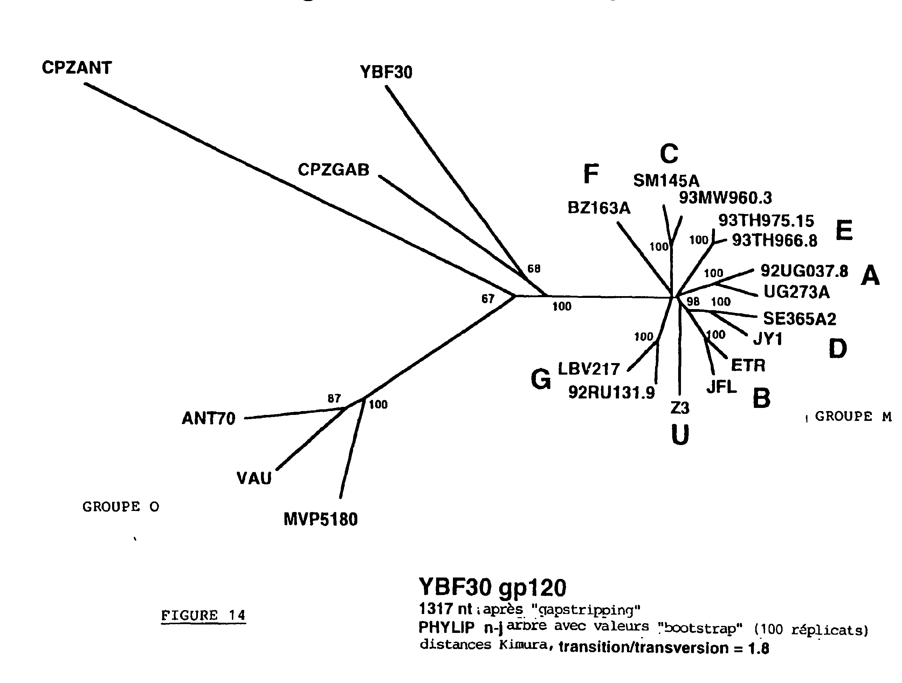

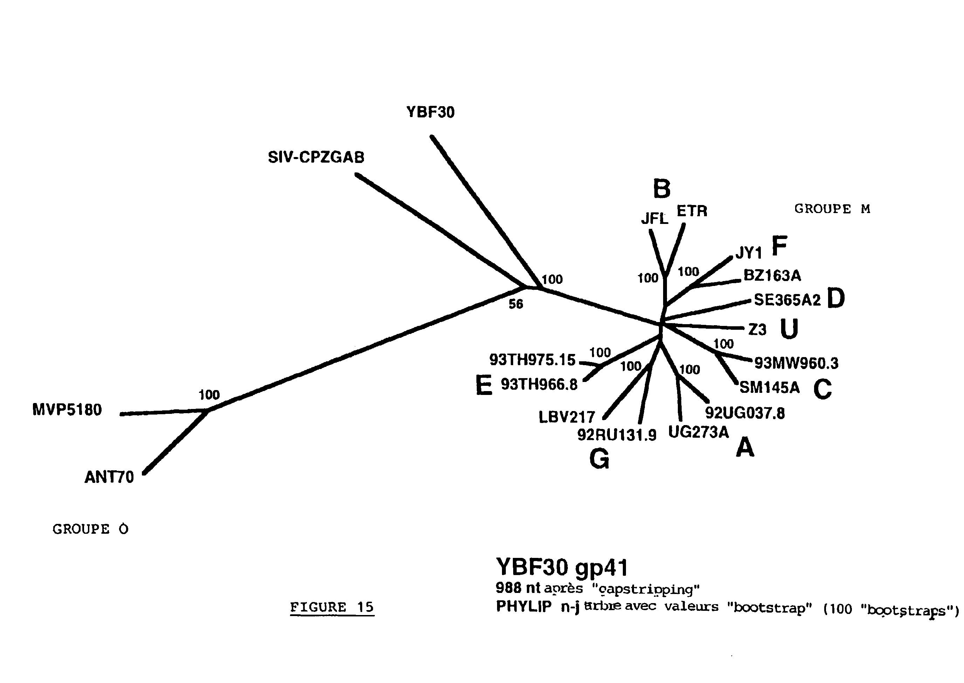

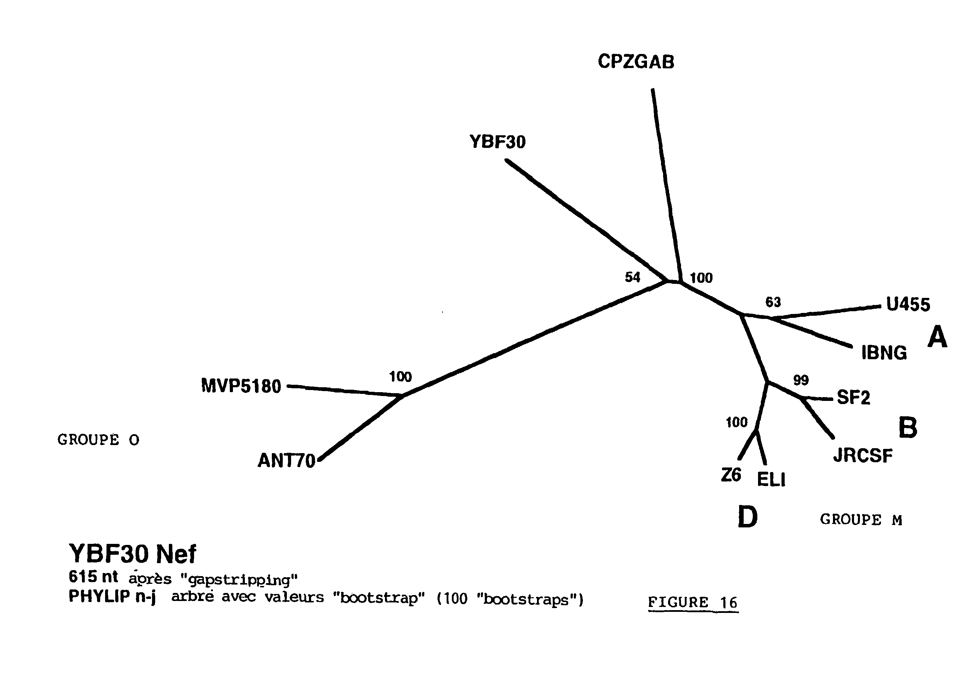

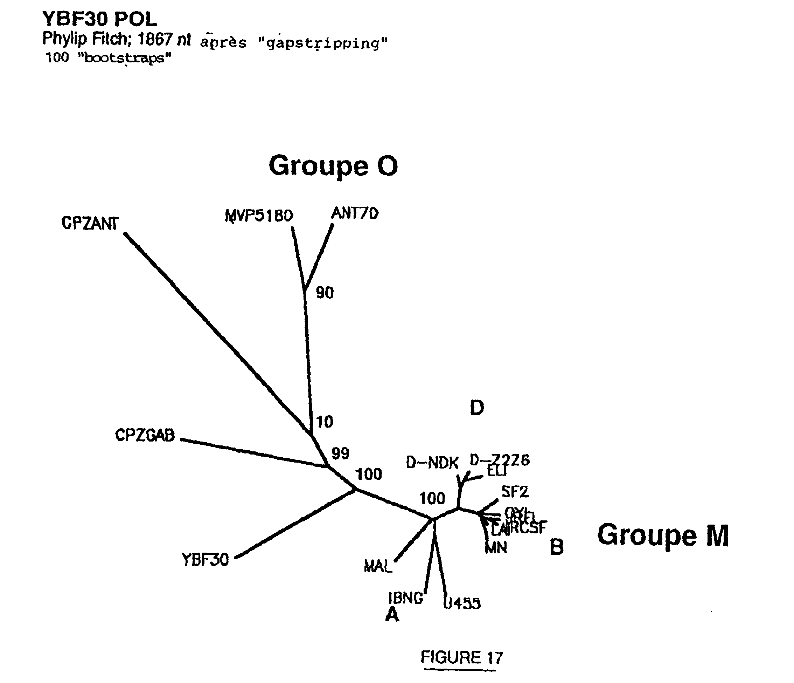

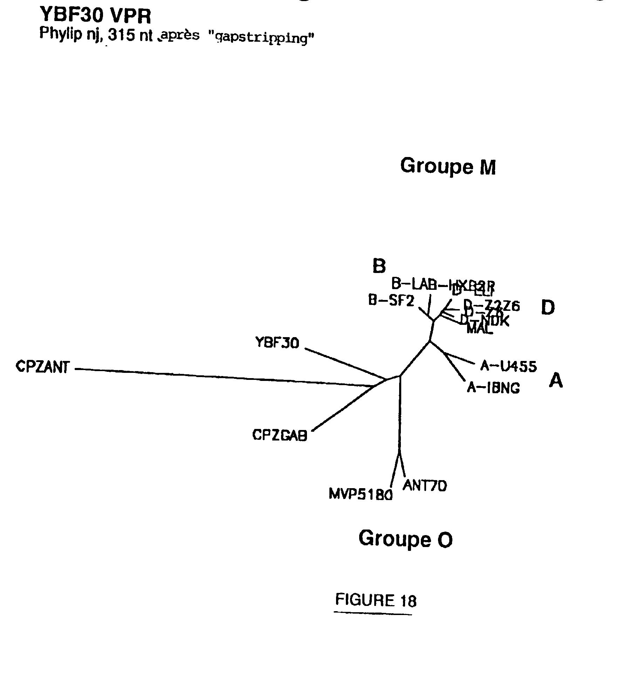

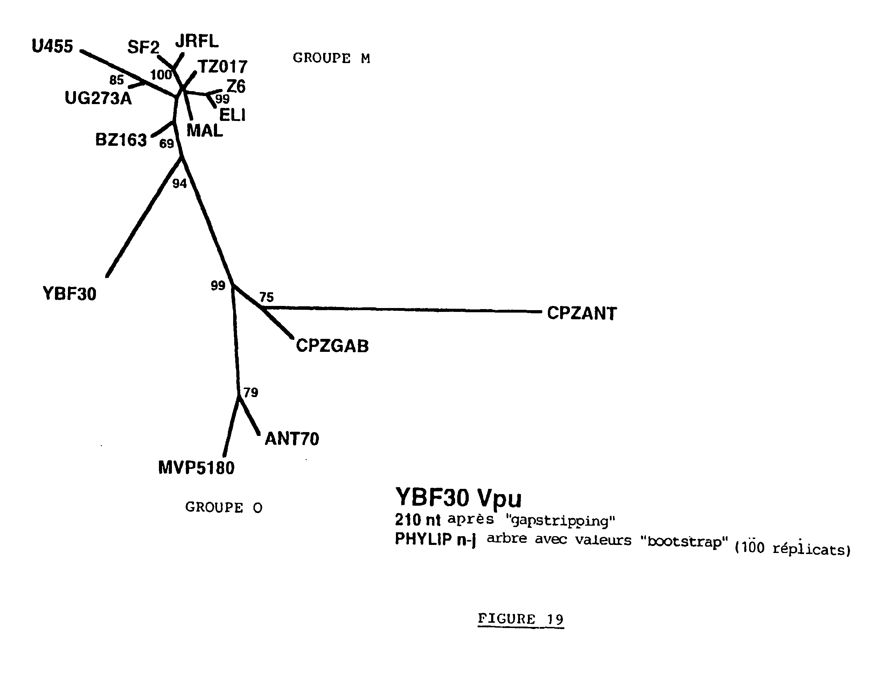

Ces deux groupes de VIH-1 forment un arbre en forme de double étoile (figures 9 à 19). Deux isolats, SIV CPZGAB, caractérisé à partir d'un chimpanzé du Gabon (Huet et al, 1990) et CPZANT, caractérisé à partir d'un chimpanzé du zoo d'Anvers ont des séquences et des organisations géniques très proche de VIH-1, mais ne s'inscrivent dans aucun de ces deux groupes et forment sur l'arbre phylogénétique deux nouvelles branches.

La mise en évidence de nouveaux variants est importante pour mettre au point des réactifs de dépistage des infections par VIH, suffisamment sensibles et spécifiques, c'est-à-dire ne conduisant pas à des résultats faussement négatifs ou faussement positifs et des compositions protectrices vis-à-vis de sous-types n'appartenant ni au groupe M, ni au groupe O.

En conséquence, la Demanderesse s'est donné pour but de pourvoir à une souche non-M, non-O, ainsi qu'à des séquences issues de cette souche, aptes à permettre la détection de variants du VIH-1 non M et non-O, qui permettent d'éviter l'obtention de résultats faussement négatifs ou faussement positifs. Pour ce faire, les Inventeurs ont notamment établi un algorithme de différenciation et de confirmation entre les infections HIV-1 des groupes M et O, ce qui leur a permis de sélectionner des variants non-M, non-O.

La présente invention a pour objet une souche de VIH-1 non-M non-O, dénommée YBF30, déposée à la Collection Nationale de Cultures de Microorganismes tenue par l'Institut Pasteur sous le numéro I-1753 le 2 juillet 1996.

On entend par variant non-M non-O, un VIH de type 1, qui sérologiquement et moléculairement ne peut être reconnu comme appartenant à l'un de ces groupes.

La présente invention a également pour objet la séquence nucléotidique complète de la souche telle que définie ci-dessus (SEQ ID N° 1) ainsi que les fragments d'acide nucléique suivants, issus de ladite souche :
- LTR YBF 30 (SEQ ID N°2),
- GAG YBF 30 (SEQ ID N°3) (gène *gag),*
- POL YBF 30 (SEQ ID N°5) (gène *pol*),
- VIF YBF 30 (SEQ ID N°7) (gène *vif*),
- VPR YBF 30 (SEQ ID N°9) (gène *vpr*),
- VPU YBF 30 (SEQ ID N°11) (gène *vpu*),
- TAT YBF 30 (SEQ ID N°13) (gène *tat*),
- REV YBF 30 (SEQ ID N°15) (gène *rev*),
- ENV gp160 YBF 30 (SEQ ID N°17) (gène *env*), et
- NEF YBF 30 (SEQ ID N° 19) (gène *nef*),

L'invention a également pour objet un acide nucléique isolé constitué d'un fragment d'au moins 10 nucléotides d'un acide nucléique ou d'un fragment d'acide nucléique tel que défini ci-dessus. De préférence, ledit acide nucléique selon l'invention est sélectionné parmi les acides nucléiques de séquences SEQ ID NO: 21 à SEQ ID NO: 57, également dénommées respectivement YLG, LPBS.1, GAG Y AS1.1, GAG Y AS1, GAG 6, GAG Y S1, GAG Y S1.1, GAG Y S 1.2, YRT AS 1.3, YRT AS 1.2, YRT AS 1.1, YRT 2, YRT AS 1, YRT 2.1, YRT 2.2, YRT 2.3, YRT 2.4, 4481-1, 4481-2, 4235.1, 4235.2, 4235.3, 4235.4, SK69.6, SK69.5, SK69.4, SK69.3, SK69.2, SK69.1, SK68.1, SK68.2, SK68.3, LSI AS1.3, LSI AS1.2, LSI AS 1.1, LSI A1, YLPA. Les séquences SEQ ID NO: 21 à 57 sont aptes à servir d'amorces et/ou de sondes pour la détection d'un VIH-1 présentant les caractéristiques morphologiques et immunologiques de la souche YBF30 selon l'invention.

De telles séquences trouvent application dans l'identification spécifique d'un VIH-1 non-M non-O, comme réactif de diagnostic, seules ou en *pool* avec d'autres réactifs, pour l'identification différentielle de n'importe quel VIH-1. Ces séquences peuvent notamment être mises en oeuvre dans des tests de diagnostic comprenant, soit une hybridation directe avec la séquence virale à détecter, soit une amplification de ladite séquence virale, en utilisant comme amorces ou comme sondes, un oligonucléotide comprenant au moins 10 nucléotides, inclus dans l'une quelconque des séquences ci-dessus et notamment l'une des séquences SEQ ID N°21-57 précitées.

Les caractéristiques de ladite souche VIH-1 non-M non-O sont les suivantes :
* peu ou pas de réactivité sérologique vis-à-vis des protéines des groupes M et O et forte réactivité sérologique vis-à-vis des protéines issues de la souche YBF30 ou de la souche SIV CPZGAB ;
* absence d'amplification génomique à l'aide des amorces des régions *env* et *gag* des VIH-1 des groupes M et O ;
* amplification génomique en présence des amorces issues de la souche YBF30, telles que définies ci-dessus ; et
* homologie des produits du gène d'enveloppe > 70 % vis-à-vis de la souche YBF30.

Les séquences décrites ci-dessus peuvent être utilisées pour la mise en oeuvre d'un procédé d'hybridation et/ou d'amplification génique de séquences nucléiques de type VIH-1, ces procédés étant applicables au diagnostic *in vitro* de l'infection potentielle d'un individu par un virus du type VIH-1 non-M non-O.

La présente invention a en outre pour objet un procédé de diagnostic *in vitro* réalisé à partir d'un échantillon biologique choisi parmi un échantillon de sérum et un échantillon de lymphocytes circulants, lequel procédé comprend :
. une étape d'extraction de l'acide nucléique à détecter, appartenant au génome du virus, éventuellement présent dans l'échantillon biologique et, le cas échéant, une étape de traitement de l'acide nucléique, à l'aide d'une transcriptase inverse, si ce dernier est sous forme d'ARN,
.au moins un cycle comprenant les étapes de dénaturation de l'acide nucléique, d'hybridation avec au moins une séquence conforme à l'invention et si nécessaire, extension de l'hybride formé, en présence de réactifs convenables comprenant un agent de polymérisation, tel qu'une ADN polymérase, et des dNTP, et
.une étape de détection de la présence éventuelle de l'acide nucléique appartenant au génome d'un virus de type VIH-1 de groupe non-M non-O.

Les conditions mises en oeuvre pour la PCR à l'aide des amorces issues de la souche YBF30 sont les suivantes :
- Extraction de l'ADN lymphocytaire par la technique phénol-chlorofome et quantification par spectrophotométrie à une longueur d'onde de 260 nm. Toutes les amplifications sont réalisées sur Perkin Elmer thermocycler 2400.
- Les PCR longues (9 kb) sont réalisées avec le kit XL PCR (Perkin Elmer) selon les conditions du fabriquant et avec les dNTP, les tampons fournis et le « hot start » de Perkin Elmer ; les cycles d'amplification de cette PCR longue sont :
   .1 cycle de dénaturation pendant 2 minutes à 94°C,
   .puis 16 cycles : 15 secondes à 94°C, 15 secondes à 55°C, 8 minutes à 68°C,
   .puis 24 cycles : 15 secondes à 94°C, 15 secondes à 55°C, 8 minutes à 68°C, en ajoutant à chaque cycle 15 secondes de plus (incrémentation).
- Les PCR nichées sont réalisées sur les produits d'amplification des PCR longues. Les conditions de réalisation des PCR nichées sont :
   .tampon et enzyme Taq polymérase « Expand High Fidelity PCR System » de Boehringer Mannheim selon les instructions du fabriquant, dNTP et « hot start » de Perkin Elmer,
   . 200 µM de chaque dNTP, 20 pmol de chaque amorce selon l'invention, 5 µl d'ADN, 10 µl de tampon PCR 10X, 2,6 unités de Taq polymérase dans un volume de 100 µl,
   . amplification : un cycle de 2 minutes à 94°C, suivie de 38 cycles : 15 secondes à 94°C, 15 secondes à 55°C, un temps d'élongation à 72°C variable selon la taille du produit de PCR à amplifier (de 30 secondes à 2 minutes) et un dernier cycle d'élongation de 10 minutes à 72°C.

La détection du produit amplifié est réalisée de préférence par séquençage direct.

La présente invention a également pour objet un peptide issu de la souche YBF30 sélectionné dans le groupe constitué par: celui exprimé par le gène *gag* (SEQ ID N° 4), celui exprimé par le gène *pol* (SEQ ID N° 6), celui exprimé par le gène *vif* (SEQ ID N° 8), celui exprimé par le gène *vpr* (SEQ ID N° 10), celui exprimé par le gène *vpu* (SEQ ID N° 12), celui exprimé par le gène *tat* (SEQ ID N° 14), celui exprimé par le gène *rev* (SEQ ID N° 16), celui exprimé par le gène *env* (SEQ ID N° 18), le fragment de la région de la boucle V3 CTRPGNNTGGQVQIGPAMTFYNIEKIVGDIRQAYC (SEQ ID N° 58) et celui exprimé par le gène *nef* (SEQ ID N° 20).

De préférence, le polypeptide selon l'invention est codé par une molécule d'acide nucléique définie par une séquence sélectionnée dans le groupe constitué par les séquences SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17 et SEQ ID NO: 19.

L'invention a également pour objet des compositions immunogènes comprenant un ou plusieurs produits de traduction des séquences nucléotidiques selon l'invention et/ou l'un des peptides tels que définis ci-dessus, obtenus notamment de manière synthétique.

Les anticorps dirigés contre l'un ou plusieurs des peptides décrits ci-dessus peuvent être utilisés pour la mise en oeuvre de méthodes de diagnostic *in vitro,* notamment différentielle, de l'infection d'un individu par un virus de type VIH-1, selon les procédés connus de l'homme du métier.

L'invention a, en outre, pour objet une méthode de diagnostic *in vitro* d'un VIH-1 non-M non-O, caractérisée en ce qu'elle comprend la mise en contact d'un échantillon biologique prélevé chez un patient, avec des anticorps tels que définis ci-dessus, éventuellement associés à des anticorps dirigés contre le virus SIV CPZGAB, et la détection des complexes immunologiques formés entre les antigènes de VIH-1, éventuellement présents dans l'échantillon biologique et lesdits anticorps.

L'invention a également pour objet un réactif de diagnostic d'un VIH-1 non-M non-O, caractérisé en ce qu'il comprend un acide nucléique selon l'invention, ou un peptide selon l'invention.

L'invention a en outre pour objet un procédé de criblage et de typage d'un VIH-1 non-M non-O caractérisé en ce qu'il comprend la mise en contact de l'un quelconque des fragments d'acides nucléiques selon l'invention avec l'acide nucléique du virus à typer, et la détection de l'hybride formé.

L'invention a également pour objet une trousse de diagnostic de VIH-1 non-M non-O, caractérisée en ce qu'elle inclut au moins un réactif selon l'invention.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :

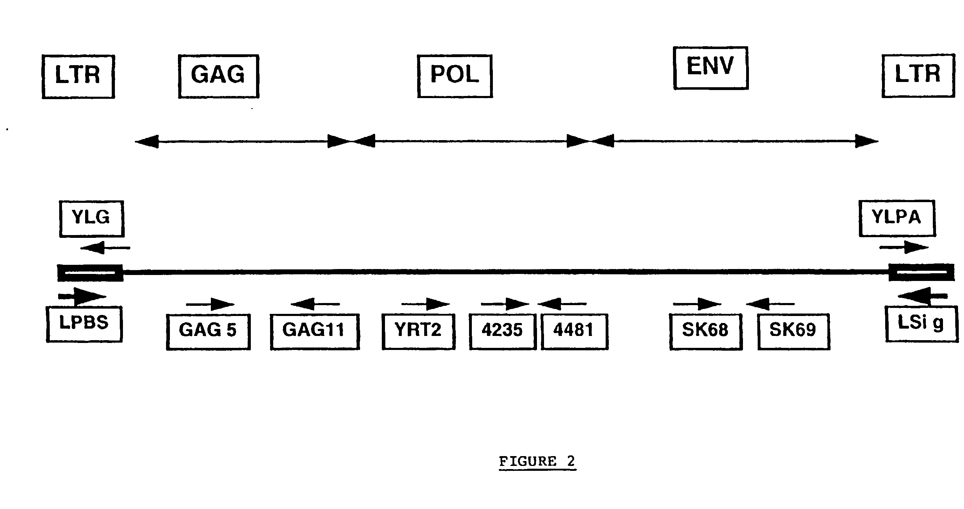

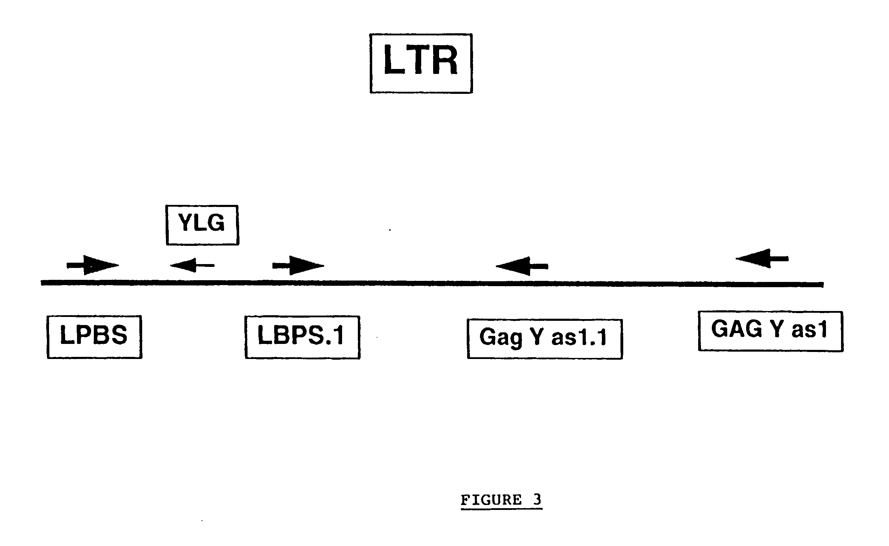

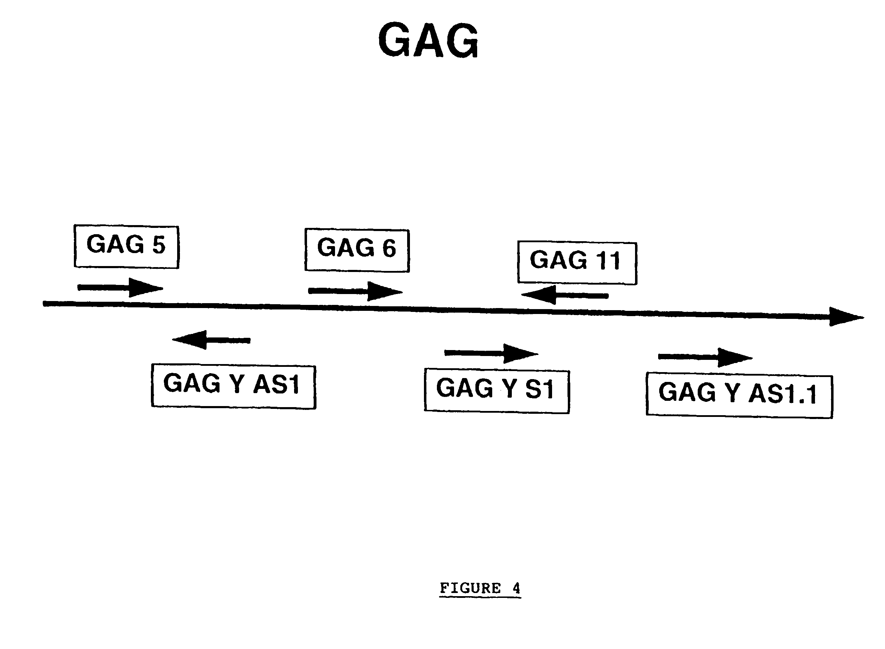

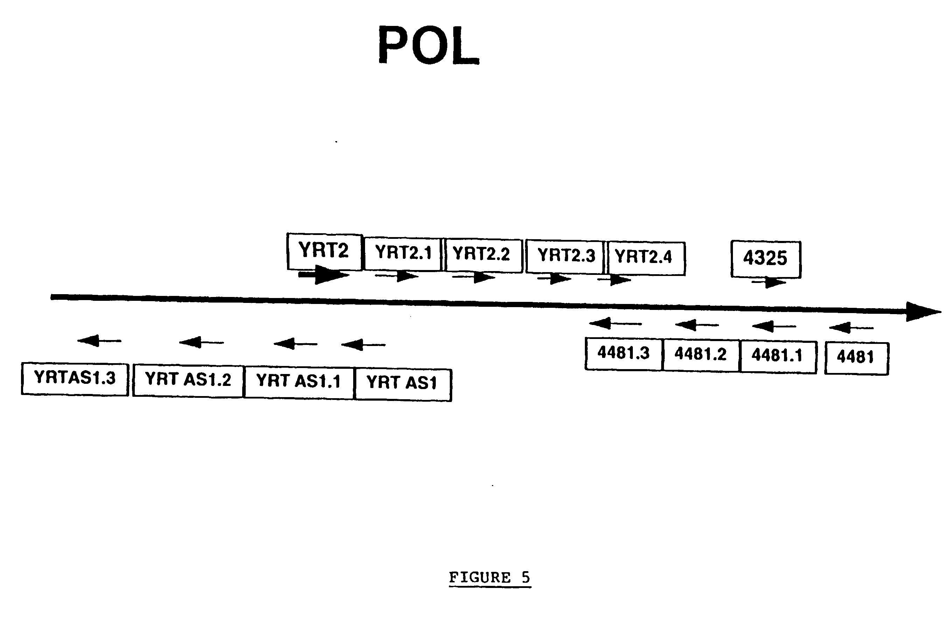

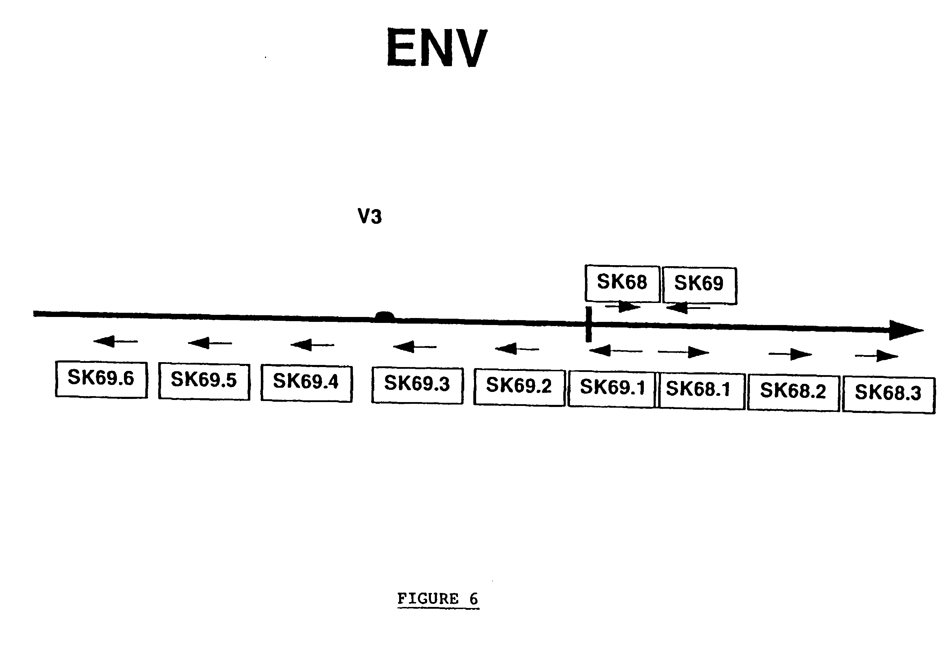

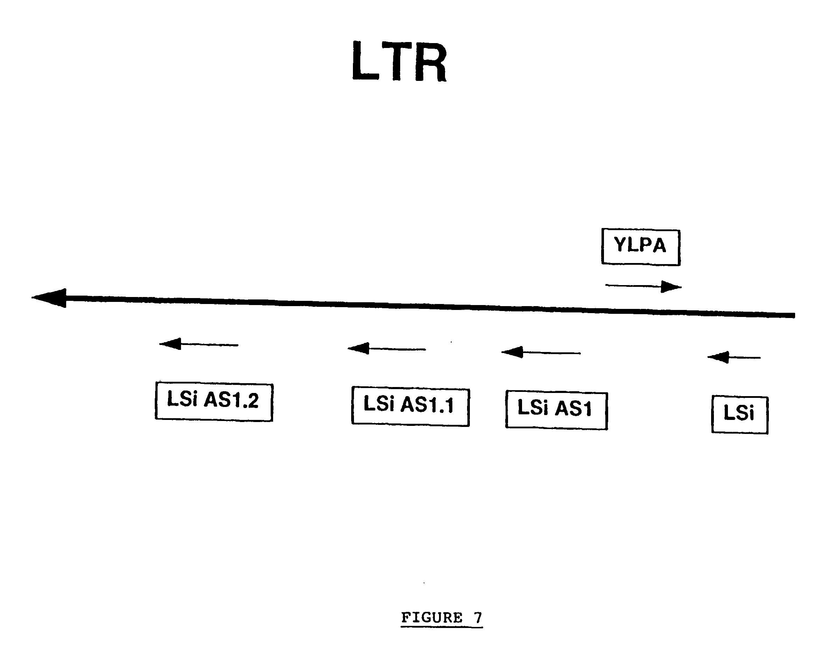

- les figures 1 à 7 illustrent l'emplacement des différentes amorces sur le génome de la souche YBF30 ;

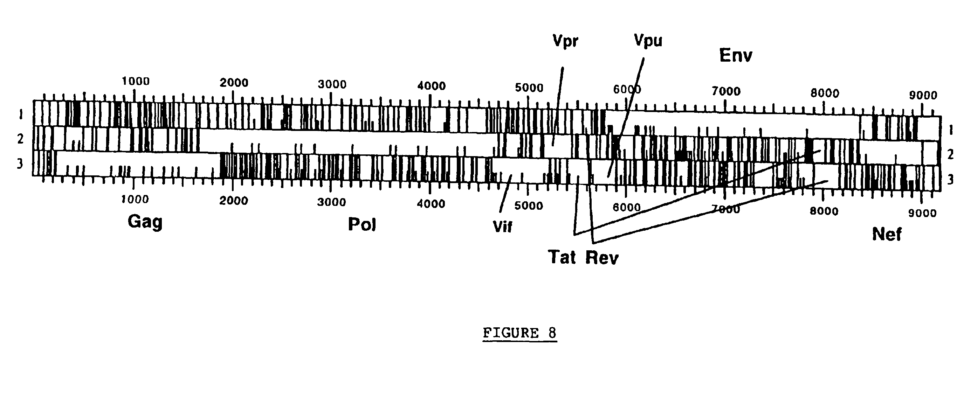

- la figure 8 illustre l'organisation génomique de la souche YBF30 ;
- les figures 9 à 19 représentent l'analyse phylogénétique des différents gènes de la souche YBF30 par rapport au VIH-1 de groupe M et de groupe O (figure 9 : gène *ltr,* figure 10 : gène *gag,* figure 11 : gène *tat,* figure 12 : gène *rev,* figure 13 : gène *vif*, figure 14 : gène *env* gp120, figure 15 : gène *env* gap41, figure 16 : gène *nef,* figure 17 : gène *pol,* figure 18 : gène *vpr,* figure 19 : gène *vpu*) ;
- la figure 20 illustre le pourcentage de distance génétique entre YBF30 et VIH-1/SIV CPZGAB.

Il doit être bien entendu, toutefois, que ces exemples sont donnés uniquement à titre d'illustration de l'objet de l'invention, dont ils ne constituent en aucune manière une limitation.

### EXEMPLE : Obtention d'un variant VIH-1 non-M non-O selon l'invention (YBF30) et ses applications.

Ceci a en particulier été possible en étudiant l'épidémiologie de l'infection par les virus de l'immunodéficience humaine acquise (VIH) au Cameroun, qui est particulièrement paradoxale. Dans ce pays, la diversité des souches est remarquable, puisque la plupart des sous-types connus à ce jour des virus VIH-1 du groupe M (Majeur) ont été rapportés. Des cas d'infections par des virus VIH-1 hautement divergeants du groupe O (O pour outlier) ont été rapportés, presque exclusivement chez des patients d'origine camerounaise. Des cas d'infections par VIH-2, HTLV-1 et HTLV-2 sous-type A et B ont été également rapportés.

Sur la base des résultats des évaluations sérologiques et génotypiques antérieures, les Inventeurs ont établi un algorithme de différenciation et de confirmation entre les infections VIH-1 des groupes M et O, afin de sélectionner des variants non-M, non-O.

Ces méthodes ont été appliquées sur des échantillons adressés au Laboratoire National de Référence des infections à VIH de Yaoundé et ont permis de caractériser un isolat VIH hautement divergeant et de définir les outils de caractérisation d'un nouveau groupe VIH-1, compte tenu des homologies observées entre cette souche humaine YBF30 et la souche simienne SIV CPZGAB.

### I - Moyen de caractérisation sérologique du variant YBF30 lors de l'étude épidémiologique.

### 1) Recueil des échantillons

Tous les sérums de patients adultes adressés au Laboratoire de référence de Yaoundé en 1994 et 1995 pour dépistage ou confirmation d'une infection HIV ont été étudiés (n=8831).

### 2) Différenciation sérologique entre VIH-1 groupe M et groupe O et sélection des variants :

En cas de positivité du dépistage anti-VIH (EIA indirect mixte HIV-1 et HIV-2 Génélavia Mixt, Sanofi-Pasteur, Paris, France), un test EIA basé sur le principe de la compétition vis à vis d'antigène spécifique du groupe M (Wellcozyme Rec HIV-1, Murex, Dartford, UK), a été associé.

En cas de positivité du test de type compétitif Wellcozyme Rec HIV-1, avec ratio de réactivité en densité optique (DO) par rapport à la valeur seuil ou *cut-off* (CO) supérieur à (CO/DO >5), le sérum est considéré comme VIH-1 positif, résultat qui doit être confirmé sur un nouveau prélèvement.

Le choix d'un ratio de réactivité supérieur à 5 pour considérer le test par compétition comme test de confirmation de l'infection à VIH-1 est basé sur l'expérience acquise par le laboratoire de virologie de l'hôpital Bichat : sur 7200 échantillons réactifs avec un ratio > 5, tous présentaient un Western Blot VIH-1 (WB, New Lav Blot 1, SDP, Marnes la Coquette) fortement positif En dehors des cas de séro-conversions VIH-1, les échantillons confirmés VIH positifs et présentant un ratio Wellcozyme < 5, correspondent soit à des infections par VIH-2, soit à des infections par VIH-1 du groupe O ou d'autres variants.

Pour éliminer les réactions faussement positives en dépistage EIA mixte, les échantillons présentant un ratio CO/DO < 5 sont systématiquement testés par un EIA mixte HIV-1/HIV-2 de troisième génération (Enzygnost Plus, Marburg, Germany) incluant les antigènes des VIH-1 des groupes M et O (recombinant gp41 de la souche MVP5180). En cas de positivité de ce test, un test rapide discriminant HIV-1 et HIV-2 (Multispot, SDP, Marnes la Coquette) et un Western Blot (WB, New Lav Blot 1 ou 2, SDP) sont réalisés.

### 3) Confirmation sérologique des infections VIH-1 groupe O et variants

Tous les échantillons présentant un ratio CO/DO < 5, différenciés positifs par WB (critères de positivité : 2 ENV +/- POL +/- GAG ou 1 ENV + POL +/GAG) et HIV-1, sont testés par un test *Dot-blot* utilisant des antigènes peptidiques des régions V3 et transmembranaires (InnoLia, Innogenetics, Ghent, Belgium).

### 4) Isolement rétroviral des souches de groupe O et des variants.

Les cellules mononucléées sanguines périphériques (PBMC) des patients séropositifs ont été isolés par gradient de Ficoll-Hypaque au Cameroun, conservées et transportées à Paris en azote liquide.

Après décongélation, les PBMC des patients ont été cocultivés avec des lymphocytes de donneurs caucasiens séronégatifs. La réplication virale dans les surnageants de cultures a été mise en évidence par la détection de l'activité transcriptase inverse et par la recherche de l'Antigène p24 (Elavia p24 polyclonal, SDP) sur une période d'un mois.

### 5) Séquences :

Les produits des PCR sont visualisés sur gels d'agarose de 1 à 1,4 % selon la taille des fragments, précipités en acétate de sodium 3M (1:10) et 3 volumes d'éthanol absolu, incubés 30 minutes à -80°C, centrifugés 20 minutes à 13 000 rpm. Le culot est séché puis repris avec 10 µl d'eau distillée (Sigma) La purification est réalisée sur « Qiaquick Gel Extraction kit » (Qiagen) selon les instructions du fabriquant ; les produits sont séquencés avec le Kit Dye Terminator Applied Biosystem sur un automate DNA Sequencer (Applied Biosystems, Inc., Foster Cit, CA), comme décrit précédemment (Loussert-Ajaka et al, 1995) ; les séquences nucléotidiques sont analysées sur logiciel Séquence Navigator (Applied Biosystems), alignés avec le logiciel GeneWorks (Intelligenetics Inc.).

### 6) Analyses phylogénétiques:

Les séquences ont été alignées avec le logiciel CLUSTAL pour les alignements multiples, en prenant comme matrice de référence, les alignements de la compilation des séquences VIH du laboratoire de Biologie et de Biophysique Théorique de Los Alamos, New Mexico, 87545 USA.

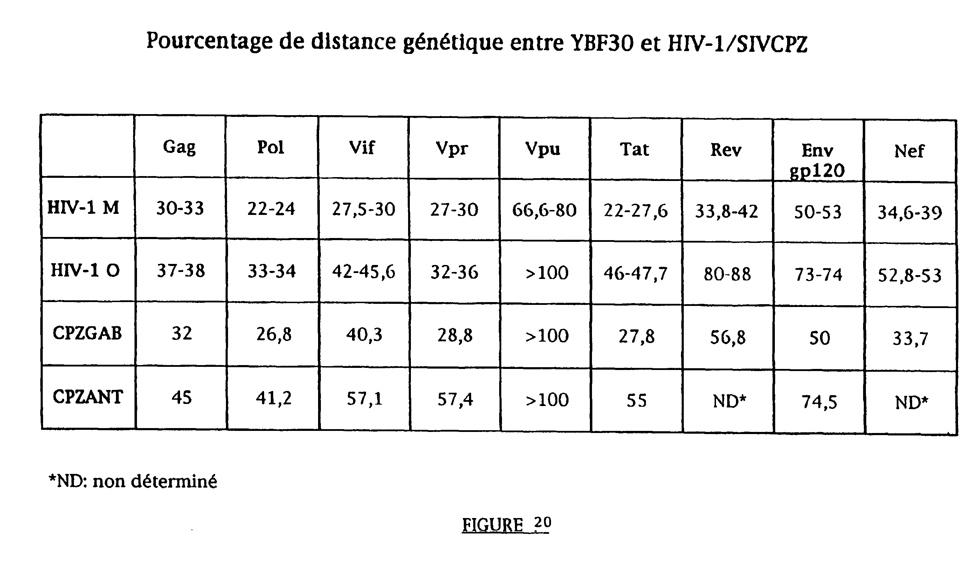

Les analyses phylogénétiques ont été faites avec le logiciel PHYLIP ; dans un premier temps, les distances ont été calculées avec DNADIST, puis l'analyse phylogénétique a ensuite été réalisée avec NEIGHBOR JOINING ou FITCH , enfin, les arbres ont été dessinés avec DRAWTREE (figures 9 à 19). Les pourcentages de distance génétique sont également illustrés à la figure 20.

Pour les analyses de « *boostrapping* », SEQBOOT a d'abord été utilisé, suivi de DNADIST et NEIGHBOR-JOINING ou FITCH. Enfin les valeurs de *boostrap* ont été obtenues avec CONSENS.

### II - Résultats de l'enquête de mise en évidence des VIH groupe O et variant :

174 échantillons, parmi 3193 échantillons positifs au dépistage, ont été considérés soit groupe O, soit groupe M avec réactivité sérologique anormale, soit comme variants.

### III - Mise en évidence d'un échantillon non groupe O et non groupe M présentant une réactivité sérologique anormale

Les 174 sérums HIV-1 positifs par WB (Western Blot), mais réactifs avec un ratio CO/DO < 5 en EIA de type compétitif ont été testés par dot blot LIA de différenciation sur les peptides V3 du groupe M, groupe O et SIV CPZGAB :
- 7 ne réagissent sur aucun des peptides (M, O ou SIV CPGGAB) représentés. L'absence de collecte cellulaire ne permet aucune conclusion.
- 82 présentent une réactivité vis à vis d'au moins un des peptides correspondant à la boucle V3 des souches du groupe O. La fréquence des réactions croisées est faible et limitée aux épitopes correspondant aux régions V3 consensus (11 %) et SIV-CPZ GAB (43 %).
- 84 sérums sont non réactifs vis-à-vis des épitopes du groupe O. Ces prélèvements ont été réalisés majoritairement chez des patients présentant un SIDA (75/84).
- un sérum, prélevé chez une patiente camerounaise (NJ) est réactif exclusivement avec le peptide SIV CPZGAB. Cette réactivité isolée vis à vis d'un antigène du SIV CPZGAB n'a jamais été décrite auparavant. Des lymphocytes ayant été collectés chez la patiente, la caractérisation virologique de cette souche nommée YBF30 a pu être poursuivie.

### IV - Résultats des examens sérologiques et virologiques sur les premiers prélèvements effectués sur cette patiente (mai 1995) (N° sérum : 95-6295) :

1) Tests ELISA commerciaux (Densité optique/valeur seuil)
   Critère de positivité : DO/CO > 1
   Génélavia = > 15
   Wellcozyme CO/DO = 1,55
   Abbott Plus = >15
   Behring Plus= 4,2
2) Western blot
   WB New Lav 1 Pasteur :
   160++, 120++, 68++,55+, 41+, 40+/-, 34++, 24++, 18+
3) LIA dot-Blot Innogenetics
   Négatif pour toutes les bandes groupe O et groupe M sauf V3 SIV CPZGAB

### 4) Résultats des examens sérologiques de recherche sur peptides spécifiques des groupes M et O

* La technique du Pr. Francis Barin du Laboratoire de Virologie du CHU de Tours a été adaptée (Barin F. et al., 1996) ; des peptides des régions transmembranaires synthétisés (BioMérieux) on été utilisés, pour mettre au point un test de différenciation entre les groupes M et O Cette technique est basée sur la compétition de liaison des anticorps entre les peptides transmembranaires gp41 des groupes O et M déposés sur la phase solide et des peptides transmembranaires gp41 soit du groupe O, soit du groupe M en concentration supérieure en une phase liquide de réaction hyperosmolaire. Les résultats sont illustrés au Tableau I ci-après, dans lequel le puits CP correspond au témoin d'inhibition 100 % et le puits CSP correspond au contrôle 0 % d'inhibition.

**Tableau I**

| **Résultats des différenciations inter groupe O - groupe M du sérum 6295** | | | | |
|---|---|---|---|---|
| | gp41 M | gp41 O | CP | CSP |
| 6295 | 0,25 | 0,36 | 0,12 | 1,98 |

Ces résultats montrent qu'il existe une forte liaison vis-à-vis des peptides de la phase solide (CSP), une nette inhibition par l'adjonction combinée des peptides M et O (CP) mais pas de nette différenciation, soit par le peptide M, soit par le peptide O. Il existe donc une évidence sérologique que la souche infectante n'appartient ni au groupe M, ni au groupe O.

* Compte tenu d'une réactivité isolée sur le dot blot InnoLia vis-à-vis des antigènes V3 SIV CPZGAB, sur les mêmes bases de compétition entre peptides, ce sérum a été étudié en mettant en compétition les peptides gp41 M, gp41 O et gp 41 SIV CPZGAB.

L'utilisation du sérum du chimpanzé dénommé 'Amandine' (donné par M. Peeters, qui a isolé la souche SIV CPZGAB, AIDS 1992) a permis, dans un premier temps, de valider cette technique. Sur le tableau II, les valeurs (DO) les plus basses indiquent le plus haut degré de liaison aux antigènes.

**Tableau II**

| **Résultats des différenciations inter groupe O - groupe M - SIVcpzGab avec le sérum du chimpanzé Amandine et le sérum 6295** | | | | | |
|---|---|---|---|---|---|
| | gp41 M | gp41 O | gp41 CPZGAB | CP | CSP |
| Amandine | 0,8 | 1,4 | 0,3 | 0,5 | 1,9 |
| 6295 | 0,7 | 1,1 | 0,7 | 0,4 | 2,1 |

La réactivité du sérum « Amandine » confirme et valide le test selon l'invention et indique que le sérum de la patiente réagit de manière identique vis-à-vis des peptides M et SIV CPZGAB, mais est sans réaction croisée avec le peptide O.

Ces résultats montrent qu'il existe une inhibition similaire avec le sérum de la patiente par les peptides gp41 du groupe M et gp41 SIV CPZGAB. Les antigènes de la souche infectante ont donc donné naissance à des anticorps reconnaissant, de façon similaire, les gp 41 du groupe M et du SIV CPZGAB.

### 4) Résultats obtenus à partir de l'isolement lymphocytaire (prélèvement mai 1995)

Un rétrovirus a été isolé à partir des lymphocytes prélevés le 22 mai 1995, selon les techniques classiques La culture avec la lignée MT2 montre que la souche YBF30 ne forme pas de syncytia (NSI).

### V - Résultats des examens sérologiques sur le deuxième prélèvement (Novembre 1995) (N° sérum : 95-3371)

### 1) LIA dot-Blot Innogenetics

Négatif pour toutes les bandes, sauf V3 SIV CPZGAB

### 2) Résultats des examens sérologiques de recherche sur peptides spécifiques des groupes M et O.

Le Tableau III illustre les résultats des différenciations gp41 inter groupe O - groupe M - SIV CPZGAB avec le sérum 3371

**Tableau III**

| **Résultats des différenciations gp41 inter groupe O - groupe M - SIV CPZGAB avec le sérum 3371** | | | | | |
|---|---|---|---|---|---|
| | gp41 M | gp410 | gp41 cpz-gab | CP | CSP |
| 3371 | 1,31 | 1,7 | 0,89 | 0,54 | 2,02 |

Ces résultats confirment sur ce nouveau prélèvement (effectué chez la même patiente, en phase terminale de la maladie) qu'il existe une inhibition marquée avec le sérum de la patiente par le peptide gp41 SIV CPZGAB.

Les antigènes de la souche infectante ont donc induit des anticorps reconnaissant de façon préférentielle la gp 41 du SIV CPZGAB.

### 3) Résultats de l'isolement lymphocytaire (prélèvement novembre 95 (95-3371-YBF31))

Un rétrovirus a été isolé à partir des lymphocytes prélevés en novembre 1995, selon les techniques classiques et dénommé YBF31 ; les éléments de séquence sont identiques à ceux de YBF30.

### VI - Amplification génomique et Séquences de YRF 30

L'ADN pour toutes les manipulations de PCR est extrait à partir des cellules de fin de culture positive.

Les PCR réalisées avec les amorces VIH-1 groupe O dans différentes régions testées sont négatives (*gag, pol, env*) *.* De même, celles réalisées avec les amorces spécifiques du VIH-1 groupe M sont négatives.

Les conditions d'amplification et d'hybridation pour les PCR du groupe O sont réalisées dans les conditions décrites dans Loussert-Ajaka, 1995 Les conditions d'amplification et d'hybridation pour les PCR du groupe M sont celles décrites par les Auteurs cités ci-après.

Ces amorces groupe M sont positionnées selon la séquence HIV-1-HXB2:
- Dans l'*env* gp120 : ED3/ED12 (position 5956-5985 ; 7822-7792) ; EDS/ED14 (6556-6581 ; 7960-7931), EDS/ED12 ; ED3/ED14 ; ES7/ES8 (7001-7020 ; 7667-7647) (Delwart et al. Science 1993; 262 1257-1261).
- Dans l'*env* gp41: première PCR ED3/M29, suivie d'une PCR nichée M28/M29 (7785-7808 ; 8099-8124) ; M28/M29 présentent les séquences suivantes:
   M28 CGGTTCTT(AG)GGAGCAGC(ACT)GGAAGCA,
   M29 : T(CT)T(ACGT)TCCCA(CT)T(AT)(CT)A(AGT)CCA(AGT)GTCAT ;
      SK68/SK69 (Ou et al. Science, 1988, 239. 295-297).
- Dans le *gag :* Amplicor Roche Diagnostics systems ; amorces *gag* nichées (Loussert-Ajaka et al. Lancet 1995, 346. 912-913) ; SK38/SK39 (Ou et al., Science, 1988; 239: 295-297).
- Dans le *pol :* A/NE1 (Boucher et al., Lancet, 1990; 336 585-590), Pol3/Pol4 (Lauré et al., Lancet, 1988, ii, 538-541 ).

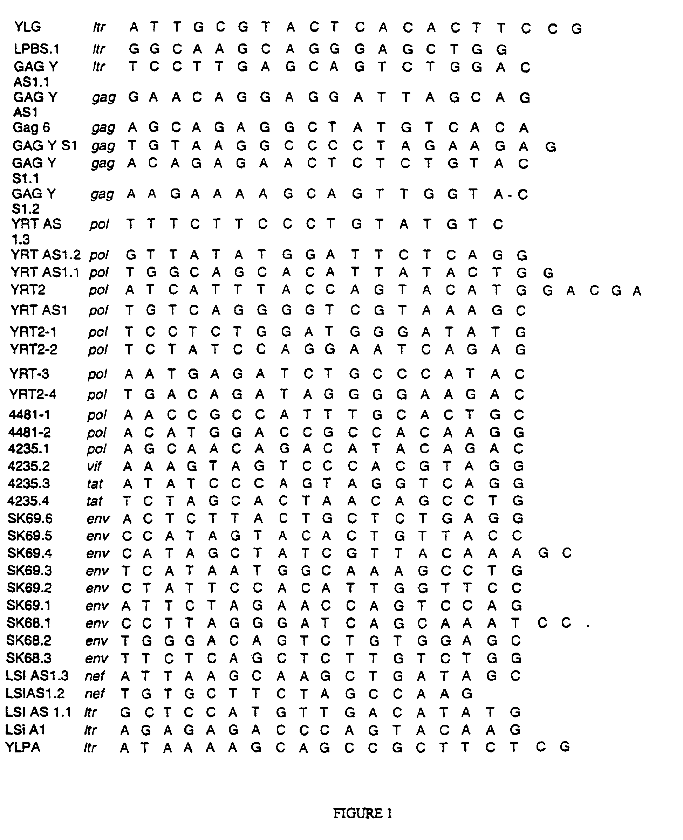

Seules les PCR réalisées avec les amorces H Pol sont positives (4235/4538) suivie d'une PCR nichée avec les amorces 4327/4481 (Fransen et al. Molecular and Cellular Probes 1994; 8: 31 7-322). Ce fragment H Pol, localisé dans l'intégrase (260 pb), a été séquencé. L'amplification avec les amorces HPOL est rendue possible, en raison de l'excès de virus. En effet, l'ADN utilisé est extrait des cellules de fin de culture fortement positive (transcriptase inverse > 100.000 cpm). L'amplification de l'ADN extrait des cellules fraîches sans coculture est impossible de par le nombre important de mésappariement entre les amorces HPOL (surtout dans la région 3') et la séquence de l'isolat YBF30. La conservation de cette extrémité 3' est très importante pour l'activité d'extension de la Taq polymérase.
1 Séquence du gène *pol :* l'utilisation d'amorces très dégénérées pour l'amplification par RT-PCR du RNA extrait du surnagent de culture positif, a donné une amplification positive. Ce sont des amorces communes à tous les rétrovirus (Donehower et al. J. Virol. Methods 1990; 28: 33-46), situés dans la région de la transcriptase inverse du gène *pol*. L'analyse du fragment après sequence a permis de générer une amorce spécifique YRT2 (SEQ ID N° 32) de l'Isolat YBF30 et d'amplifier le gène *pol* en utilisant l'amorce Hpol 4481 (Fransen et al., 1994 précité), comme amorce anti-sens. La séquence du fragment a été réalisée en synthétisant au fur et à mesure des amorces spécifiques pour chaque fragment généré (Figure 1).
2 Séquence du gène *env* : la deuxième approche a été de faire une PCR longue (XL-PCR, Perkin Elmer) amplifiant tout le virus (9000 pb) en utilisant des amorces situées dans le LTR : LPBS 1 (SEQ ID N°22) ; LSiGi, suivie d'une PCR nichée avec YRT2 (SEQ ID N° 32)/SK69 de 6000 pb, et de séquencer toute l'enveloppe en suivant la même procédure. La séquence de la région gp41 a été réalisée en utilisant une PCR nichée avec les amorces SK68/LSiGi.
3 Séquence du gène *gag :* utilisation d'une PCR nichée, réalisée par PCR longue (LPBS 1 /LSiGi), avec les amorces Gag 5 et Gag 1 li, et en générant au fur et à mesure des amorces spécifiques, afin de marcher sur le génome viral.

### VII - Résultats des séquences

La souche YBF30 a été complètement séquencée (voir liste des séquences). La souche YBF31 de Novembre 1995 a été partiellement séquencée et l'absence de variation significative confirme la validité des séquences de YBF30

### VIII - Synthèse de peptides de la régions de la boucle V3 de la souche YRF30.

L'étude des séquences de la région de la boucle V3 a permis de synthétiser le peptide correspondant et de comparer les acides aminés de cette région de la souche YBF 30 avec ceux des autres sous-types M et des souches O.

Les séquences des peptides sont :

| | |
|---|---|
| YBF30 | SEQ N° ID 58 |
| SIV CPZGAB : | CHRPGNNTRGEVQIGPGMTFYNIENVYGDTRSAYC (SEQ ID N° 59) |
| GROUPE O : (ANT70) | CIRPGNRTYRNLQIGPGMTFYNVEIATGDIRKAFC (SEQ ID N° 60) |
| GROUPE M : (SS-TYPE A) | CTRPNNNTRKSVRIGPGQAFYATGDIIGDIRQAHC (SEQ ID N° 61) |

Le peptide a été synthétisé, à partir des 2 asparagines de la région 5' de la boucle et utilisé selon le même principe que décrit précédemment (voir IV 4)), à savoir en compétition par rapport aux peptides du groupe M, du groupe O et du SIV CPZGAB. Les résultats illustrés au Tableau IV confirment l'originalité de cette souche et l'extension possible de ces souches puisque les résultats sérologiques sont en faveur d'infection du type YBF30 au Cameroun. En outre, l'étude de 200 sérums sélectionnés VIH-1 positifs du Cameroun met en évidence un nouveau cas présentant un profil similaire à celui de YBF30.

**Tableau IV**

| **Etude de réactivité de 200 sérums** | | | | | | |
|---|---|---|---|---|---|---|
| **Sérum** | **Origine** | **V3A** | **V3cpz** | **V3YBF30** | **CP** | **CSP** |
| 953371 | Cameroun | 1,66 | 0,38 | 1,39 | 0,39 | 1,64 |
| 956295. | Cameroun | 1,72 | 0,37 | 1,16 | 0,51 | 1,73 |
| 967321 | Cameroun | 0,07 | 0,17 | 0,5 | 0,05 | 0,27 |
| Amandine | SIVGAB | 1,74 | 0,14 | 1,48 | 0,19 | 1,74 |
| NOA.* | SIVANT | 2,66 | 0,31 | 1,88 | 0,46 | 1,9 |

| | | | | | | |
|---|---|---|---|---|---|---|
| * sérum du SIV CPZ ANT | | | | | | |

Sur ce nouveau test, la réactivité des sérums 953371 et 956295, correspondant à la patiente chez qui la souche YBF30 a été isolée, avec le peptide SIV-CPZ, a été confirmée. La plus faible réactivité vis à vis de son propre antigène V3 est classique lors des stades tardifs de la maladie. Cette réactivité reste cependant supérieure à celle relevée vis à vis du peptide M. Un autre patient camerounais (sérum 967321) présente le même profil de réactivité peptidique.

### Bibliographie :

* Barin F. et al., Aids Research and Human Retroviruses, 1996, 12, 13, 1279-1289, Diversity of Antibody Binding to V3 Peptides Representing Consensus Sequences of HIV Type 1 Genotypes A to E: An Approach for HIV Type 1 Serological Subtyping*.*
* Chameau P., Borman AM., Quillent C., Guétard D , Chamaret S., Cohen J., Rémy G., Montagnier L., and F. Clavel, Virology, 1994, 205, 247-253, Isolation and envelope sequence of a highly divergent HIV-1 isolate : definition of a new HIV -1 group*.*
* Descamps D., Collin G., Loussert-Ajaka I., Saragosti S., Simon F. and F. Brun-Vezinet. AIDS, 1995, 9, 977-978, HIV-1 group O sensitivity to antiretroviral drugs*.*
* Huet, T., Cheynier R., Meyerhans A., Roelants G., and S. Wain-Hobson, Nature, 1990, 345, 356-359, Genetic organization of a chimpanzee lentivirus related to HIV-1*.*
* Korber BTM., MacInnes K., Smith R. and G. Myers, J. Virol., 1994, 68, 6730-6744, Mutational trends in V3 loop protein sequences observed in different genetic lineages of HIV-1*.*
* Loussert-Ajaka I., Ly TD., Chaix ML., Ingrand D., Saragosti S., Couroucé AM., Brun-Vezinet F. and F. Simon, Lancet, 1994, 343, 1393-1394, HIV-1/HIV-2 seronegativity in HIV-1 J subtype O infected patients*.*
* Loussert-Ajaka I., Chaix ML., Korber B., Letourneur F., Gomas E., Allen E., Ly TD., Brun-Vezinet F., Simon F. and S. Saragosti, J. Virol., 1995, 69, 5640-5649, Variability of HIV type 1 group O strains isolaled from Cameroonian patients living in FRANCE*.*
* Murphy, E., B. Korber, Georges-Courbot, MC., You B., Pinter A., Cook D., Kienky MP., Georges A., Mathiot C., Barré-Sinoussi F., and M. Girard, AIDS Res. Hum. Retroviruses, 1993, 9, 997-1006, Diversity of V3 region sequences of human immunodeficiency viruses type 1 from the Central African Republic*.*
* G. Myers, Aids Res. Hum. Retrovir., 1994, 10, 11, 1317-1324, Tenth Anniversary Perspectives on AIDS*.*
* Nkengasong, J.N., Janssens W., Heyndrickx L., Fransen K., Ndumbe PM., Motte J., Leonaers A., Ngolle M., Ayuk J., Piot P., and G. Van der Groen, AIDS, 1994, 8, 1405-1412, Genotypic subtypes of HIV-1 in Cameroon*.*
* Sharp P.M. et al., AIDS, 1994, 8, suppl. 1, S27-S42, Origins and diversity of human immunodeficiency viruses*.*
* Simon, F., T.D. Ly, A. Baillou-Beaufils, V. Schneider-Fauveau, J. de Saint-Martin, I. Loussert-Ajaka, M.L. Chaix, S. Saragosti, A.M. Couroucé, D. Ingrand, C. Janot, and F. Brun-Vezinet. AIDS, 1994, 8, 1628-1629. Sensitivity of screening kits for anti-HIV-1 subtype O antibodies*.*
* Zekeng, L, L. Gurtler, E. Afane Ze, A. Sam-Abbenyi, G. Mbouni, Essomba, E. Mpoudi-Ngolle, M. Monny-Lobbe, J.B. Tapko, and L. Kaptue, AIDS, 1994, 8, 1626-1628, Prevalence of HIV subtype O infection in Cameroon : preliminary results*.*

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

## Revendications

1. Souche de VIH-1 non-M non-O, dénommée YBF30, déposée à la Collection Nationale de Cultures de Microorganismes tenue par l'Institut Pasteur sous le numéro I-1753 le 2 juillet 1996.

2. Acide nucléique isolé, issu de la souche selon la revendication 1, **caractérisé en ce qu'**il est sélectionné dans le groupe constitué par les acides nucléiques de séquences suivantes : SEQ ID N°1, SEQ ID N°2, SEQ ID N°3, SEQ ID N°5, SEQ ID N°7, SEQ ID N°9, SEQ ID N°11, SEQ ID N°13, SEQ ID N°15, SEQ ID N°17, et SEQ ID N°19.

3. Acide nucléique isolé, **caractérisé en ce qu'**il est constitué d'un fragment d'au moins 10 nucléotides d'un acide nucléique selon la revendication 2.

4. Acide nucléique selon la revendication 3, **caractérisé en ce qu'**il est sélectionné parmi les acides nucléiques de séquences SEQ ID N°21 à 57 et **en ce qu'**il est apte à servir d'amorce et/ou de sonde pour la détection d'un VIH-1 présentant les caractéristiques morphologiques et immunologiques de la souche VIH-1 non-M, non-O selon la revendication 1.

5. Procédé de diagnostic *in vitro* d'un VIH-1 de groupe non-M non-O, par hybridation et/ou amplification génique, réalisé à partir d'un échantillon biologique choisi parmi un échantillon de sérum et un échantillon de lymphocytes circulants, lequel procédé est **caractérisé en ce qu'**il comprend :
. une étape d'extraction de l'acide nucléique à détecter, appartenant au génome du virus, éventuellement présent dans l'échantillon biologique et, le cas échéant, une étape de traitement de l'acide nucléique, à l'aide d'une transcriptase inverse, si ce dernier est sous forme d'ARN,
. au moins un cycle comprenant les étapes de dénaturation de l'acide nucléique, d'hybridation avec au moins une séquence selon la revendication 2 ou la revendication 4 et si nécessaire, extension de l'hybride formé, en présence de réactifs convenables comprenant un agent de polymérisation et des dNTP, et
. une étape de détection de la présence éventuelle de l'acide nucléique appartenant au génome d'un virus de type VIH-1 de groupe non-M non-O.

6. Peptide isolé, **caractérisé en ce qu'**il consiste en une séquence choisie dans le groupe constitué par les séquences SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20 et SEQ ID NO: 58.

7. Peptide selon la revendication 6, **caractérisé en ce qu'**il est codé par une molécule d'acide nucléique définie par une séquence choisie dans le groupe constitué par les séquences SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17 et SEQ ID NO: 19.

8. Compositions immunogènes comprenant un ou plusieurs produits de traduction des séquences nucléotidiques selon la revendication 2 et/ou l'un des peptides selon la revendication 6 ou la revendication 7.

9. Méthode de diagnostic *in vitro* d'un VIH-1 non-M non-O, **caractérisée en ce qu'**elle comprend la mise en contact d'un échantillon biologique prélevé chez un patient, avec des anticorps dirigés contre l'un ou plusieurs des peptides selon la revendication 6 et la détection des complexes immunologiques formés entre les antigènes de VIH-1, éventuellement présents dans l'échantillon biologique et lesdits anticorps.

10. Méthode selon la revendication 9, **caractérisée en ce que** lesdits anticorps dirigés contre l'un ou plusieurs des peptides selon la revendication 6 sont associés à des anticorps dirigés contre le virus SIV CPZGAB.

11. Réactif de diagnostic d'un VIH-1 non-M non-O, **caractérisé en ce qu'**il comprend un acide nucléique selon l'une quelconque des revendications 2 et 4, ou un peptide selon l'une quelconque des revendications 6 et 7.

12. Procédé de criblage et de typage d'un VIH-1 non-M non-O, **caractérisé en ce qu'**il comprend la mise en contact de l'un quelconque des fragments nucléotidiques selon la revendication 3 ou la revendication 4 avec l'acide nucléique du virus à typer et la détection de l'hybride formé.

13. Trousse de diagnostic de VIH-1 non-M non-O, **caractérisée en ce qu'**elle inclut au moins un réactif selon la revendication 11.

## Claims

1. Strain of non-M non-O HIV-1 called YBF30, filed in the Collection Nationale de Cultures de Microorganismes held by the Institut Pasteur under number I-1753 on 2 July 1996.

2. Isolated nucleic acid from the strain according to claim 1, **characterised in that** it is selected in the group consisting of the nucleic acids of the following sequences: SEQ ID no. 1, SEQ ID no. 2, SEQ ID no. 3, SEQ ID no. 5, SEQ ID no. 7, SEQ ID no. 9, SEQ ID no. 11, SEQ ID no. 13, SEQ ID no. 15, SEQ ID no. 17 and SEQ ID no. 19.

3. Isolated nucleic acid, **characterised in that** it consists of a fragment of at least 10 nucleotides of a nucleic acid according to claim 2.

4. Nucleic acid according to claim 3, **characterised in that** it is selected from the nucleic acids of sequences SEQ ID no. 21 to 57 and **in that** it is capable of serving as a bootstrap and/or probe for the detection of an HIV-1 having the morphological and immunological characteristics of the non-M, non-O HIV-1 strain according to claim 1.

5. In vitro diagnostic process for an HIV-1 of non-M non-O group by gene hybridisation and/or amplification, carried out using a biological sample selected from a sample of serum and a sample of circulating lymphocytes, which process is **characterised in that** it comprises:
- an extraction step of the nucleic acid to be detected belonging to the virus genome, possibly present in the biological sample and, where necessary, a treatment step of the nucleic acid, using a reverse transcriptase, if this latter is in RNA form,
- at least one cycle comprising the steps of denaturation of the nucleic acid, hybridisation with at least one sequence according to claim 2 or claim 4 and, if necessary, extension of the hybrid formed, in the presence of suitable reagents comprising a polymerisation agent and dNTPs, and
- a detection step of the possible presence of the nucleic acid belonging to the genome of an HIV-1 non-M non-O group type virus.

6. Isolated peptide, **characterised in that** it consists of a selected sequence in the group consisting of the sequences SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20 and SEQ ID NO: 58.

7. Peptide according to claim 6, **characterised in that** it is coded by a nucleic acid molecule defined by a selected sequence in the group consisting of the sequences SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17 and SEQ ID NO: 19.

8. Immunogenic compositions comprising one or more translation products of the nucleotide sequences according to claim 2 and/or one of the peptides according to claim 6 or claim 7.

9. In vitro diagnostic method for a non-M non-O HIV-1, **characterised in that** it comprises contacting a biological sample taken from a patient with the antibodies directed against one or more of the peptides according to claim 6 and the detection of the immunological complexes formed between the HIV-1 antigens possibly present in the biological sample and the said antibodies.

10. Method according to claim 9, **characterised in that** the said antibodies directed against one or more of the peptides according to claim 6 are associated with antibodies directed against the SIV virus CPZGAB.

11. Diagnostic reagent of a non-M non-O HIV-1, **characterised in that** it comprises a nucleic acid according to either of claims 2 and 4, or a peptide according to either of claims 6 and 7.

12. Process for screening and typing a non-M non-O HIV-1, **characterised in that** it comprises contacting any of the nucleotide fragments according to claim 3 or claim 4 with the nucleic acid of the virus to be typed and detection of the hybrid formed.

13. Non-M non-O HIV-1 diagnostic kit, **characterised in that** it includes at least one reagent according to claim 11.

## Patentansprüche

1. Nicht-M, nicht-O HIV-1-Stamm, mit dem Namen YBF30, der am 2. Juli 1996 in der Collection Nationale de Cultures de Microorganismes, die vom Institut Pasteur unterhalten wird, unter der Nummer 1-1753 hinterlegt wurde.

2. Isolierte Nukleinsäure, die aus dem Stamm nach Anspruch 1 stammt, **dadurch gekennzeichnet, dass** sie ausgewählt ist aus der Gruppe, bestehend aus den Nukleinsäuren der folgenden Sequenzen: SEQ ID Nr. 1, SEQ ID Nr. 2, SEQ ID Nr. 3, SEQ ID Nr. 5, SEQ ID Nr. 7, SEQ ID Nr. 9, SEQ ID Nr. 11, SEQ ID Nr. 13, SEQ ID Nr. 15, SEQ ID Nr. 17 und SEQ ID Nr. 19.

3. Isolierte Nukleinsäure, **dadurch gekennzeichnet, dass** sie aus einem Fragment aus mindestens 10 Nukleotiden einer Nukleinsäure nach Anspruch 2 besteht.

4. Nukleinsäure nach Anspruch 3, **dadurch gekennzeichnet, dass** sie ausgewählt ist aus den Nukleinsäuren der Sequenzen SEQ ID Nr. 21 bis 57 und **dadurch**, dass sie in der Lage ist, als Primer und/oder Sonde zum Nachweis eines HIV-1 zu dienen, der die morphologischen und immunologischen Merkmale des nicht-M, nicht-O VIH-1-Stamms nach Anspruch 1 aufweist.

5. Verfahren zur In-vitro-Diagnose eines HIV-1 der Gruppe nicht-M, nicht-O, durch Hybridisierung und/oder gentechnische Amplifikation, die aus einer biologischen Probe ausgeführt wurde, ausgewählt aus einer Serumprobe und einer Probe aus zirkulierenden Lymphozyten, wobei das Verfahren **dadurch gekennzeichnet ist, dass** es umfasst:
einen Schritt der Extraktion der nachzuweisenden Nukleinsäure, die zum Genom des Virus gehört, die eventuell in der biologischen Probe vorhanden ist, und, gegebenenfalls einen Schritt zur Behandlung der Nukleinsäure mit Hilfe einer Umkehrtranskriptase, wenn letztgenannte in RNA-Form vorliegt,
mindestens einen Zyklus, der die Schritte zur Denaturierung der Nukleinsäure, der Hybridisierung mit mindestens einer Sequenz nach Anspruch 2 oder Anspruch 4 und wenn notwendig, die Extension des gebildeten Hybrids in Gegenwart von geeigneten Reagenzien umfasst, die ein Mittel zur Polymerisation und dNTP umfassen, und
einen Schritt zum Nachweis der eventuellen Gegenwart der Nukleinsäure, die zum Genom eines Virus vom Typ HIV-1 der Gruppe nicht-M, nicht-O gehört.

6. Isoliertes Peptid, **dadurch gekennzeichnet, dass** es aus einer Sequenz besteht, ausgewählt aus der Gruppe, bestehend aus den Sequenzen SEQ ID Nr. 4, SEQ ID Nr. 6, SEQ ID Nr. 8, SEQ ID Nr. 10, SEQ ID Nr. 12, SEQ ID Nr. 14, SEQ ID Nr. 16, SEQ ID Nr. 18, SEQ ID Nr. 20, SEQ ID Nr. 58.

7. Peptid nach Anspruch 6, **dadurch gekennzeichnet, dass** es mit einem Molekül der Nukleinsäure kodiert, die durch eine Sequenz definiert ist, ausgewählt aus der Gruppe, bestehend aus den Sequenzen SEQ ID Nr. 1, SEQ ID Nr. 3, SEQ ID Nr. 5, SEQ ID Nr. 7, SEQ ID Nr. 9, SEQ ID Nr. 11, SEQ ID Nr. 13, SEQ ID Nr. 15, SEQ ID Nr. 17 und SEQ ID Nr. 19.

8. Immunogene Zusammensetzungen, die ein oder mehrere Translationsprodukte der Nukleotidsequenzen nach Anspruch 2 und/oder eines der Peptide nach Anspruch 6 oder Anspruch 7 umfassen.

9. Verfahren zur In-vitro-Diagnose eines nicht-M, nicht-O HIV-1, **dadurch gekennzeichnet, dass** es das Inkontaktbringen einer biologischen Probe, die einem Patienten entnommen wurde, mit Antikörpern umfasst, die sich gegen ein oder mehrere der Peptide nach Anspruch 6 richten, und den Nachweis von immunologischen Komplexen, die sich zwischen den HIV-1-Antigenen, die eventuell in der biologischen Probe vorhanden sind, und den Antikörpern gebildet haben.

10. Verfahren nach Anspruch 9, **dadurch gekennzeichnet, dass** die Antikörper, die gegen ein oder mehrere Peptide nach Anspruch 6 gerichtet sind, mit Antikörpern assoziiert sind, die gegen den Virus SIV CPZGAB gerichtet sind.

11. Reagens zur Diagnose eines nicht-M, nicht-O HIV-1, **dadurch gekennzeichnet, dass** es eine Nukleinsäure nach einem der Ansprüche 2 und 4 oder ein Peptid nach einem der Ansprüche 6 und 7 umfasst.

12. Verfahren zum Screenen und zur Typisierung eines nicht-M, nicht-O HIV-1, **dadurch gekennzeichnet, dass** es das Inkontaktbringen irgendeines der Nukleotidfragmente nach Anspruch 3 oder Anspruch 4 mit der Nukleinsäure des zu typisierenden Virus und den Nachweis des gebildeten Hybrids umfasst.

13. Kit zur Diagnose von nicht-M, nicht-O HIV-1, **dadurch gekennzeichnet, dass** es mindestens ein Reagens nach Anspruch 11 beinhaltet.
